# EUROPEAN PATENT SPECIFICATION

(11) **EP 2 572 726 B1**
(45) Date of publication and mention of the grant of the patent: **04.05.2016**
(21) Application number: 12197549.4
(22) Date of filing: 01.08.2008
(51) Int. Cl.: A61K 39/09, C07K 14/315, C07K 19/00, C12N 1/21

(54) **Compositions comprising pneumococcal antigens**
Zusammensetzungen mit Pneumokokken-Antigenen
Compositions comprenant des antigènes pneumocoques

(30) Priority: 01.08.2007 GB 0714963; 29.08.2007 US 966866 P
(43) Date of publication of application: 27.03.2013
(62) Divisional of application: 08826694.5
(73) Proprietor: GlaxoSmithKline Biologicals SA, 1330 Rixensart (BE)
(72) Inventor: Donati, Claudio, 53100 Siena (IT); Muzzi, Alessandro, 53100 Siena (IT); Masignani, Vega, 53100 Siena (IT); Bagnoli, Fabio, 53100 Siena (IT); Ruggiero, Paolo, 53100 Siena (IT); Barrochi, Michéle, 53100 Siena (IT)
(74) Representative: Courgeon, Antoine

(56) References cited:
- WO-A1-2013/068949
- WO-A2-2004/092209
- WAI-LEUNG NG ET AL: "Defective cell wall synthesis in Streptococcus pneumoniae R6 depleted for the essential PcsB putative murein hydrolase or the VicR (YycF) response regulator", MOLECULAR MICROBIOLOGY, vol. 53, no. 4, 1 August 2004 (2004-08-01) , pages 1161-1175, XP055052844, ISSN: 0950-382X, DOI: 10.1111/j.1365-2958.2004.04196.x
- M. F. MILLS ET AL: "Localization of PcsB of Streptococcus pneumoniae and Its Differential Expression in Response to Stress", JOURNAL OF BACTERIOLOGY, vol. 189, no. 12, 15 June 2007 (2007-06-15), pages 4544-4546, XP055052789, ISSN: 0021-9193, DOI: 10.1128/JB.01831-06
- NG W-L ET AL: "Regulation of the pspA virulence factor and essential pcsB murein biosynthetic genes by the phosphorylated VicR (YycF) response regulator in Streptococcus pneumoniae", JOURNAL OF BACTERIOLOGY, AMERICAN SOCIETY FOR MICROBIOLOGY, WASHINGTON, DC; US, vol. 187, no. 21, 1 November 2005 (2005-11-01), pages 7444-7459, XP002549018, ISSN: 0021-9193, DOI: 10.1128/JB.187.21.7444-7459.2005
- GREGOR ZYSK ET AL.: "Detection of 23 immunogenic pneumococcal proteins using convalescent-phase serum", INFECTION AND IMMUNITY, vol. 68, no. 6, June 2000 (2000-06), pages 3740-3743, XP001098495,
- GIEFING C ET AL: "Discovery of a novel class of highly conserved vaccine antigens using genomic scale antigenic fingerprinting of pneumococcus with human antibodies", THE JOURNAL OF EXPERIMENTAL MEDICINE, ROCKEFELLER UNIVERSITY PRESS, US, vol. 205, no. 1, 21 January 2008 (2008-01-21), pages 117-131, XP002535798, ISSN: 0022-1007, DOI: 10.1084/JEM.20071168
- ESPOSITO D ET AL: "Enhancement of soluble protein expression through the use of fusion tags", CURRENT OPINION IN BIOTECHNOLOGY, LONDON, GB, vol. 17, no. 4, 1 August 2006 (2006-08-01) , pages 353-358, XP024962788, ISSN: 0958-1669, DOI: 10.1016/J.COPBIO.2006.06.003 [retrieved on 2006-08-01]

## Description

### TECHNICAL FIELD

This invention relates to antigens derived from *S.pneumoniae* and their use in immunisation, as defined in the claims.

### BACKGROUND ART

*Streptococcus pneumoniae,* also known as pneumococcus, is a Gram-positive spherical bacterium. It is the most common cause of acute bacterial meningitis in adults and in children over 5 years of age. Current pneumococcal vaccines are based on capsular saccharides. The only pediatric vaccine is PREVNAR™, which is a mixture of conjugated saccharides from 7 different serotypes. An adult vaccine based on a mixture of conjugated saccharides from 23 different serotypes is also available. Both of these vaccines are difficult to manufacture, though, and there are more than 90 pneumococcal serotypes in total. Thus there remains a need to identify further and improved antigens for use in pneumococcal vaccines.

Reference 10 discloses the identification of *S*. *pneumoniae* hyperimmune serum reactive antigens useful for vaccination.

### DISCLOSURE OF THE INVENTION

The invention provides an immunogenic composition comprising a combination of *S.pneumoniae* antigens, said combination comprising a spr0096 antigen and a spr2021 antigen, wherein:
said spr0096 antigen is a polypeptide that comprises an amino acid sequence:
   (a) having 80% or more identity to SEQ ID NO: 12; and/or (b) comprising a fragment of at least 10 consecutive amino acids, comprising an epitope of SEQ ID NO: 12; and
said spr2021 antigen is a polypeptide that comprises an amino acid sequence:
   (a) having 80% or more identity to SEQ ID NO: 11; and/or (b) comprising a fragment of at least 10 consecutive amino acids, comprising an epitope of SEQ ID NO: 11.

The invention also provides a polypeptide of formula NH2-A-{-X-L-}n-B-COOH, wherein:
X is an amino acid sequence of a pneumococcal antigen, selected from the group consisting of: (1) a spr0057 antigen; (2) a spr0096 antigen; (3) a spr0565 antigen; and (4) a spr2021 antigen; (5) a RrgA antigen; and (6) a RrgB antigen;
L is an optional linker amino acid sequence;
A is an optional N-terminal amino acid sequence; B is an optional C-terminal amino acid sequence, n is an integer of 2 or more; and
at least one instance of X is spr0096 and at least one instance of X is spr2021; wherein:
   said spr0057 antigen comprises an amino acid sequence: (a) having 80% or more identity to SEQ ID NO: 1; and/or (b) comprises a fragment of at least 10 consecutive amino acids, comprising an epitope of SEQ ID NO: 1;
   said spr0096 antigen comprises an amino acid sequence: (a) having 80% or more identity to SEQ ID NO: 12; and/or (b) comprises a fragment of at least 10 consecutive amino acids, comprising an epitope of SEQ ID NO: 12;
   said spr0565 antigen comprises an amino acid sequence: (a) having 80% or more identity to SEQ ID NO: 3; and/or (b) comprises a fragment of at least 10 consecutive amino acids, comprising an epitope of SEQ ID NO: 3;
   said spr2021 antigen comprises an amino acid sequence: (a) having 80% or more identity to SEQ ID NO: 11; and/or (b) comprises a fragment of at least 10 consecutive amino acids, comprising an epitope of SEQ ID NO: 11;
   said RrgA antigen comprises an amino acid sequence: (a) having 80% or more identity to SEQ ID NO: 172 or 179; and/or (b) comprises a fragment of at least 10 consecutive amino acids, comprising an epitope of SEQ ID NO: 172 or 179; and/or
   said RrgB antigen comprises an amino acid sequence: (a) having 80% or more identity to SEQ ID NO: 173, 174 or 175; and/or (b) comprises a fragment of at least 10 consecutive amino acids, comprising an epitope of SEQ ID NO: 173, 174, or 175.

The invention further provides a polypeptide having 75% or more sequence identity to an amino acid sequence selected from the group consisting of SEQ ID NOs: 194 and 205.

Similar to references 1 and 2, the present inventors believe that an effective *S.pneumoniae* vaccine may require several antigenic components, and so they have identified various combinations of pneumococcal polypeptides for use in immunisation. They have also identified some pneumococcal polypeptides that may be useful as single antigens. These polypeptides may optionally be used in combination with pneumococcal saccharides or other pneumococcal polypeptides. The antigens may be used in pneumococcal vaccines, but may also be used as components in vaccines for immunising against multiple pathogens.

The inventors have identified the following 7 pneumococcal polypeptides: spr0057; spr0286; spr0565; spr1098; spr1345; spr1416; spr1418. This set of antigens is referred to herein as 'the first antigen group'. Thus the disclosure provides an immunogenic composition comprising a combination of *S.pneumoniae* antigens, said combination comprising two or more (*i.e.* 2, 3, 4, 5, 6 or all 7) antigens selected from the group consisting of: (1) a spr0057 antigen; (2) a spr0286 antigen; (3) a spr0565 antigen; (4) a spr1098 antigen; (5) a spr1345 antigen; (6) a spr1416 antigen; and/or (7) a spr1418 antigen.

The inventors have identified the following 4 pneumococcal polypeptides: spr0867; spr1431; spr1739; spr2021. This set of antigens is referred to herein as 'the second antigen group'. Thus the disclosure provides an immunogenic composition comprising a combination of *S.pneumoniae* antigens, said combination comprising two or more (*i.e.* 2, 3 or all 4) antigens selected from the group consisting of: (1) a spr0867 antigen; (2) a spr1431 antigen; (3) a spr1739 antigen; and/or (4) a spr2021 antigen

The inventors have identified the following 3 pneumococcal polypeptides: spr0096; spr1433; spr1707. This set of antigens is referred to herein as 'the third antigen group'. Thus the disclosure provides an immunogenic composition comprising a combination of *S.pneumoniae* antigens, said combination comprising two or three antigens selected from the group consisting of: (1) a spr0096 antigen; (2) a spr1433 antigen; and/or (3) a spr1707 antigen.

The combination of 11 pneumococcal polypeptides in the first and second antigen groups is referred to herein as 'the fourth antigen group'. Thus the disclosure provides an immunogenic composition comprising a combination of *S.pneumoniae* antigens, said combination comprising two or more (*i.e.* 2, 3, 4, 5, 6, 7, 8, 9, 10 or all 11) antigens selected from the group consisting of: (1) a spr0057 antigen; (2) a spr0286 antigen; (3) a spr0565 antigen; (4) a spr1098 antigen; (5) a spr1345 antigen; (6) a spr1416 antigen; (7) a spr1418 antigen; (8) a spr0867 antigen; (9) a spr1431 antigen; (10) a spr1739 antigen; and/or (11) a spr2021 antigen.

The combination of 10 pneumococcal polypeptides in the first and third antigen groups is referred to herein as 'the fifth antigen group'. Thus the disclosure provides an immunogenic composition comprising a combination of *S.pneumoniae* antigens, said combination comprising two or more (*i.e.* 2, 3, 4, 5, 6, 7, 8, 9, or all 10) antigens selected from the group consisting of: (1) a spr0057 antigen; (2) a spr0286 antigen; (3) a spr0565 antigen; (4) a spr1098 antigen; (5) a spr1345 antigen; (6) a spr1416 antigen; (7) a spr1418 antigen; (8) a spr0096 antigen; (9) a spr1433 antigen; and/or (10) a spr1707 antigen.

The combination of 7 pneumococcal polypeptides in the second and third antigen groups is referred to herein as 'the sixth antigen group'. Thus the disclosure provides an immunogenic composition comprising a combination of *S.pneumoniae* antigens, said combination comprising two or more (*i.e.* 2, 3, 4, 5, 6, or all 7) antigens selected from the group consisting of: (1) a spr0867 antigen; (2) a spr1431 antigen; (3) a spr1739 antigen; (4) a spr2021 antigen; (5) a spr0096 antigen; (6) a spr1433 antigen; and/or (7) a spr1707 antigen.

The combination of 14 pneumococcal polypeptides in the first, second and third antigen groups is referred to herein as 'the seventh antigen group'. Thus the disclosure provides an immunogenic composition comprising a combination of *S.pneumoniae* antigens, said combination comprising two or more (*i.e.* 2, 3, 4, 5, 6, 7, 8, 9, 10, 11, 12, 13, or all 14) antigens selected from the group consisting of: (1) a spr0057 antigen; (2) a spr0286 antigen; (3) a spr0565 antigen; (4) a spr1098 antigen; (5) a spr1345 antigen; (6) a spr1416 antigen; (7) a spr1418 antigen; (8) a spr0867 antigen; (9) a spr1431 antigen; (10) a spr1739 antigen; (11) a spr2021 antigen; (12) a spr0096 antigen; (13) a spr1433 antigen; and/or (14) a spr1707 antigen.

Within the seventh antigen group, a preferred subset of four antigens is the 'eighth antigen group', which includes an antigen from each of the first, second and third groups, namely spr0057, spr0096, spr0565 and spr2021. Thus the invention provides an immunogenic composition comprising a combination of *S.pneumoniae* antigens, said combination comprising two or more (*i.e.* 2, 3 or all 4) antigens selected from the group consisting of: (1) a spr0057 antigen; (2) a spr0096 antigen; (3) a spr0565 antigen; and/or (4) a spr2021 antigen, as defined in the claims. Within this eighth group, the composition may comprise: (1), (2) and (3); (1), (2) and (4); (1), (3) and (4); (2), (3) and (4); or (1), (2), (3) and (4). Expression of these four antigens has been immunologically confirmed across a panel of 32 pneumococcal strains with various serotypes.

The 'ninth antigen group' is the eighth antigen group plus a RrgB pilus antigen. Thus the invention also provides an immunogenic composition comprising a combination of *S.pneumoniae* antigens, said combination comprising one or more RrgB pilus antigen(s) and two or more (*i.e.* 2, 3 or all 4) antigens selected from the group consisting of: (1) a spr0057 antigen; (2) a spr0096 antigen; (3) a spr0565 antigen; and/or (4) a spr2021 antigen, as defined in the claims.

The 'tenth antigen group' is the eighth antigen group plus a Pmp polypeptide. Thus the invention also provides an immunogenic composition comprising a combination of *S.pneumoniae* antigens, said combination comprising two or more (*i.e.* 2, 3, 4 or all 5) antigens selected from the group consisting of: (1) a spr0057 antigen; (2) a spr0096 antigen; (3) a spr0565 antigen; (4) a spr2021 antigen; and/or (5) a Pmp polypeptide, as defined in the claims.

Specific combinations of interest comprise: (i) a spr0057 antigen and a spr0096 antigen; (ii) a spr0057 antigen and a spr2021 antigen; (iii) a spr0057 antigen, a spr0096 antigen and a spr2021 antigen; (iv) a spr0057 antigen and a spr0565 antigen; (v) a spr0565 antigen and a spr2021 antigen; (vi) a spr0057 antigen, a spr0565 antigen and a spr2021 antigen; (vii) a spr0565 antigen, a spr2021 antigen and a spr1739 antigen e.g. detoxified; and (viii) a spr0565 antigen, a spr2021 antigen and a Pmp polypeptide, as defined in the claims.

The invention also provides an immunogenic composition comprising two or more different pneumococcal exoglycosidases, as defined in the claims.

The invention also provides an immunogenic composition comprising at least one pneumococcal exoglycosidase and at least one pneumococcal peptidoglycan hydrolase, as defined in the claims.

The invention also provides an immunogenic composition comprising two or more different peptidoglycan hydrolases, as defined in the claims.

Advantageous combinations of the invention are those in which two or more antigens act synergistically. Thus the protection against pneumococcal disease achieved by their combined administration exceeds that expected by mere addition of their individual protective efficacy.

### Further polypeptides antigens

In addition to antigens from the various antigen groups of the invention, immunogenic compositions, as defined in the claims, may include one or more of the following polypeptides to enhance the anti-pneumococcal immune response elicited by the composition:
- One or more subunits of a pneumococcal pilus, such as RrgA, RrgB and/or RrgC.
- A ClpP polypeptide.
- A LytA polypeptide.
- A CPL1 polypeptide.
- A PhtA polypeptide.
- A PhtB polypeptide.
- A PhtD polypeptide.
- A PhtE polypeptide.
- A CbpD polypeptide
- A CbpG polypeptide
- A PvaA polypeptide.
- A Hic polypeptide.
- A Pmp polypeptide.
- A ZmpB polypeptide.
- A PspA polypeptide
- A PsaA polypeptide
- A PspC polypeptide.
- A PrtA polypeptide.
- A Sp91 polypeptide.
- A Sp133 polypeptide.
- A PiuA polypeptide and/or a PiaA polypeptide.
- A spr0222 polypeptide.
- An antigen selected from the group consisting of: IC1; IC2; IC3; IC4; IC5; IC6; IC7; IC8; IC9; IC10; IC11; IC12; IC13; IC14; IC15; IC16; IC17; IC18; IC19; IC20; IC21; IC22; IC23; IC24; IC25; IC26; IC27; IC28; IC29; IC30; IC31; IC32; IC33; IC34; IC35; IC36; IC37; IC38; IC39; IC40; IC41; IC42; IC43; IC44; IC45; IC46; IC47; IC48; IC49; IC50; IC51; IC52; IC53; IC54; IC55; IC56; IC57; IC58; IC59; IC60; IC61; IC62; IC63; IC64; IC65; IC66; IC67; IC68; IC69; IC70; IC71; IC72; IC73; IC74; IC75; IC76; IC77; IC78; IC79; IC80; IC81; IC82; IC83; IC84; IC85; IC86; IC88; IC89; IC90; IC91; IC92; IC93; IC94; IC95; IC96; IC97; IC98; IC99; IC100; IC101; IC102; IC103; IC104; IC105; IC106; IC107; IC108; IC109; IC110; IC111; IC112; IC113; IC114; IC115; IC116; IC117; IC118; IC119; IC120; IC121; IC122; IC123; IC124; IC125; IC126; IC127; IC128; IC129; IC130; and IC131.

An antigen selected from the group consisting of: ID-204, ID-212, ID-213, ID-214, ID-215, ID-ID-216, ID-217, ID-219, ID-220, ID-225, ID-301, ID-302, ID-303, ID-304, ID-305, ID-306, as disclosed in reference 3.
- An antigen selected from the group consisting of: Sit1A, Sit1B, Sit1C, Sit2B, Sit2C, Sit2D, Sit3A, Sit3B, Sit3C, Sit3D, ORF1, ORF2, ORF3, ORF4, ORF5, ORF6, ORF6, ORF7, ORF8, ORF9, ORF10, ORF11, ORF12, ORF13, ORF14, MS1, MS2, MS3, MS4, MS5, MS6, MS7, MS8, MS9, MS 10 or MS11, as disclosed in reference 4.
- An antigen disclosed in reference 5.
- An antigen disclosed in Tables 1-3 of reference 6, such as CbiO.
- An antigen disclosed in reference 7, such as the 30S ribosomal protein S8.
- An antigen selected from the group consisting of: a phosphoenolpyruvate protein phosphotransferase; a phosphomannomutase; a trigger factor; an elongation factor G; a tetracycline resistance protein (tetO); a DNA-directed RNA polymerase alpha-chain; a NADH oxidase; a glutamyl-tRNA amidotransferase subunit A; a N utilization substance protein A homolog; a Xaa-His dipeptidase; a cell division protein ftsz; a zinc metalloproteinase; a L-lactate dehydrogenase; a glyceraldehyde 3-phosphate dehydrogenase (GAPDH); a fructose-biphosphate aldolase; a UDP-glucose 4-epimerase; a GTP binding protein typA/BipA a GMP synthase; a glutamyl-tRNA synthetase; a NADP-specific glutamate dehydrogenase; an elongation factor TS; a phosphoglycerate kinase; a pyridine nucleotide-disulfide oxidoreductase; a 40S ribosomal protein S1; a 6-phosphogluconate dehydrogenase; an aminopeptidase C; a carbomyl-phosphate synthase (large subunit); a PTS system mannose-specific IIAB component; a ribosomal protein S2; a dihydroorotate dehydrogenase; an aspartate carbamoyltransferase; an elongation factor Tu; a pneumococcal surface immunogenic protein A (PsipA); a phosphogycerate kinase; an ABC transporter substrate-binding protein endopeptidase O; a pneumococcal surface immunogenic protein B (PsipB); or a pneumococcal surface immunogenic protein C (PsipC) [8].

### Pili

Many strains of *S*.*pneumoniae* possess a pilus, encoded within a pathogenicity islet (*rlrA*). The islet encodes three surface proteins (RrgA, RrgB, and RrgC) and three sortase enzymes. In some embodiments of the invention, a composition, as defined in the claims, will includeone or more of: RrgA; RrgB; RrgC; SrtB; SrtC; and/or SrtD. Of these six proteins, including one or more of RrgA, RrgB and/or RrgC is preferred. RrgB is the most preferred pilus protein to be included.

Some strains possess a different pilus type [9], 'PI-2'. The PI-2 operon encodes PitA, SipA, PitB, SrtG1, and SrtG2. In some embodiments of the invention, a composition, as defined in the claims, will includeone or more of: PitA, SipA, PitB, SrtG1, and/or SrtG2. IC1 to IC131

As mentioned above, in some embodiments of the invention a composition, as defined in the claims, will includeone or more antigens selected from the group consisting of IC1 to IC131. These 131 polypeptides are disclosed in reference 10, being the 144 polypeptides of Table 3 therein except for those listed as SP0117, SP0641, SP0664, SP1003, SP1004, SP1174, SP1175, SP1573, SP1687, SP1693, SP1937 and SP2190. Within the 132 polypeptides IC1 to IC131, a preferred subset from which the one or more polypeptide(s) may be selected is: IC1; IC8; IC16; IC23; IC31; IC34; IC40; IC45; IC47; IC57; IC58; IC60; and IC69.

### Combinations with pneumococcal conjugates

The individual antigens identified in the antigen groups may be used in combination with pneumococcal conjugates. Thus the invention provides an immunogenic composition comprising a combination of:
(1) one or more antigen(s) selected from the sixth, seventh, eighth, ninth and tenth antigen groups (as defined above) and as defined in the claims; and
(2) one or more conjugates of a pneumococcal capsular saccharide and a carrier protein.

A conjugate used in component (2) of this combination includes a saccharide moiety and a carrier moiety. The saccharide moiety is from the capsular saccharide of a pneumococcus. The saccharide may be a polysaccharide having the size that arises during purification of the saccharide from bacteria, or it may be an oligosaccharide achieved by fragmentation of such a polysaccharide. In the 7-valent PREVNAR™ product, for instance, 6 of the saccharides are presented as intact polysaccharides while one (the 18C serotype) is presented as an oligosaccharide.

A composition may include a capsular saccharide from one or more of the following pneumococcal serotypes: 1, 2, 3, 4, 5, 6A, 6B, 7F, 8, 9N, 9V, 10A, 11A, 12F, 14, 15B, 17F, 18C, 19A, 19F, 20, 22F, 23F and/or 33F. A composition may include multiple serotypes *e.g.* 2, 3, 4, 5, 6, 7, 8, 9, 10, 11, 12, 13, 14, 15, 16, 17, 18, 19, 20, 21, 22, 23 or more serotypes. 7-valent, 9-valent, 10-valent, 11-valent and 13-valent conjugate combinations are already known in the art, as is a 23-valent unconjugated combination.

For example, an 10-valent combination may include saccharide from serotypes 1, 4, 5, 6B, 7F, 9V, 14, 18C, 19F and 23F. An 11-valent combination may further include saccharide from serotype 3. A 12-valent combination may add to the 10-valent mixture: serotypes 6A and 19A; 6A and 22F; 19A and 22F; 6A and 15B; 19A and 15B; r 22F and 15B; A 13-valent combination may add to the 11-valent mixture: serotypes 19A and 22F; 8 and 12F; 8 and 15B; 8 and 19A; 8 and 22F; 12F and 15B; 12F and 19A; 12F and 22F; 15B and 19A; 15B and 22F. *etc.*

The carrier moiety will usually be a protein, but preferably not one of the antigens of (1). Typical carrier proteins are bacterial toxins, such as diphtheria or tetanus toxins, or toxoids or mutants thereof. The CRM₁₉₇ diphtheria toxin mutant [11] is useful, and is the carrier in the PREVNAR™ product. Other suitable carrier proteins include the *N.meningitidis* outer membrane protein complex [12], synthetic peptides [13,14], heat shock proteins [15,16], pertussis proteins [17,18], cytokines [19], lymphokines [19], hormones [19], growth factors [19], artificial proteins comprising multiple human CD4⁺ T cell epitopes from various pathogen-derived antigens [20] such as N19 [21], protein D from *H.influenzae* [22-24], pneumolysin [25] or its non-toxic derivatives [26], pneumococcal surface protein PspA [27], iron-uptake proteins [28], toxin A or B from *C.difficile* [29], recombinant *P.aeruginosa* exoprotein A (rEPA) [30], *etc.*

Where a composition includes more than one conjugate, each conjugate may use the same carrier protein or a different carrier protein. Reference 31 describes potential advantages when using different carrier proteins in multivalent pneumococcal conjugate vaccines

In some embodiments, a single conjugate may carry saccharides from multiple serotypes [32]. Usually, however, each conjugate will include saccharide from a single serotype.

Conjugates may have excess carrier (w/w) or excess saccharide (w/w). In some embodiments, a conjugate may include equal weights of each.

The carrier molecule may be covalently conjugated to the carrier directly or via a linker. Direct linkages to the protein may be achieved by, for instance, reductive amination between the saccharide and the carrier, as described in, for example, references 33 and 34. The saccharide may first need to be activated e.g. by oxidation. Linkages via a linker group may be made using any known procedure, for example, the procedures described in references 35 and 36. A preferred type of linkage is an adipic acid linker, which may be formed by coupling a free -NH₂ group (*e.g*. introduced to a glucan by amination) with adipic acid (using, for example, diimide activation), and then coupling a protein to the resulting saccharide-adipic acid intermediate [37,38]. Another preferred type of linkage is a carbonyl linker, which may be formed by reaction of a free hydroxyl group of a saccharide CDI [39, 40] followed by reaction with a protein to form a carbamate linkage. Other linkers include β-propionamido [41], nitrophenyl-ethylamine [42], haloacyl halides [43], glycosidic linkages [44], 6-aminocaproic acid [45], ADH [46], C₄ to C₁₂ moieties [47], *etc.* Carbodiimide condensation can also be used [48].

The individual antigens identified in the antigen groups may be used as carrier proteins for pneumococcal capsular saccharides, to form a covalent conjugate. Thus the disclosure provides an immunogenic composition comprising a conjugate of (1) an antigen selected from the first, second, third, fourth, fifth, sixth, seventh, eighth, ninth or tenth antigen groups and (2) a pneumococcal capsular saccharide. Further characteristics of such a conjugate are described above. The use of pneumococcal proteins as carriers in conjugates is known in the art [*e.g.* refs. 25, 27 & 67]. These conjugates may be combined with any of the further antigens disclosed herein. Combinations with non-pneumococcal antigens

The individual antigens identified in the antigen groups may be used in combination with non-pneumococcal antigens. Thus the invention provides an immunogenic composition comprising a combination of:
(1) one or more antigen(s) selected from the sixth, seventh, eighth, ninth or tenth antigen groups (as defined above) and as defined in the claims; and
(2) one or more antigen(s) selected from the group consisting of: diphtheria toxoid; tetanus toxoid; hepatitis B virus surface antigen; an inactivated poliovirus antigen; one or more acellular pertussis antigens; a conjugate of the capsular saccharide antigen from *Haemophilus influenzae* type B; a conjugate of the capsular saccharide antigen from serogroup C of *Neisseria meningitidis*; a conjugate of the capsular saccharide antigen from serogroup Y of *Neisseria meningitidis*; a conjugate of the capsular saccharide antigen from serogroup W135 of *Neisseria meningitidis*; and a conjugate of the capsular saccharide antigen from serogroup A of *Neisseria meningitidis.*

Diphtheria toxoid can be obtained by treating (*e.g*. using formaldehyde) diphtheria toxin from *Corynebacterium diphtheriae.* Diphtheria toxoids are disclosed in more detail in chapter 13 of reference 49.

Tetanus toxoid can be obtained by treating (*e.g.* using formaldehyde) tetanus toxin from *Clostridium tetani.* Tetanus toxoids are disclosed in more detail in chapter 27 of reference 49.

Hepatitis B virus surface antigen (HBsAg) is the major component of the capsid of hepatitis B virus. It is conveniently produced by recombinant expression in a yeast, such as a *Saccharomyces cerevisiae.*

Inactivated poliovirus antigens are prepared from viruses grown on cell culture and then inactivated (*e.g*. using formaldehyde). Because poliomyelitis can be caused by one of three types of poliovirus, as explained in chapter 24 of reference 49, a composition may include three poliovirus antigens: poliovirus Type 1 (*e.g*. Mahoney strain), poliovirus Type 2 (*e.g.* MEF-1 strain), and poliovirus Type 3 (e*.g.* Saukett strain).

Acellular pertussis antigen(s) comprise specific purified *B.pertussis* antigens, either purified from the native bacterium or purified after expression in a recombinant host. It is usual to use more than one acellular antigen, and so a composition may include one, two or three of the following well-known and well-characterized *B.pertussis* antigens: (1) detoxified pertussis toxin (pertussis toxoid, or 'PT'); (2) filamentous hemagglutinin ('FHA'); (3) pertactin (also known as the '69 kiloDalton outer membrane protein'). FHA and pertactin may be treated with formaldehyde prior to use according to the invention. PT may be detoxified by treatment with formaldehyde and/or glutaraldehyde but, as an alternative to this chemical detoxification procedure, it may be a mutant PT in which enzymatic activity has been reduced by mutagenesis [50]. Further acellular pertussis antigens that can be used include fimbriae (*e.g*. agglutinogens 2 and 3).

When a composition includes one of diphtheria toxoid, tetanus toxoid or an acellular pertussis antigen in component (2) then it will usually include all three of them *i.e.* component (2) will include a D-T-Pa combination.

### First antigen group

### (1) spr0057

The original 'spr0057' sequence was annotated in reference 84 as 'Beta-N-acetyl-hexosaminidase precursor' (see GI:15902101). For reference purposes, the amino acid sequence of full length spr0057 as found in the R6 strain is given as SEQ ID NO: 1 herein.

Preferred spr0057 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 1; and/or (b) comprising a fragment of at least 'n' consecutive amino acids of SEQ ID NO: 1, wherein 'n' is 10 or more (*e.g.* 10, 12, 14, 16, 18, 20, 25, 30, 35, 40, 50, 60, 70, 80, 90, 100, 150, 200, 250 or more). These spr0057 proteins include variants of SEQ ID NO: 1. Preferred fragments of (b) comprise an epitope from SEQ ID NO: 1. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 1 while retaining at least one epitope of SEQ ID NO: 1. Other fragments omit one or more protein domains. One suitable fragment is SEQ ID NO: 180, which omits the natural leader peptide and sortase recognition sequences.

Combinations of spr0057 with other pneumococcal antigens have shown good synergistic effects.

### (2) spr0286

The original 'spr0286' sequence was annotated in reference 84 as 'Hyaluronate lyase precursor' (see GI:15902330). For reference purposes, the amino acid sequence of full length spr0286 as found in the R6 strain is given as SEQ ID NO: 2 herein.

Preferred spr0286 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 2; and/or (b) comprising a fragment of at least 'n' consecutive amino acids of SEQ ID NO: 2, wherein 'n' is 10 or more (*e.g.* 10, 12, 14, 16, 18, 20, 25, 30, 35, 40, 50, 60, 70, 80, 90, 100, 150, 200, 250 or more). These spr0286 proteins include variants of SEQ ID NO: 2. Preferred fragments of (b) comprise an epitope from SEQ ID NO: 2. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 2 while retaining at least one epitope of SEQ ID NO: 2. Other fragments omit one or more protein domains. One suitable fragment is SEQ ID NO: 181, which omits the natural leader peptide and sortase recognition sequences. Other suitable fragments are SEQ ID NOs: 182 and 183.

### (3) spr0565

The original 'spr0565' sequence was annotated in reference 84 as 'beta-galactosidase precursor' (see GI:15902609). For reference purposes, the amino acid sequence of full length spr0565 as found in the R6 strain is given as SEQ ID NO: 3 herein.

Preferred spr0565 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 3; and/or (b) comprising a fragment of at least 'n' consecutive amino acids of SEQ ID NO: 3, wherein 'n' is 10 or more (*e.g.* 10, 12, 14, 16, 18, 20, 25, 30, 35, 40, 50, 60, 70, 80, 90, 100, 150, 200, 250 or more). These spr0565 proteins include variants of SEQ ID NO: 3 (*e.g.* SEQ ID NO: 66; see below). Preferred fragments of (b) comprise an epitope from SEQ ID NO: 3. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 3 while retaining at least one epitope of SEQ ID NO: 3. Other fragments omit one or more protein domains. One suitable fragment is SEQ ID NO: 184, which omits the natural leader peptide and sortase recognition sequences. Other suitable fragments are SEQ ID NOs: 177 and 178.

A variant form of spr0565 is SEQ ID NO: 66 herein. The use of this variant form for immunisation is reported in reference 10 (SEQ ID NO: 178 therein). Useful spr0565 polypeptides may comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 66; and/or (b) comprising a fragment of at least 'n' consecutive amino acids of SEQ ID NO: 66, wherein 'n' is 10 or more (*e.g.* 10, 12, 14, 16, 18, 20, 25, 30, 35, 40, 50, 60, 70, 80, 90, 100, 150, 200, 250 or more). These polypeptides include variants of SEQ ID NO: 66. Preferred fragments of (b) comprise an epitope from SEQ ID NO: 66. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 66 while retaining at least one epitope of SEQ ID NO: 66. Other fragments omit one or more protein domains.

Immunogenic fragments of SEQ ID NO: 66 are identified in table 1 of reference 10.

Because spr0565 is naturally a long polypeptide (>2000 aa) it can be more convenient to express fragments. Thus a suitable form of spr0565 for use with the invention may be less than 1500 amino acids long (*e.g.* <1400, <1300, <1200, <1100, *etc*.). Such short forms of spr0565 include 'spr0565A' (SEQ ID NO: 177) and 'spr0565B' (SEQ ID NO: 178).

Combinations of spr0565 with other pneumococcal antigens have shown good synergistic effects. (4) *spr1098*

The original 'spr1098' sequence was annotated in reference 84 as 'Sortase' (see GI:15903141). For reference purposes, the amino acid sequence of full length spr1098 as found in the R6 strain is given as SEQ ID NO: 4 herein.

Preferred spr1098 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 4; and/or (b) comprising a fragment of at least 'n' consecutive amino acids of SEQ ID NO: 4, wherein 'n' is 10 or more (*e.g.* 10, 12, 14, 16, 18, 20, 25, 30, 35, 40, 50, 60, 70, 80, 90, 100, 150, 200, 250 or more). These spr1098 proteins include variants of SEQ ID NO: 4. Preferred fragments of (b) comprise an epitope from SEQ ID NO: 4. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 4 while retaining at least one epitope of SEQ ID NO: 4. Other fragments omit one or more protein domains. One suitable fragment is SEQ ID NO: 187, which omits the natural leader peptide sequence.

### (5) spr1345

The original 'spr1345' sequence was annotated in reference 84 as 'hypothetical protein' (see GI:15903388). For reference purposes, the amino acid sequence of full length spr1345 as found in the R6 strain is given as SEQ ID NO: 5 herein.

Preferred spr1345 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 5; and/or (b) comprising a fragment of at least 'n' consecutive amino acids of SEQ ID NO: 5, wherein 'n' is 10 or more (*e.g.* 10, 12, 14, 16, 18, 20, 25, 30, 35, 40, 50, 60, 70, 80, 90, 100, 150, 200, 250 or more). These spr1345 proteins include variants of SEQ ID NO: 5. Preferred fragments of (b) comprise an epitope from SEQ ID NO: 5. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 5 while retaining at least one epitope of SEQ ID NO: 5. Other fragments omit one or more protein domains. One suitable fragment is SEQ ID NO: 188, which omits the natural leader peptide and sortase recognition sequences.

### (6) spr1416

The original 'spr1416' sequence was annotated in reference 84 as 'hypothetical protein' (see GI:15903459). For reference purposes, the amino acid sequence of full length spr1416 as found in the R6 strain is given as SEQ ID NO: 6 herein.

Preferred spr1416 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 6; and/or (b) comprising a fragment of at least 'n' consecutive amino acids of SEQ ID NO: 6, wherein 'n' is 10 or more (*e.g.* 10, 12, 14, 16, 18, 20, 25, 30, 35, 40, 50, 60, 70, 80, 90, 100, 150, 200, 250 or more). These spr1416 proteins include variants of SEQ ID NO: 6. Preferred fragments of (b) comprise an epitope from SEQ ID NO: 6. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 6 while retaining at least one epitope of SEQ ID NO: 6. Other fragments omit one or more protein domains.

### (7) spr1418

The original 'spr1418' sequence was annotated in reference 84 as 'hypothetical protein' (see GI:15903461). For reference purposes, the amino acid sequence of full length spr1418 as found in the R6 strain is given as SEQ ID NO: 7 herein.

Preferred spr1418 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 7; and/or (b) comprising a fragment of at least 'n' consecutive amino acids of SEQ ID NO: 7, wherein 'n' is 10 or more (*e.g.* 10, 12, 14, 16, 18, 20, 25, 30, 35, 40, 50, 60, 70, 80, 90, 100, 150, 200, 250 or more). These spr1418 proteins include variants of SEQ ID NO: 7. Preferred fragments of (b) comprise an epitope from SEQ ID NO: 7. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 7 while retaining at least one epitope of SEQ ID NO: 7. Other fragments omit one or more protein domains.

### Second antigen group

### (1) spr0867

The original 'spr0867' sequence was annotated in reference 84 as 'Endo-beta-N-acetylglucosaminidase' (see GI:15902911). For reference purposes, the amino acid sequence of full length spr0867 as found in the R6 strain is given as SEQ ID NO: 8 herein.

Preferred spr0867 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 8; and/or (b) comprising a fragment of at least 'n' consecutive amino acids of SEQ ID NO: 8, wherein 'n' is 10 or more (*e.g.* 10, 12, 14, 16, 18, 20, 25, 30, 35, 40, 50, 60, 70, 80, 90, 100, 150, 200, 250 or more). These spr0867 proteins include variants of SEQ ID NO: 8. Preferred fragments of (b) comprise an epitope from SEQ ID NO: 8. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 8 while retaining at least one epitope of SEQ ID NO: 8. Other fragments omit one or more protein domains. One suitable fragment is SEQ ID NO: 185, which omits the natural leader peptide sequence.

### (2) spr1431

The original 'spr1431' sequence was annotated in reference 84 as '1,4-beta-N-acetylmuramidase' (see GI:15903474). It is also known as 'LytC', and its use for immunisation is reported in reference 67. For reference purposes, the amino acid sequence of full length spr1431 as found in the R6 strain is given as SEQ ID NO: 9 herein.

Preferred spr1431 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 9; and/or (b) comprising a fragment of at least 'n' consecutive amino acids of SEQ ID NO: 9, wherein 'n' is 10 or more (*e.g.* 10, 12, 14, 16, 18, 20, 25, 30, 35, 40, 50, 60, 70, 80, 90, 100, 150, 200, 250 or more). These spr1431 proteins include variants of SEQ ID NO: 9. Preferred fragments of (b) comprise an epitope from SEQ ID NO: 9. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 9 while retaining at least one epitope of SEQ ID NO: 9. Other fragments omit one or more protein domains. One suitable fragment is SEQ ID NO: 189, which omits the natural leader peptide sequence.

### (3) spr1739

The'spr1739' polypeptide is pneumolysin (*e.g*. see GI:15903781). For reference purposes, the amino acid sequence of full length spr1739 as found in the R6 strain is given as SEQ ID NO: 10 herein.

Preferred spr1739 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 10; and/or (b) comprising a fragment of at least 'n' consecutive amino acids of SEQ ID NO: 10, wherein 'n' is 10 or more (*e.g.* 10, 12, 14, 16, 18, 20, 25, 30, 35, 40, 50, 60, 70, 80, 90, 100, 150, 200, 250 or more). These spr1739 proteins include variants of SEQ ID NO: 10. Preferred fragments of (b) comprise an epitope from SEQ ID NO: 10. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 10 while retaining at least one epitope of SEQ ID NO: 10. Other fragments omit one or more protein domains.

Mutant forms of pneumolysin for vaccination use are known in the art [26, 51-56], and these mutant forms may be used with the invention. Detoxification can be achieved by C-terminal truncation (*e.g*. see ref. 57) *e.g.* deleting 34 amino acids, 45 amino acids, 7 amino acids [58], *etc.* Further mutations, numbered according to SEQ ID NO: 20, include Pro325→Leu (*e.g*. SEQ ID NO: 169) and/or Trp433→Phe (*e.g*. SEQ ID NO: 171). These mutations may be combined with C-terminal truncations *e.g.* to combine a Pro325→Leu mutation with a 7-mer truncation (*e.g.* SEQ ID NO: 170).

### (4) spr2021

The original 'spr2021' sequence was annotated in reference 84 as 'General stress protein GSP-781' (see GI:15904062). For reference purposes, the amino acid sequence of full length spr2021 as found in the R6 strain is given as SEQ ID NO: 11 herein.

Preferred spr2021 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 11; and/or (b) comprising a fragment of at least 'n' consecutive amino acids of SEQ ID NO: 11, wherein 'n' is 10 or more (*e.g.* 10, 12, 14, 16, 18, 20, 25, 30, 35, 40, 50, 60, 70, 80, 90, 100, 150, 200, 250 or more). These spr2021 proteins include variants of SEQ ID NO: 11. Preferred fragments of (b) comprise an epitope from SEQ ID NO: 11. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 11 while retaining at least one epitope of SEQ ID NO: 11. Other fragments omit one or more protein domains. One suitable fragment is SEQ ID NO: 190, which omits the natural leader peptide sequence.

Combinations of spr2021 with other pneumococcal antigens have shown good synergistic effects.

Reference 10 annotates spr2021 as a secreted 45kDa protein with homology to GbpB and discloses its use as an immunogen (SEQ ID NO: 243 therein; SP2216). Immunogenic fragments of spr2021 are identified in table 1 of reference 10 (page 73). Another useful fragment of spr2021 is disclosed as SEQ ID NO: 1 of reference 59 (amino acids 28-278 of SEQ ID NO: 11 herein).

### Third antigen group

### (1) spr0096

The original 'spr0096' sequence was annotated in reference 84 as 'hypothetical protein' (see GI:15902140). For reference purposes, the amino acid sequence of full length spr0096 as found in the R6 strain is given as SEQ ID NO: 12 herein.

Preferred spr0096 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 12; and/or (b) comprising a fragment of at least 'n' consecutive amino acids of SEQ ID NO: 12, wherein 'n' is 10 or more (*e.g.* 10, 12, 14, 16, 18, 20, 25, 30, 35, 40, 50, 60, 70, 80, 90, 100, 150, 200, 250 or more). These spr0096 proteins include variants of SEQ ID NO: 12 (*e.g.* SEQ ID NO: 40; see below). Preferred fragments of (b) comprise an epitope from SEQ ID NO: 12. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 12 while retaining at least one epitope of SEQ ID NO: 12. Other fragments omit one or more protein domains.

Combinations of spr0096 with other pneumococcal antigens have shown good synergistic effects.

A variant form of spr0096, with an insert near its C-terminus relative to SEQ ID NO: 12, is SEQ ID NO: 40 herein. The use of this variant for immunisation is reported in reference 10 (SEQ ID NO: 150 therein), where it is annotated as a LysM domain protein. Thus a spr0096 for use with the invention may comprise an amino acid sequence: (a) having 80% or more identity (*e.g.* 80%, 85%, 90%, 91%, 92%, 93%, 94%, 95%, 96%, 97%, 98%, 99%, 99.5% or more) to SEQ ID NO: 40; and/or (b) comprising a fragment of at least 'n' consecutive amino acids of SEQ ID NO: 40, wherein 'n' is 10 or more (*e.g.* 10, 12, 14, 16, 18, 20, 25, 30, 35, 40, 50, 60, 70, 80, 90, 100, 150, 200, 250 or more). These polypeptides include variants of SEQ ID NO: 40. Preferred fragments of (b) comprise an epitope from SEQ ID NO: 40. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 40 while retaining at least one epitope of SEQ ID NO: 40. Other fragments omit one or more protein domains. Immunogenic fragments of SEQID NO: 40 are identified in table 1 of reference 10.

A spr0096 polypeptide may be used in the form of a dimer *e.g.* a homodimer.

### (2) spr1433

The original 'spr1433' sequence was annotated in reference 84 as 'hypothetical protein' (see GI:15903476). For reference purposes, the amino acid sequence of full length spr1433 as found in the R6 strain is given as SEQ ID NO: 13 herein.

Preferred spr1433 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 13; and/or (b) comprising a fragment of at least 'n' consecutive amino acids of SEQ ID NO: 13, wherein 'n' is 10 or more (*e.g.* 10, 12, 14, 16, 18, 20, 25, 30, 35, 40, 50, 60, 70, 80, 90, 100, 150, 200, 250 or more). These spr1433 proteins include variants of SEQ ID NO: 13. Preferred fragments of (b) comprise an epitope from SEQ ID NO: 13. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 13 while retaining at least one epitope of SEQ ID NO: 13. Other fragments omit one or more protein domains.

### (3) spr1707

The original 'spr1707' sequence was annotated in reference 84 as 'ABC transporter substrate-binding protein - oligopeptide transport' (see GI:15903749). For reference purposes, the amino acid sequence of full length spr1707 as found in the R6 strain is given as SEQ ID NO: 14 herein. Preferred spr1707 polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 14; and/or (b) comprising a fragment of at least 'n' consecutive amino acids of SEQ ID NO: 14, wherein 'n' is 10 or more (*e.g.* 10, 12, 14, 16, 18, 20, 25, 30, 35, 40, 50, 60, 70, 80, 90, 100, 150, 200, 250 or more). These spr1707 proteins include variants of SEQ ID NO: 14 (*e.g.* SEQ ID NO: 100; see below). Preferred fragments of (b) comprise an epitope from SEQ ID NO: 14. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 14 while retaining at least one epitope of SEQ ID NO: 14. Other fragments omit one or more protein domains.

A variant form of spr1707, differing from SEQ ID NO: 14 by 4 amino acids, is SEQ ID NO: 100 herein. The use of SEQ ID NO: 100 for immunisation is reported in reference 10 (SEQ ID NO: 220 therein). Thus a spr1707 polypeptide for use with the invention may comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 100; and/or (b) comprising a fragment of at least 'n' consecutive amino acids of SEQ ID NO: 100, wherein 'n' is 10 or more (*e.g.* 10, 12, 14, 16, 18, 20, 25, 30, 35, 40, 50, 60, 70, 80, 90, 100, 150, 200, 250 or more). These polypeptides include variants of SEQ ID NO: 100. Preferred fragments of (b) comprise an epitope from SEQ ID NO: 100. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 100 while retaining at least one epitope of SEQ ID NO: 100. Other fragments omit one or more protein domains.

Immunogenic fragments of SEQ ID NO: 100 are identified in table 1 of reference 10.

### Other Pneumococcal antigens

### ClpP

ClpP is the ATP-dependent Clp protease proteolytic subunit. For reference purposes, the amino acid sequence of full length ClpP is SEQ ID NO: 16 herein. In the R6 genome ClpP is spr0656 [84].

The use of ClpP for immunisation is reported in references 60 and 61. It may advantageously be used in combination with PspA and PsaA and/or PspC [60].

### LytA

LytA is the N-acetylmuramoyl-L-alanine amidase (autolysin). For reference purposes, the amino acid sequence of full length LytA is SEQ ID NO: 17 herein. In the R6 genome LytA is spr1754 [84].

The use of LytA for immunisation is reported in reference 62, particularly in a form comprising the LytA choline binding domain fused to a heterologous promiscuous T helper epitope.

### PhtA

PhtA is the Pneumococcal histidine triad protein A. For reference purposes, the amino acid sequence of full length PhtA precursor is SEQ ID NO: 18 herein. In the R6 genome PhtA is spr1061 [84].

The use of PhtA for immunisation is reported in references 63 and 64. PhtB

PhtB is the pneumococcal histidine triad protein B. For reference purposes, the amino acid sequence of full length PhtB precursor is SEQ ID NO: 19 herein. Xaa at residue 578 can be Lysine.

The use of PhtB for immunisation is reported in reference 2, 63 and 64.

### Ph tD

PhtD is the Pneumococcal histidine triad protein D. For reference purposes, the amino acid sequence of full length PhtD precursor is SEQ ID NO: 20 herein. In the R6 genome PhtD is spr0907 [84].

The use of PhtD for immunisation is reported in references 63, 64 and 65.

### PhtE

PhtE is the Pneumococcal histidine triad protein E. For reference purposes, the amino acid sequence of full length PhtE precursor is SEQ ID NO: 21 herein. In the R6 genome PhtE is spr0908 [84].

The use of PhtE for immunisation is reported in references 63 and 64.

### ZmpB

ZmpB is the zinc metalloprotease. For reference purposes, the amino acid sequence of full length ZmpB is SEQ ID NO: 22 herein. In the R6 genome ZmpB is spr0581 [84].

### CbpD

CbpD is the Choline binding protein D. For reference purposes, the amino acid sequence of full length CbpD is SEQ ID NO: 23 herein. In the R6 genome CbpD is spr2006 [84].

The use of CbpD for immunisation is reported in reference 67. A variant of SEQ ID NO: 23 is SEQ ID NO: 119 herein. The use of SEQ ID NO: 119 for immunisation is reported in reference 10 (SEQ ID NO: 241 therein). Immunogenic fragments of SEQ ID NO: 119 are identified in table 1 of reference 10.

### CbpG

CbpG is the Choline binding protein G. For reference purposes, the amino acid sequence of full length CbpG is SEQ ID NO: 24 herein. In the R6 genome CbpG is spr0350 [84].

The use of CbpG for immunisation is reported in reference 67.

### PvaA

PvaA (Streptococcus pneumoniae pneumococcal vaccine antigen A) is also known as sp101. For reference purposes, the amino acid sequence of full length PvaA is SEQ ID NO: 25 herein. In the R6 genome PvaA is spr0930 [84].

The use of PvaA for immunisation is reported in references 1 and 318.

### CPL1

CPL1 is the pneumococcal phage CP1 lysozyme. For reference purposes, the amino acid sequence of full length CPL1 is SEQ ID NO: 26 herein.

The use of CPL1 for immunisation is reported in reference 62, particularly in a form comprising the CPL1 choline binding domain fused to a heterologous promiscuous T helper epitope.

### PspC

PspC is the pneumococcal surface protein C [66] and is also known as choline-binding protein A (CbpA). Its use for immunisation is reported in references 1 and 67. In the R6 strain it is spr1995 and, for reference, the amino acid sequence of full length spr1995 is SEQ ID NO: 15 herein.

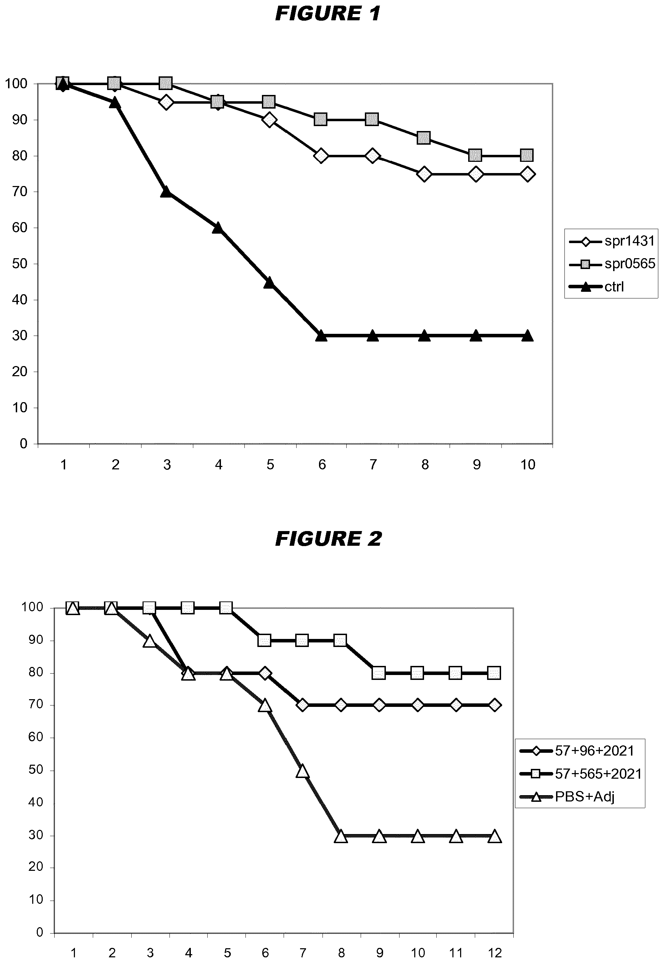

A variant of PspC is known as 'Hic'. It is similar to PspC, as shown in Figure 1 of reference 68, where it is reported to bind to factor H (fH). For reference purposes, the amino acid sequence of full length Hic is SEQ ID NO: 27 herein. A Hic protein may be used with the invention in addition to or in place of a PspC polypeptide.

PspC and/or Hic can advantageously be used in combination with PspA and/or PsaA.

### Pmp

Pmp is a peptidylprolyl isomerase, also known as protease maturation protein. For reference purposes, the amino acid sequence of full length Pmp is SEQ ID NO: 28 herein. In the R6 genome Pmp is spr0884 [84].

Preferred Pmp polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 28; and/or (b) comprising a fragment of at least 'n' consecutive amino acids of SEQ ID NO: 28, wherein 'n' is 10 or more (*e.g.* 10, 12, 14, 16, 18, 20, 25, 30, 35, 40, 50, 60, 70, 80, 90, 100, 150, 200, 250 or more). These Pmp proteins include variants of SEQ ID NO: 28. Preferred fragments of (b) comprise an epitope from SEQ ID NO: 28. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 28 while retaining at least one epitope of SEQ ID NO: 28. Other fragments omit one or more protein domains. One suitable fragment is SEQ ID NO: 186, which omits the natural leader peptide sequence.

The use of Pmp for immunisation is reported in reference 69.

### PspA

PspA is the Pneumococcal surface protein A. For reference purposes, the amino acid sequence of full length PspA is SEQ ID NO: 29 herein. In the R6 genome PspA is spr0121 [84]. The use of PspA for immunisation is reported *inter alia* in reference 70. It can advantageously be administered in combination with PspC.

### PsaA

PsaA is the Pneumococcal surface adhesin. For reference purposes, the amino acid sequence of full length PsaA is SEQ ID NO: 30 herein.

Other fragments omit one or more protein domains. A useful fragment of PsaA is disclosed as SEQ ID NO: 3 in reference 59 (corresponding to amino acids 21-309 of SEQ ID NO: 30 herein).

The use of PsaA for immunisation is reported in reference 71. It can be used in combination with PspA and/or PspC.

### PrtA

PrtA is the cell wall-associated serine proteinase. It has also been known as sp128 and sp130, and is in a subtilisin-like serine protease. For reference purposes, the amino acid sequence of full length PrtA precursor is SEQ ID NO: 31 herein. In the R6 genome PrtA is spr0561 [84].

The use of PrtA for immunisation is reported in references 72 & 73, and also in reference 1.

### Sp133

Sp133 is a conserved pneumococcal antigen. For reference purposes, the amino acid sequence of full length Sp133 is SEQ ID NO: 32 herein. In the R6 genome Sp133 is spr0931 [84].

The use of Sp133 for immunisation is reported in reference 74.

### PiaA

PiaA is the membrane permease involved in iron acquisition by pneumococcus. For reference purposes, the amino acid sequence of full length PiaA is SEQ ID NO: 33 herein. In the R6 genome PiaA is spr0935 [84].

The use of PiaA for immunisation is reported in references 75, 76 and 77, particularly in combination with PiuA.

### PiuA

PiuA is the ABC transporter substrate-binding protein for ferric iron transport. It is also known as FatB. For reference purposes, the amino acid sequence of full length PiuA is SEQ ID NO: 34 herein. In the R6 genome PiuA is spr1687 [84].

The use of PiuA for immunisation is reported in refs 75 to 77, particularly in combination with PiaA.

### IC1

IC1 is annotated in reference 10 as a hypothetical protein. For reference purposes, the amino acid sequence of full length IC1 is SEQ ID NO: 35 herein. In the R6 genome IC1 is spr0008 [84]. The use of IC1 for immunisation is reported in reference 10 (SEQ ID NO: 145 therein).

Other fragments omit one or more protein domains. Immunogenic fragments of IC1 are identified in table 1 of reference 10.

### IC2

IC2 is the polA DNA polymerase I. For reference purposes, the amino acid sequence of full length IC2 is SEQ ID NO: 36 herein. In the R6 genome IC2 is spr0032 [84]. The use of IC2 for immunisation is reported in reference 10 (SEQ ID NO: 146 therein). Immunogenic fragments of IC2 are identified in table 1 of reference 10.

### IC3

IC3 is a choline-binding protein. For reference purposes, the amino acid sequence of full length IC3 is SEQ ID NO: 37 herein. In the R6 genome IC3 is spr1945 [84]. The use of IC3 for immunisation is reported in reference 10 (SEQ ID NO: 147 therein).

Immunogenic fragments of IC3 are identified in table 1 of reference 10.

### IC4

IC4 is an IgA1 protease. For reference purposes, the amino acid sequence of full length IC4 is SEQ ID NO: 38 herein. In the R6 genome IC4 is spr1042 [84]. The use of IC4 for immunisation is reported in reference 10 (SEQ ID NO: 148 therein).

Immunogenic fragments of IC4 are identified in table 1 of reference 10.

### IC5

IC5 is annotated as a hypothetical protein, but is maybe a cell wall surface anchor. For reference purposes, the amino acid sequence of full length IC5 is SEQ ID NO: 39 herein. In the R6 genome IC5 is spr0075 [84]. The use of IC5 for immunisation is reported in reference 10 (SEQ ID NO: 149 therein).

Immunogenic fragments of IC5 are identified in table 1 of reference 10.

### IC6

IC6 is a variant form of spr0096, as reported above (SEQ ID NO: 40 herein).

### IC7

IC7 is annotated in reference 10 as a hypothetical protein. For reference purposes, the amino acid sequence of full length IC7 is SEQ ID NO: 41 herein. In the R6 genome IC7 is spr0174 [84]. The use of IC7 for immunisation is reported in reference 10 (SEQ ID NO: 152 therein). Immunogenic fragments of IC7 are identified in table 1 of reference 10.

### IC8

IC8 is a Dihydrofolate:folylpolyglutamate synthetase. For reference purposes, the amino acid sequence of full length IC8 is SEQ ID NO: 42 herein. In the R6 genome IC8 is spr0178 [84]. The use of IC8 for immunisation is reported in reference 10 (SEQ ID NO: 153 therein).

Immunogenic fragments of IC8 are identified in table 1 of reference 10.

### IC9

IC9 is a 50S ribosomal protein L2. For reference purposes, the amino acid sequence of full length IC9 is SEQ ID NO: 43 herein. In the R6 genome IC9 is spr0191 [84]. The use of IC9 for immunisation is reported in reference 10 (SEQ ID NO: 154 therein).

Immunogenic fragments of IC9 are identified in table 1 of reference 10.

### IC10

IC10 is a 30S Ribosomal protein S14. For reference purposes, the amino acid sequence of full length IC10 is SEQ ID NO: 44 herein. In the R6 genome IC10 is spr0202 [84]. The use of IC10 for immunisation is reported in reference 10 (SEQ ID NO: 155 therein).

Immunogenic fragments of IC10 are identified in table 1 of reference 10.

### IC11

IC11 is annotated in reference 10 as a hypothetical protein. For reference purposes, the amino acid sequence of full length IC11 is SEQ ID NO: 45 herein. In the R6 genome IC11 is spr0218 [84]. The use of IC11 for immunisation is reported in reference 10 (SEQ ID NO: 156 therein).

Immunogenic fragments of IC11 are identified in table 1 of reference 10. IC12
IC12 is a Formate acetyltransferase 3. For reference purposes, the amino acid sequence of full length
IC12 is SEQ ID NO: 46 herein. In the R6 genome IC12 is spr0232 [84]. The use of IC12 for immunisation is reported in reference 10 (SEQ ID NO: 157 therein).

Immunogenic fragments of IC12 are identified in table 1 of reference 10.

### IC13

IC13 is a 30S ribosomal protein S9. For reference purposes, the amino acid sequence of full length IC13 is SEQ ID NO: 47 herein. In the R6 genome IC13 is spr0272 [84]. The use of IC13 for immunisation is reported in reference 10 (SEQ ID NO: 158 therein).

Immunogenic fragments of IC 13 are identified in table 1 of reference 10.

### IC14

IC14 is a Transcription regulator. For reference purposes, the amino acid sequence of full length IC14 is SEQ ID NO: 48 herein. In the R6 genome IC14 is spr0298 [84]. The use of IC14 for immunisation is reported in reference 10 (SEQ ID NO: 159 therein).

Immunogenic fragments of IC 14 are identified in table 1 of reference 10.

### IC15

IC15 is annotated in reference 10 as a cell wall surface anchor family protein. For reference purposes, the amino acid sequence of full length IC15 is SEQ ID NO: 49 herein. In the R6 genome IC15 is spr0328 [84]. The use of IC15 for immunisation is reported in reference 10 (SEQ ID NO: 160 therein), and it is shown to be protective in reference 78 (antigen SP0368).

Immunogenic fragments ofIC15 are identified in table 1 of reference 10.

### IC16

IC16 is a Penicillin-binding protein 1A. For reference purposes, the amino acid sequence of full length IC16 is SEQ ID NO: 50 herein. In the R6 genome IC16 is spr0329 [84]. The use of IC16 for immunisation is reported in reference 10 (SEQ ID NO: 161 therein).

Immunogenic fragments of IC16 are identified in table 1 of reference 10.

### IC17

IC17 is annotated in reference 10 as a hypothetical protein. For reference purposes, the amino acid sequence of full length IC17 is SEQ ID NO: 51 herein. In the R6 genome IC17 is spr0334 [84]. The use of IC 17 for immunisation is reported in reference 10 (SEQ ID NO: 162 therein).

Immunogenic fragments of IC 17 are identified in table 1 of reference 10.

### IC18

IC18 is annotated in reference 10 as choline-binding protein F. For reference purposes, the amino acid sequence of full length IC18 is SEQ ID NO: 52 herein. In the R6 genome IC18 is spr0337 [84]. The use of IC18 for immunisation is reported in reference 10 (SEQ ID NO: 163 therein).

Immunogenic fragments of IC18 are identified in table 1 of reference 10. IC19
IC19 is annotated in reference 10 as a choline-binding protein J (cbpJ). For reference purposes, the amino acid sequence of full length IC19 is SEQ ID NO: 53 herein. The use of IC19 for immunisation is reported in reference 10 (SEQ ID NO: 164 therein).

Immunogenic fragments of IC 19 are identified in table 1 of reference 10.

### IC20

IC20 is a choline binding protein G. For reference purposes, the amino acid sequence of full length IC20 is SEQ ID NO: 54 herein. In the R6 genome IC20 is spr0349 [84]. The use of IC20 for immunisation is reported in reference 10 (SEQ ID NO: 165 therein).

Immunogenic fragments of IC20 are identified in table 1 of reference 10.

### IC21

IC21 is annotated in reference 10 as a hypothetical protein. For reference purposes, the amino acid sequence of full length IC21 is SEQ ID NO: 55 herein. In the R6 genome IC21 is spr0410 [84]. The use of IC21 for immunisation is reported in reference 10 (SEQ ID NO: 166 therein).

Immunogenic fragments of IC21 are identified in table 1 of reference 10.

### IC22

IC22 is annotated in reference 10 as cell wall surface anchor family protein. For reference purposes, the amino acid sequence of full length IC22 is SEQ ID NO: 56 herein. In the R6 genome IC22 is spr0051 [84]. The use of IC22 for immunisation is reported in reference 10 (SEQ ID NO: 167 therein).

Immunogenic fragments of IC22 are identified in table 1 of reference 10.

### IC23

IC23 is a Sortase (*cf.* spr1098). For reference purposes, the amino acid sequence of full length IC23 is SEQ ID NO: 57 herein. The use of IC23 for immunisation is reported in reference 10 (SEQ ID NO: 168 therein).

Immunogenic fragments of IC23 are identified in table 1 of reference 10.

### IC24

IC24 is a Sortase (*cf* spr1098). For reference purposes, the amino acid sequence of full length IC24 is SEQ ID NO: 58 herein. The use of IC24 for immunisation is reported in reference 10 (SEQ ID NO: 169 therein).

Immunogenic fragments of IC24 are identified in table 1 of reference 10.

### IC25

IC25 is annotated in reference 10 as a putative endo-β-N-acetylglucosaminidase. For reference purposes, the amino acid sequence of full length IC25 is SEQ ID NO: 59 herein. In the R6 genome IC25 is spr0440 [84]. The use of IC25 for immunisation is reported in reference 10 (SEQ ID NO: 170 therein).

Immunogenic fragments of IC25 are identified in table 1 of reference 10. IC26
IC26 is a EcoE type I restriction modification enzyme. For reference purposes, the amino acid sequence of full length IC26 is SEQ ID NO: 60 herein. In the R6 genome IC26 is spr0449 [84]. The use of IC26 for immunisation is reported in reference 10 (SEQ ID NO: 171 therein).

Immunogenic fragments of IC26 are identified in table 1 of reference 10.

### IC27

IC27 is annotated in reference 10 as dnaJ protein. For reference purposes, the amino acid sequence of full length IC27 is SEQ ID NO: 61 herein. In the R6 genome IC27 is spr0456 [84]. The use of IC27 for immunisation is reported in reference 10 (SEQ ID NO: 172 therein).

Immunogenic fragments of IC27 are identified in table 1 of reference 10.

### IC28

IC28 is annotated in reference 10 as a BlpC ABC transporter (blpB). For reference purposes, the amino acid sequence of full length IC28 is SEQ ID NO: 62 herein. In the R6 genome IC28 is spr0466 [84]. The use of IC28 for immunisation is reported in reference 10 (SEQ ID NO: 173 therein).

Immunogenic fragments of IC28 are identified in table 1 of reference 10.

### IC29

IC29 is annotated in reference 10 as a hypothetical protein. For reference purposes, the amino acid sequence of full length IC29 is SEQ ID NO: 63 herein. In the R6 genome IC29 is spr0488 [84]. The use of IC29 for immunisation is reported in reference 10 (SEQ ID NO: 174 therein).

Immunogenic fragments of IC29 are identified in table 1 of reference 10.

### IC30

IC30 is a ABC transporter substrate-binding protein. For reference purposes, the amino acid sequence of full length IC30 is SEQ ID NO: 64 herein. In the R6 genome IC30 is spr0534 [84]. The use of IC30 for immunisation is reported in reference 10 (SEQ ID NO: 175 therein).

Immunogenic fragments of IC30 are identified in table 1 of reference 10.

### IC31

IC31 is annotated in reference 10 as a metallo-β-lactamase superfamily protein. For reference purposes, the amino acid sequence of full length IC31 is SEQ ID NO: 65 herein. In the R6 genome IC31 is spr0538 [84]. The use of IC31 for immunisation is reported in reference 10 (SEQ ID NO: 176 therein).

Immunogenic fragments of IC31 are identified in table 1 of reference 10.

### IC32

IC32 is a variant form of spr0565, as mentioned above (SEQ ID NO: 66 herein). Immunogenic fragments of SEQ ID NO: 66 are identified in table 1 of reference 10.

### IC33

IC33 is annotated in reference 10 as a putative pneumococcal surface protein. For reference purposes, the amino acid sequence of full length IC33 is SEQ ID NO: 67 herein. In the R6 genome IC33 is spr0583 [84]. The use of IC33 for immunisation is reported in reference 10 (SEQ ID NO: 180 therein) and it is shown to be protective in reference 78 (antigen SP0667). Immunogenic fragments ofIC33 are identified in table 1 of reference 10.

### IC34

IC34 is a UDP-N-acetylmuramoyl-L-alanyl-D-glutamate synthetase. For reference purposes, the amino acid sequence of full length IC34 is SEQ ID NO: 68 herein. In the R6 genome IC34 is spr0603 [84]. The use of IC34 for immunisation is reported in reference 10 (SEQ ID NO: 181 therein).

Immunogenic fragments of IC34 are identified in table 1 of reference 10.

### IC35

IC35 is a ABC transporter substrate-binding protein. For reference purposes, the amino acid sequence of full length IC35 is SEQ ID NO: 69 herein. In the R6 genome IC35 is spr0659 [84]. The use of IC35 for immunisation is reported in reference 10 (SEQ ID NO: 182 therein).

Immunogenic fragments of IC35 are identified in table 1 of reference 10.

### IC36

IC36 is a ABC transporter ATP-binding protein. For reference purposes, the amino acid sequence of full length IC36 is SEQ ID NO: 70 herein. In the R6 genome IC36 is spr0678 [84]. The use of IC36 for immunisation is reported in reference 10 (SEQ ID NO: 183 therein).

Immunogenic fragments of IC36 are identified in table 1 of reference 10.

### IC37

IC37 is annotated in reference 10 as a hypothetical protein. For reference purposes, the amino acid sequence of full length IC37 is SEQ ID NO: 71 herein. In the R6 genome IC37 is spr0693 [84]. The use of IC37 for immunisation is reported in reference 10 (SEQ ID NO: 184 therein).

Immunogenic fragments of IC37 are identified in table 1 of reference 10. IC38
IC38 is annotated in reference 10 as a nodulin-related protein with truncation. For reference purposes, the amino acid sequence of full length IC38 is SEQ ID NO: 72 herein. In the R6 genome IC38 is spr0814 [84]. The use of IC38 for immunisation is reported in reference 10 (SEQ ID NO: 185 therein).

Immunogenic fragments of IC38 are identified in table 1 of reference 10.

### IC39

IC39 is a Teichoic acid phosphorylcholine esterase/choline binding protein E (cbpE). It may also be known as 'LytD'. For reference purposes, the amino acid sequence of full length IC39 is SEQ ID NO: 73 herein. In the R6 genome IC39 is spr0831 [84]. The use of IC39 for immunisation is reported in reference 10 (SEQ ID NO: 186 therein).

Immunogenic fragments of IC39 are identified in table 1 of reference 10.

### IC40

IC40 is a glucose-inhibited division protein A. For reference purposes, the amino acid sequence of full length IC40 is SEQ ID NO: 74 herein. In the R6 genome IC40 is spr0844 [84]. The use of IC40 for immunisation is reported in reference 10 (SEQ ID NO: 187 therein). Immunogenic fragments of IC40 are identified in table 1 of reference 10.

### IC41

IC41 is a Alanine dehydrogenase, truncation. For reference purposes, the amino acid sequence of full length IC41 is SEQ ID NO: 75 herein. In the R6 genome IC41 is spr0854 [84]. The use of IC41 for immunisation is reported in reference 10 (SEQ ID NO: 188 therein).

Immunogenic fragments of IC41 are identified in table 1 of reference 10.

### IC42

IC42 is a glycogen syntase. For reference purposes, the amino acid sequence of full length IC42 is SEQ ID NO: 76 herein. In the R6 genome IC42 is spr1032 [84]. The use of IC42 for immunisation is reported in reference 10 (SEQ ID NO: 191 therein).

Immunogenic fragments of IC42 are identified in table 1 of reference 10.

### IC43

IC43 is a Immunoglobulin A1 protease. For reference purposes, the amino acid sequence of full length IC43 is SEQ ID NO: 77 herein. The use of IC43 for immunisation is reported in reference 10 (SEQ ID NO: 192 therein).

Immunogenic fragments of IC43 are identified in table 1 of reference 10.

### IC44

IC44 is a Uncharacterized restriction enzyme. For reference purposes, the amino acid sequence of full length IC44 is SEQ ID NO: 78 herein. In the R6 genome IC44 is spr1101 [84]. The use of IC44 for immunisation is reported in reference 10 (SEQ ID NO: 195 therein).

Immunogenic fragments of IC44 are identified in table 1 of reference 10. IC45
IC45 is a Response regulator. For reference purposes, the amino acid sequence of full length IC45 is SEQ ID NO: 79 herein. In the R6 genome IC45 is spr1107 [84]. The use of IC45 for immunisation is reported in reference 10 (SEQ ID NO: 196 therein).

Immunogenic fragments of IC45 are identified in table 1 of reference 10.

### IC46

IC46 is a ABC transporter membrane spanning permease. For reference purposes, the amino acid sequence of full length IC46 is SEQ ID NO: 80 herein. In the R6 genome IC46 is spr1120 [84]. The use of IC46 for immunisation is reported in reference 10 (SEQ ID NO: 197 therein).

Immunogenic fragments ofIC46 are identified in table 1 of reference 10.

### IC47

IC47 is a Signal recognition particle. For reference purposes, the amino acid sequence of full length IC47 is SEQ ID NO: 81 herein. In the R6 genome IC47 is spr1166 [84]. The use of IC47 for immunisation is reported in reference 10 (SEQ ID NO: 198 therein).

Immunogenic fragments of IC47 are identified in table 1 of reference 10.

### IC48

IC48 is a N-acetylmannosamine-6-phosphate 2-epimerase. For reference purposes, the amino acid sequence of full length IC48 is SEQ ID NO: 82 herein. In the R6 genome IC48 is spr1529 [84]. The use of IC48 for immunisation is reported in reference 10 (SEQ ID NO: 199 therein).

Immunogenic fragments of IC48 are identified in table 1 of reference 10.

### IC49

IC49 is a chorismate synthase. For reference purposes, the amino acid sequence of full length IC49 is SEQ ID NO: 83 herein. In the R6 genome IC49 is spr1232 [84]. The use of IC49 for immunisation is reported in reference 10 (SEQ ID NO: 200 therein).

Immunogenic fragments of IC49 are identified in table 1 of reference 10.

### IC50

IC50 is annotated in reference 10 as a hypothetical protein. For reference purposes, the amino acid sequence of full length IC50 is SEQ ID NO: 84 herein. In the R6 genome IC50 is spr1236 [84]. The use of IC50 for immunisation is reported in reference 10 (SEQ ID NO: 201 therein).

Immunogenic fragments of IC50 are identified in table 1 of reference 10.

### IC51

IC51 is a Protease. For reference purposes, the amino acid sequence of full length IC51 is SEQ ID NO: 85 herein. In the R6 genome IC51 is spr1284 [84]. The use of IC51 for immunisation is reported in reference 10 (SEQ ID NO: 202 therein).

Immunogenic fragments of IC51 are identified in table 1 of reference 10.

### IC52

IC52 is a annotated in reference 10 as an oxidoreductase or aldo/keto reductase. For reference purposes, the amino acid sequence of full length IC52 is SEQ ID NO: 86 herein. In the R6 genome IC52 is spr1332 [84]. The use of IC52 for immunisation is reported in reference 10 (SEQ ID NO: 203 therein).

Immunogenic fragments of IC52 are identified in table 1 of reference 10.

### IC53

IC53 is annotated in reference 10 as a hypothetical protein. For reference purposes, the amino acid sequence of full length IC53 is SEQ ID NO: 87 herein. In the R6 genome IC53 is spr1370 [84]. The use of IC53 for immunisation is reported in reference 10 (SEQ ID NO: 204 therein).

Immunogenic fragments of IC53 are identified in table 1 of reference 10.

### IC54

IC54 is annotated as a conserved domain protein. For reference purposes, the amino acid sequence of full length IC54 is SEQ ID NO: 88 herein. In the R6 genome IC54 is spr1374 [84]. The use of IC54 for immunisation is reported in reference 10 (SEQ ID NO: 205 therein).

Immunogenic fragments of IC54 are identified in table 1 of reference 10.

### IC55

IC55 is a ABC transporter substrate-binding protein. For reference purposes, the amino acid sequence of full length IC55 is SEQ ID NO: 89 herein. In the R6 genome IC55 is spr1382 [84]. The use of IC55 for immunisation is reported in reference 10 (SEQ ID NO: 206 therein).

Immunogenic fragments of IC55 are identified in table 1 of reference 10.

### IC56

IC56 is annotated in reference 10 as a hypothetical protein. For reference purposes, the amino acid sequence of full length IC56 is SEQ ID NO: 90 herein. In the R6 genome IC56 is spr1457 [84]. The use of IC56 for immunisation is reported in reference 10 (SEQ ID NO: 208 therein).

Immunogenic fragments of IC56 are identified in table 1 of reference 10. IC57

IC57 is a Cell-division initiation protein. For reference purposes, the amino acid sequence of full length IC57 is SEQ ID NO: 91 herein. In the R6 genome IC57 is spr1505 [84]. The use of IC57 for immunisation is reported in reference 10 (SEQ ID NO: 209 therein).

Immunogenic fragments of IC57 are identified in table 1 of reference 10.

### IC58

IC58 is annotated in reference 10 as ylmF protein. For reference purposes, the amino acid sequence of full length IC58 is SEQ ID NO: 92 herein. In the R6 genome IC58 is spr1508 [84]. The use of IC58 for immunisation is reported in reference 10 (SEQ ID NO: 210 therein).

Immunogenic fragments of IC58 are identified in table 1 of reference 10.

### IC59

IC59 is a N-acetylneuraminate lyase subunit. For reference purposes, the amino acid sequence of full length IC59 is SEQ ID NO: 93 herein. In the R6 genome IC59 is spr1186 [84]. The use of IC59 for immunisation is reported in reference 10 (SEQ ID NO: 211 therein).

Immunogenic fragments of IC59 are identified in table 1 of reference 10.

### IC60

IC60 is a Eukaryotic-type serine/threonine kinase (StkP). For reference purposes, the amino acid sequence of full length IC60 is SEQ ID NO: 94 herein. In the R6 genome IC60 is spr1577 [84]. The use of IC60 for immunisation is reported in reference 10 (SEQ ID NO: 214 therein), and it is reported to be a lead vaccine candidate in reference 78.

Immunogenic fragments of IC60 are identified in table 1 of reference 10. A further useful fragment is disclosed as SEQ ID NO: 2 in reference 59 (corresponding to amino acids 345-659 of SEQ ID NO: 94 herein).

### IC61

IC61 is a methionyl-tRNA formyltransferase. For reference purposes, the amino acid sequence of full length IC61 is SEQ ID NO: 95 herein. In the R6 genome IC61 is spr1580 [84]. The use of IC61 for immunisation is reported in reference 10 (SEQ ID NO: 215 therein).

Immunogenic fragments of IC61 are identified in table 1 of reference 10.

### IC62

IC62 is a translocase. For reference purposes, the amino acid sequence of full length IC62 is SEQ ID NO: 96 herein. In the R6 genome IC62 is spr1544 [84]. The use of IC62 for immunisation is reported in reference 10 (SEQ ID NO: 216 therein).

Immunogenic fragments of IC62 are identified in table 1 of reference 10.

### IC63

IC63 is annotated in reference 10 as a cell wall surface anchor family protein. For reference purposes, the amino acid sequence of full length IC63 is SEQ ID NO: 97 herein. In the R6 genome IC63 is spr1403 [84]. The use of IC63 for immunisation is reported in reference 10 (SEQ ID NO: 217 therein).

Immunogenic fragments of IC63 are identified in table 1 of reference 10. *IC64*
IC64 is annotated in reference 10 as a putative general stress protein 24. For reference purposes, the amino acid sequence of full length IC64 is SEQ ID NO: 98 herein. In the R6 genome IC64 is spr1625 [84]. The use of IC64 for immunisation is reported in reference 10 (SEQ ID NO: 218 therein).

Immunogenic fragments of IC64 are identified in table 1 of reference 10.

### IC65

IC65 is a ABC transporter ATP-binding protein. For reference purposes, the amino acid sequence of full length IC65 is SEQ ID NO: 99 herein. In the R6 genome IC65 is spr1704 [84]. The use of IC65 for immunisation is reported in reference 10 (SEQ ID NO: 219 therein).

Immunogenic fragments of IC65 are identified in table 1 of reference 10.

### IC66

IC66 is, as mentioned above, a variant form of spr1707. Useful IC66 polypeptides for use with the invention comprise an amino acid sequence: (a) having 80% or more identity (*e.g.* 80%, 85%, 90%, 91%, 92%, 93%, 94%, 95%, 96%, 97%, 98%, 99%, 99.5% or more) to SEQ ID NO: 100; and/or (b) comprising a fragment of at least 'n' consecutive amino acids of SEQ ID NO: 100, wherein 'n' is 10 or more (*e.g.* 10, 12, 14, 16, 18, 20, 25, 30, 35, 40, 50, 60, 70, 80, 90, 100, 150, 200, 250 or more). These IC66 proteins include variants of SEQ ID NO: 100. Preferred fragments of (b) comprise an epitope from SEQ ID NO: 100. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 100 while retaining at least one epitope of SEQ ID NO: 100. Other fragments omit one or more protein domains.

### IC67

IC67 is a Subtilisin-like serine protease. For reference purposes, the amino acid sequence of full length IC67 is SEQ ID NO: 101 herein. In the R6 genome IC67 is spr1771 [84]. The use of IC67 for immunisation is reported in reference 10 (SEQ ID NO: 222 therein).

Immunogenic fragments of IC67 are identified in table 1 of reference 10.

### IC68

IC68 is a Cmp-binding-factor 1. For reference purposes, the amino acid sequence of full length IC68 is SEQ ID NO: 102 herein. In the R6 genome IC68 is spr1794 [84]. The use of IC68 for immunisation is reported in reference 10 (SEQ ID NO: 223 therein).

Immunogenic fragments of IC68 are identified in table 1 of reference 10. IC69
IC69 is annotated in reference 10 as cell wall surface anchor family protein. For reference purposes, the amino acid sequence of full length IC69 is SEQ ID NO: 103 herein. In the R6 genome IC69 is spr1806 [84]. The use of IC69 for immunisation is reported in reference 10 (SEQ ID NO: 224 therein).

Immunogenic fragments of IC69 are identified in table 1 of reference 10.

### IC70

IC70 is a Catabolite control protein A. For reference purposes, the amino acid sequence of full length IC70 is SEQ ID NO: 104 herein. In the R6 genome IC70 is spr1813 [84]. The use of IC70 for immunisation is reported in reference 10 (SEQ ID NO: 225 therein).

Immunogenic fragments of IC70 are identified in table 1 of reference 10.

### IC71

IC71 is a Beta-glucosidase. For reference purposes, the amino acid sequence of full length IC71 is SEQ ID NO: 105 herein. In the R6 genome IC71 is spr1833 [84]. The use of IC71 for immunisation is reported in reference 10 (SEQ ID NO: 226 therein). Immunogenic fragments of IC71 are identified in table 1 of reference 10.

### IC72

IC72 is annotated in reference 10 as a hypothetical protein. For reference purposes, the amino acid sequence of full length IC72 is SEQ ID NO: 106 herein. In the R6 genome IC72 is spr1838 [84]. The use of IC72 for immunisation is reported in reference 10 (SEQ ID NO: 227 therein).

Immunogenic fragments of IC72 are identified in table 1 of reference 10.

### IC73

IC73 is annotated in reference 10 as a hypothetical protein. For reference purposes, the amino acid sequence of full length IC73 is SEQ ID NO: 107 herein. In the R6 genome IC73 is spr1850 [84]. The use of IC73 for immunisation is reported in reference 10 (SEQ ID NO: 228 therein).

Immunogenic fragments of IC73 are identified in table 1 of reference 10.

### IC74

IC74 is annotated in reference 10 as a hypothetical protein. For reference purposes, the amino acid sequence of full length IC74 is SEQ ID NO: 108 herein. In the R6 genome IC74 is spr1859 [84]. The use of IC74 for immunisation is reported in reference 10 (SEQ ID NO: 229 therein).

Immunogenic fragments of IC74 are identified in table 1 of reference 10.

### IC75

IC75 is a Competence protein. For reference purposes, the amino acid sequence of full length IC75 is SEQ ID NO: 109 herein. In the R6 genome IC75 is spr1862 [84]. The use of IC75 for immunisation is reported in reference 10 (SEQ ID NO: 230 therein).

Immunogenic fragments of IC75 are identified in table 1 of reference 10. IC76
IC76 is a UTP-glucose-1-phosphate uridylyltransferase. For reference purposes, the amino acid sequence of full length IC76 is SEQ ID NO: 110 herein. In the R6 genome IC76 is spr1903 [84]. The use of IC76 for immunisation is reported in reference 10 (SEQ ID NO: 231 therein).

Immunogenic fragments of IC76 are identified in table 1 of reference 10.

### IC77

IC77 is a Penicillin-binding protein 1b. For reference purposes, the amino acid sequence of full length IC77 is SEQ ID NO: 111 herein. In the R6 genome IC77 is spr1909 [84]. The use of IC77 for immunisation is reported in reference 10 (SEQ ID NO: 232 therein).

Immunogenic fragments of IC77 are identified in table 1 of reference 10.

### IC78

IC78 is a ABC transporter substrate-binding protein- maltose/maltodextrin. For reference purposes, the amino acid sequence of full length IC78 is SEQ ID NO: 112 herein. In the R6 genome IC78 is spr1918 [84]. The use of IC78 for immunisation is reported in reference 10 (SEQ ID NO: 233 therein). Immunogenic fragments of IC78 are identified in table 1 of reference 10.

### IC79

IC79 is annotated in reference 10 as a hypothetical protein. For reference purposes, the amino acid sequence of full length IC79 is SEQ ID NO: 113 herein. In the R6 genome IC79 is spr2120 [84]. The use of IC79 for immunisation is reported in reference 10 (SEQ ID NO: 234 therein).

Immunogenic fragments of IC79 are identified in table 1 of reference 10.

### IC80

IC80 is a Putative transketolase n-terminal section. For reference purposes, the amino acid sequence of full length IC80 is SEQ ID NO: 114 herein. In the R6 genome IC80 is spr1937 [84]. The use of IC80 for immunisation is reported in reference 10 (SEQ ID NO: 235 therein).

Immunogenic fragments of IC80 are identified in table 1 of reference 10.

### IC81

IC81 is a Choline-binding protein. For reference purposes, the amino acid sequence of full length IC81 is SEQ ID NO: 115 herein. Its C-terminus is related to IC3. The use of IC81 for immunisation is reported in reference 10 (SEQ ID NO: 236 therein).

Immunogenic fragments of IC81 are identified in table 1 of reference 10.

### IC82

IC82 is a glycosyl hydrolase-related protein. For reference purposes, the amino acid sequence of full length IC82 is SEQ ID NO: 116 herein. In the R6 genome IC82 is spr2141 [84]. The use of IC82 for immunisation is reported in reference 10 (SEQ ID NO: 237 therein).

Immunogenic fragments of IC82 are identified in table 1 of reference 10. *IC83*
IC83 is annotated in reference 10 as a hypothetical protein. For reference purposes, the amino acid sequence of full length IC83 is SEQ ID NO: 117 herein. In the R6 genome IC83 is spr1983 [84]. The use of IC83 for immunisation is reported in reference 10 (SEQ ID NO: 238 therein).

Immunogenic fragments of IC83 are identified in table 1 of reference 10.

### IC84

IC84 is a Class III stress response-related ATPase. For reference purposes, the amino acid sequence of full length IC84 is SEQ ID NO: 118 herein. In the R6 genome IC84 is spr2000 [84]. The use of IC84 for immunisation is reported in reference 10 (SEQ ID NO: 240 therein).

Immunogenic fragments of IC84 are identified in table 1 of reference 10.

### IC85

IC85 is a variant of SEQ ID NO: 23, mentioned above (SEQ ID NO: 119). Other fragments omit one or more protein domains.

### IC86

IC86 is a 50S ribosomal protein L9. For reference purposes, the amino acid sequence of full length IC86 is SEQ ID NO: 120 herein. In the R6 genome IC86 is spr2009 [84]. The use of IC86 for immunisation is reported in reference 10 (SEQ ID NO: 242 therein).

Immunogenic fragments of IC86 are identified in table 1 of reference 10.

### IC87

IC87 is annotated in reference 10 as a hypothetical protein. For reference purposes, the amino acid sequence of full length IC87 is SEQ ID NO: 166 herein. In the R6 genome IC87 is spr0987 [84]. The use of IC87 for immunisation is reported in reference 10 (SEQ ID NO: 288 therein).

Immunogenic fragments of IC87 are identified in table 1 of reference 10. *IC88*
IC88 is a Choline binding protein. For reference purposes, the amino acid sequence of full length IC88 is SEQ ID NO: 122 herein. In the R6 genome IC88 is spr1274 [84]. The use of IC88 for immunisation is reported in reference 10 (SEQ ID NO: 244 therein).

Immunogenic fragments of IC88 are identified in table 1 of reference 10.

### IC89

IC89 is annotated in reference 10 as a hypothetical protein. For reference purposes, the amino acid sequence of full length IC89 is SEQ ID NO: 123 herein. The use of IC89 for immunisation is reported in reference 10 (SEQ ID NO: 245 therein).

Immunogenic fragments of IC89 are identified in table 1 of reference 10.

### IC90

IC90 is annotated in reference 10 as a hypothetical protein. For reference purposes, the amino acid sequence of full length IC90 is SEQ ID NO: 124 herein. The use of IC90 for immunisation is reported in reference 10 (SEQ ID NO: 246 therein).

Immunogenic fragments of IC90 are identified in table 1 of reference 10.

### IC91

IC91 is annotated in reference 10 as a hypothetical protein. For reference purposes, the amino acid sequence of full length IC91 is SEQ ID NO: 125 herein. In the R6 genome IC91 is spr0415 [84]. The use of IC91 for immunisation is reported in reference 10 (SEQ ID NO: 247 therein).

Immunogenic fragments of IC91 are identified in table 1 of reference 10.

### IC92

IC92 is annotated in reference 10 as a hypothetical protein. For reference purposes, the amino acid sequence of full length IC92 is SEQ ID NO: 126 herein. In the R6 genome IC92 is spr0695 [84]. The use of IC92 for immunisation is reported in reference 10 (SEQ ID NO: 248 therein).

Immunogenic fragments of IC92 are identified in table 1 of reference 10.

### IC93

IC93 is annotated in reference 10 as a hypothetical protein. For reference purposes, the amino acid sequence of full length IC93 is SEQ ID NO: 127 herein. In the R6 genome IC93 is spr1334 [84]. The use of IC93 for immunisation is reported in reference 10 (SEQ ID NO: 249 therein).

Immunogenic fragments of IC93 are identified in table 1 of reference 10.

### IC94

IC94 is annotated in reference 10 as a hypothetical protein. For reference purposes, the amino acid sequence of full length IC94 is SEQ ID NO: 128 herein. In the R6 genome IC94 is spr0242 [84]. The use of IC94 for immunisation is reported in reference 10 (SEQ ID NO: 250 therein).

Immunogenic fragments of IC94 are identified in table 1 of reference 10.

### IC95

IC95 is annotated in reference 10 as a hypothetical protein. For reference purposes, the amino acid sequence of full length IC95 is SEQ ID NO: 129 herein. In the R6 genome IC95 is spr1367 [84]. The use of IC95 for immunisation is reported in reference 10 (SEQ ID NO: 251 therein). Immunogenic fragments of IC95 are identified in table 1 of reference 10.

### IC96

IC96 is annotated in reference 10 as a hypothetical protein. For reference purposes, the amino acid sequence of full length IC96 is SEQ ID NO: 130 herein. The use of IC96 for immunisation is reported in reference 10 (SEQ ID NO: 252 therein).

Immunogenic fragments of IC96 are identified in table 1 of reference 10.

### IC97

IC97 is annotated in reference 10 as a hypothetical protein. For reference purposes, the amino acid sequence of full length IC97 is SEQ ID NO: 131 herein. In the R6 genome IC97 is spr1502 [84]. The use of IC97 for immunisation is reported in reference 10 (SEQ ID NO: 253 therein).

Immunogenic fragments of IC97 are identified in table 1 of reference 10.

### IC98

IC98 is annotated in reference 10 as a hypothetical protein. For reference purposes, the amino acid sequence of full length IC98 is SEQ ID NO: 132 herein. In the R6 genome IC98 is spr0730 [84]. The use of IC98 for immunisation is reported in reference 10 (SEQ ID NO: 254 therein).

Immunogenic fragments ofIC98 are identified in table 1 of reference 10.

### IC99

IC99 is annotated in reference 10 as a hypothetical protein. For reference purposes, the amino acid sequence of full length IC99 is SEQ ID NO: 133 herein. In the R6 genome IC99 is spr1961 [84]. The use of IC99 for immunisation is reported in reference 10 (SEQ ID NO: 255 therein).

Immunogenic fragments of IC99 are identified in table 1 of reference 10. *IC100*
IC100 is annotated in reference 10 as a hypothetical protein. For reference purposes, the amino acid sequence of full length IC100 is SEQ ID NO: 134 herein. The use of IC100 for immunisation is reported in reference 10 (SEQ ID NO: 256 therein).

Immunogenic fragments of IC100 are identified in table 1 of reference 10.

### IC101

IC101 is annotated in reference 10 as a hypothetical protein. For reference purposes, the amino acid sequence of full length IC101 is SEQ ID NO: 135 herein. In the R6 genome IC101 is spr0516 [84]. The use of IC101 for immunisation is reported in reference 10 (SEQ ID NO: 257 therein).

Immunogenic fragments of IC101 are identified in table 1 of reference 10.

### IC102

IC102 is annotated in reference 10 as a hypothetical protein. For reference purposes, the amino acid sequence of full length IC102 is SEQ ID NO: 136 herein. In the R6 genome IC102 is spr1785 [84]. The use of IC102 for immunisation is reported in reference 10 (SEQ ID NO: 258 therein).

Immunogenic fragments of IC102 are identified in table 1 of reference 10.

### IC103

IC103 is annotated in reference 10 as a hypothetical protein. For reference purposes, the amino acid sequence of full length IC103 is SEQ ID NO: 137 herein. In the R6 genome IC103 is spr0215 [84]. The use of IC103 for immunisation is reported in reference 10 (SEQ ID NO: 259 therein).

Immunogenic fragments of IC103 are identified in table 1 of reference 10.

### IC104

IC104 is annotated in reference 10 as a hypothetical protein. For reference purposes, the amino acid sequence of full length IC104 is SEQ ID NO: 138 herein. In the R6 genome IC104 is spr1815 [84]. The use of IC104 for immunisation is reported in reference 10 (SEQ ID NO: 260 therein).

Immunogenic fragments of IC104 are identified in table 1 of reference 10.

### IC105

IC105 is annotated in reference 10 as a hypothetical protein. For reference purposes, the amino acid sequence of full length IC105 is SEQ ID NO: 139 herein. In the R6 genome IC105 is spr0102 [84]. The use of IC105 for immunisation is reported in reference 10 (SEQ ID NO: 261 therein).

Immunogenic fragments of IC105 are identified in table 1 of reference 10.

### IC106

IC106 is annotated in reference 10 as a hypothetical protein. For reference purposes, the amino acid sequence of full length IC106 is SEQ ID NO: 140 herein. In the R6 genome IC106 is spr1994 [84]. The use of IC106 for immunisation is reported in reference 10 (SEQ ID NO: 262 therein).

Immunogenic fragments of IC106 are identified in table 1 of reference 10.

### IC107

IC107 is annotated in reference 10 as a hypothetical protein. For reference purposes, the amino acid sequence of full length IC107 is SEQ ID NO: 141 herein. The use of IC107 for immunisation is reported in reference 10 (SEQ ID NO: 263 therein). Immunogenic fragments of IC107 are identified in table 1 of reference 10.

### IC108

IC108 is annotated in reference 10 as a hypothetical protein. For reference purposes, the amino acid sequence of full length IC108 is SEQ ID NO: 142 herein. The use of IC108 for immunisation is reported in reference 10 (SEQ ID NO: 264 therein).

Immunogenic fragments of IC108 are identified in table 1 of reference 10.

### IC109

IC109 is annotated in reference 10 as a hypothetical protein. For reference purposes, the amino acid sequence of full length IC109 is SEQ ID NO: 143 herein. In the R6 genome IC109 is spr0309 [84]. The use of IC109 for immunisation is reported in reference 10 (SEQ ID NO: 265 therein).

Immunogenic fragments of IC109 are identified in table 1 of reference 10.

### IC110

IC110 is annotated in reference 10 as a hypothetical protein. For reference purposes, the amino acid sequence of full length IC110 is SEQ ID NO: 144 herein. In the R6 genome IC110 is spr1070 [84]. The use of IC110 for immunisation is reported in reference 10 (SEQ ID NO: 266 therein).

Immunogenic fragments of IC110 are identified in table 1 of reference 10.

### IC111

IC111 is annotated in reference 10 as a hypothetical protein. For reference purposes, the amino acid sequence of full length IC111 is SEQ ID NO: 145 herein. In the R6 genome IC111 is spr0258 [84]. The use of IC111 for immunisation is reported in reference 10 (SEQ ID NO: 267 therein).

Immunogenic fragments of IC111 are identified in table 1 of reference 10. *IC112*
IC112 is annotated in reference 10 as a hypothetical protein. For reference purposes, the amino acid sequence of full length IC112 is SEQ ID NO: 146 herein. In the R6 genome IC112 is spr0254 [84]. The use of IC 112 for immunisation is reported in reference 10 (SEQ ID NO: 268 therein).

Immunogenic fragments of IC 112 are identified in table 1 of reference 10.

### IC113

IC113 is annotated in reference 10 as a hypothetical protein. For reference purposes, the amino acid sequence of full length IC113 is SEQ ID NO: 147 herein. In the R6 genome IC113 is spr0171 [84]. The use of IC113 for immunisation is reported in reference 10 (SEQ ID NO: 269 therein).

Immunogenic fragments of IC113 are identified in table 1 of reference 10.

### IC114

IC114 is annotated in reference 10 as a hypothetical protein. For reference purposes, the amino acid sequence of full length IC114 is SEQ ID NO: 148 herein. The use of IC114 for immunisation is reported in reference 10 (SEQ ID NO: 270 therein).

Immunogenic fragments of IC114 are identified in table 1 of reference 10.

### IC115

IC115 is annotated in reference 10 as a hypothetical protein. For reference purposes, the amino acid sequence of full length IC115 is SEQ ID NO: 149 herein. In the R6 genome IC115 is spr0464 [84]. The use of IC 115 for immunisation is reported in reference 10 (SEQ ID NO: 271 therein).

Immunogenic fragments of IC 115 are identified in table 1 of reference 10.

### IC116

IC116 is annotated in reference 10 as a hypothetical protein. For reference purposes, the amino acid sequence of full length IC116 is SEQ ID NO: 150 herein. In the R6 genome IC116 is spr0026 [84]. The use of IC116 for immunisation is reported in reference 10 (SEQ ID NO: 272 therein).

Immunogenic fragments of IC116 are identified in table 1 of reference 10.

### IC117

IC117 is annotated in reference 10 as a hypothetical protein. For reference purposes, the amino acid sequence of full length IC117 is SEQ ID NO: 151 herein. In the R6 genome IC117 is spr1652 [84]. The use of IC 117 for immunisation is reported in reference 10 (SEQ ID NO: 273 therein).

Immunogenic fragments of IC 117 are identified in table 1 of reference 10.

### IC118

IC118 is annotated in reference 10 as a hypothetical protein. For reference purposes, the amino acid sequence of full length IC118 is SEQ ID NO: 152 herein. In the R6 genome IC118 is spr1783 [84]. The use of IC118 for immunisation is reported in reference 10 (SEQ ID NO: 274 therein).

Immunogenic fragments of IC118 are identified in table 1 of reference 10.

### IC119

IC119 is annotated in reference 10 as a hypothetical protein. For reference purposes, the amino acid sequence of full length IC 119 is SEQ ID NO: 153 herein. The use of IC 119 for immunisation is reported in reference 10 (SEQ ID NO: 275 therein). Immunogenic fragments of IC 119 are identified in table 1 of reference 10.

### IC120

IC120 is annotated in reference 10 as a hypothetical protein. For reference purposes, the amino acid sequence of full length IC120 is SEQ ID NO: 154 herein. In the R6 genome IC120 is spr1153 [84]. The use of IC120 for immunisation is reported in reference 10 (SEQ ID NO: 276 therein).

Immunogenic fragments of IC120 are identified in table 1 of reference 10.

### IC121

IC121 is annotated in reference 10 as a hypothetical protein. For reference purposes, the amino acid sequence of full length IC121 is SEQ ID NO: 155 herein. In the R6 genome IC121 is spr1977 [84]. The use of IC121 for immunisation is reported in reference 10 (SEQ ID NO: 277 therein).

Immunogenic fragments of IC121 are identified in table 1 of reference 10.

### IC122

IC122 is annotated in reference 10 as a hypothetical protein. For reference purposes, the amino acid sequence of full length IC122 is SEQ ID NO: 156 herein. The use of IC122 for immunisation is reported in reference 10 (SEQ ID NO: 278 therein).

Immunogenic fragments of IC 122 are identified in table 1 of reference 10.

### IC123

IC123 is annotated in reference 10 as a hypothetical protein. For reference purposes, the amino acid sequence of full length IC123 is SEQ ID NO: 157 herein. In the R6 genome IC123 is spr1049 [84]. The use of IC123 for immunisation is reported in reference 10 (SEQ ID NO: 279 therein).

Immunogenic fragments of IC123 are identified in table 1 of reference 10. *IC124*
IC124 is annotated in reference 10 as a hypothetical protein. For reference purposes, the amino acid sequence of full length IC124 is SEQ ID NO: 158 herein. In the R6 genome IC124 is spr1811 [84]. The use of IC124 for immunisation is reported in reference 10 (SEQ ID NO: 280 therein).

Immunogenic fragments of IC124 are identified in table 1 of reference 10.

### IC125

IC125 is annotated in reference 10 as a hypothetical protein. For reference purposes, the amino acid sequence of full length IC125 is SEQ ID NO: 159 herein. In the R6 genome IC125 is spr0381 [84]. The use of IC125 for immunisation is reported in reference 10 (SEQ ID NO: 281 therein).

Immunogenic fragments of IC125 are identified in table 1 of reference 10.

### IC126

IC126 is annotated in reference 10 as a hypothetical protein. For reference purposes, the amino acid sequence of full length IC126 is SEQ ID NO: 160 herein. The use of IC126 for immunisation is reported in reference 10 (SEQ ID NO: 282 therein).

Immunogenic fragments of IC126 are identified in table 1 of reference 10.

### IC127

IC127 is annotated in reference 10 as a hypothetical protein. For reference purposes, the amino acid sequence of full length IC127 is SEQ ID NO: 161 herein. In the R6 genome IC127 is spr0061 [84]. The use of IC127 for immunisation is reported in reference 10 (SEQ ID NO: 283 therein).

Immunogenic fragments of IC127 are identified in table 1 of reference 10.

### IC128

IC128 is annotated in reference 10 as a hypothetical protein. For reference purposes, the amino acid sequence of full length IC128 is SEQ ID NO: 162 herein. In the R6 genome IC128 is spr0641 [84]. The use of IC128 for immunisation is reported in reference 10 (SEQ ID NO: 284 therein).

Immunogenic fragments of IC128 are identified in table 1 of reference 10.

### IC129

IC129 is annotated in reference 10 as a hypothetical protein. For reference purposes, the amino acid sequence of full length IC129 is SEQ ID NO: 163 herein. In the R6 genome IC129 is spr1205 [84]. The use of IC129 for immunisation is reported in reference 10 (SEQ ID NO: 285 therein).

Immunogenic fragments of IC129 are identified in table 1 of reference 10.

### IC130

IC130 is annotated in reference 10 as a hypothetical protein. For reference purposes, the amino acid sequence of full length IC130 is SEQ ID NO: 164 herein. In the R6 genome IC130 is spr1841 [84]. The use of IC130 for immunisation is reported in reference 10 (SEQ ID NO: 286 therein).

Immunogenic fragments of IC130 are identified in table 1 of reference 10.

### IC131

IC131 is annotated in reference 10 as a hypothetical protein. For reference purposes, the amino acid sequence of full length IC 131 is SEQ ID NO: 165 herein. In the R6 genome IC 131 is spr1777 [84]. The use of IC 131 for immunisation is reported in reference 10 (SEQ ID NO: 287 therein). Immunogenic fragments of IC 131 are identified in table 1 of reference 10.

### spr0222

The original 'spr0222' sequence was annotated in reference 228 as 'ABC transporter ATP-binding protein - iron transport' (see GI:15457768). For reference purposes, the amino acid sequence of full length spr0222 as found in the R6 strain is given as SEQ ID NO: 121 herein. Its use in immunisation is suggested in reference 5.

### CbiO

CbiO is annotated as a cobalt transporter ATP-binding subunit. For reference purposes, the amino acid sequence of full length CbiO is SEQ ID NO: 167 herein. In the R6 genome CbiO is spr2025 [84]. The use of CbiO for immunisation is reported in reference 6 ('ID2' therein).

### 30S ribosomal protein S8

For reference purposes, the amino acid sequence of 30S ribosomal protein S8 is SEQ ID NO: 168 herein. In the R6 genome the S8 subunit is spr0203 [84].

### RrgA

RrgA is one of the surface subunits of the pneumococcal pilus [79,80] and is an important adhesin [81].There are at least two allelic forms of RrgA and, for reference purposes, their amino acid sequences are SEQ ID NOs: 172 and 179 herein. The two alleles are well conserved at their N- and C-termini but deviate in between.

Preferred RrgA polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 172; and/or (b) comprising a fragment of at least 'n' consecutive amino acids of SEQ ID NO: 172, wherein 'n' is 10 or more (*e.g.* 10, 12, 14, 16, 18, 20, 25, 30, 35, 40, 50, 60, 70, 80, 90, 100, 150, 200, 250 or more). These RrgA proteins include variants of SEQ ID NO: 172. Preferred fragments of (b) comprise an epitope from SEQ ID NO: 172. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 172 while retaining at least one epitope of SEQ ID NO: 172. Other fragments omit one or more protein domains. One suitable fragment is SEQ ID NO: 192, which omits the natural leader peptide and sortase recognition sequences.

Other preferred RrgA polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 179; and/or (b) comprising a fragment of at least 'n' consecutive amino acids of SEQ ID NO: 179, wherein 'n' is 10 or more (*e.g.* 10, 12, 14, 16, 18, 20, 25, 30, 35, 40, 50, 60, 70, 80, 90, 100, 150, 200, 250 or more). These RrgA proteins include variants of SEQ ID NO: 179. Preferred fragments of (b) comprise an epitope from SEQ ID NO: 179. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 179 while retaining at least one epitope of SEQ ID NO: 179. Other fragments omit one or more protein domains. One suitable fragment is SEQ ID NO: 191, which omits the natural leader peptide and sortase recognition sequences.

### RrgB

RrgB is one of the surface subunits of the pneumococcal pilus [79,80]. There are at least three allelic forms of RrgB and, for reference purposes, their amino acid sequences are SEQ ID NOs: 173, 174 and 175 herein. The three alleles are well conserved at their N- and C-termini but deviate in between.

Preferred RrgB polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 173; and/or (b) comprising a fragment of at least 'n' consecutive amino acids of SEQ ID NO: 173, wherein 'n' is 10 or more (*e.g*. 10, 12, 14, 16, 18, 20, 25, 30, 35, 40, 50, 60, 70, 80, 90, 100, 150, 200, 250 or more). These RrgB proteins include variants of SEQ ID NO: 173. Preferred fragments of (b) comprise an epitope from SEQ ID NO: 173. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 173 while retaining at least one epitope of SEQ ID NO: 173. Other fragments omit one or more protein domains.

Other preferred RrgB polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 174; and/or (b) comprising a fragment of at least 'n' consecutive amino acids of SEQ ID NO: 174, wherein 'n' is 10 or more (*e.g.* 10, 12, 14, 16, 18, 20, 25, 30, 35, 40, 50, 60, 70, 80, 90, 100, 150, 200, 250 or more). These RrgB proteins include variants of SEQ ID NO: 174. Preferred fragments of (b) comprise an epitope from SEQ ID NO: 174. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or more) from the N-terminus of SEQ ID NO: 174 while retaining at least one epitope of SEQ ID NO: 174. Other fragments omit one or more protein domains.

Other preferred RrgB polypeptides for use with the invention comprise an amino acid sequence: (a) having 80% or more identity (*e.g*. 80%, 85%, 90%, 91%, 92%, 93%, 94%, 95%, 96%, 97%, 98%, 99%, 99.5% or more) to SEQ ID NO: 175; and/or (b) comprising a fragment of at least 'n' consecutive amino acids of SEQ ID NO: 175, wherein 'n' is 10 or more (*e.g.* 10, 12, 14, 16, 18, 20, 25, 30, 35, 40, 50, 60, 70, 80, 90, 100, 150, 200, 250 or more). These RrgB proteins include variants of SEQ ID NO: 175. Preferred fragments of (b) comprise an epitope from SEQ ID NO: 175. Other preferred fragments lack one or more amino acids (*e.g.* 1, 2, 3, 4, 5, 6, 7, 8, 9, 10, 15, 20, 25 or more) from the C-terminus and/or one or more amino acids (*e.g.* 1, 2, 3, 4, 5, 6, 7, 8, 9, 10, 15, 20, 25 or 3more) from the N-terminus of SEQ ID NO: 175 while retaining at least one epitope of SEQ ID NO: 175. Other fragments omit one or more protein domains.

### RrgC

RrgC is one of the surface subunits of the pneumococcal pilus [79,80]. For reference purposes, the amino acid sequence of RrgC is SEQ ID NO: 176 herein.

### Hybrid polypeptides

Pneumococcal antigens used in the invention may be present in the composition as individual separate polypeptides. Where more than one antigen is used, however, they do not have to be present as separate polypeptides. Instead, at least two (*e.g.* 2, 3, 4, 5, or more) antigens can be expressed as a single polypeptide chain (a 'hybrid' polypeptide). Hybrid polypeptides offer two main advantages: first, a polypeptide that may be unstable or poorly expressed on its own can be assisted by adding a suitable hybrid partner that overcomes the problem; second, commercial manufacture is simplified as only one expression and purification need be employed in order to produce two polypeptides which are both antigenically useful.

The hybrid polypeptide may comprise two or more polypeptide sequences from the first antigen group. The hybrid polypeptide may comprise one or more polypeptide sequences from the first antigen group and one or more polypeptide sequences from the second antigen group. The hybrid polypeptide may comprise one or more polypeptide sequences from the first antigen group and one or more polypeptide sequences from the third antigen group. The hybrid polypeptide may comprise one or more polypeptide sequences from the second antigen group and one or more polypeptide sequences from the third antigen group. The hybrid polypeptide may comprise two or more polypeptide sequences from the seventh antigen group. The hybrid polypeptide may comprise two or more polypeptide sequences from the eighth antigen group. The hybrid polypeptide may comprise two or more polypeptide sequences from the ninth antigen group. The hybrid polypeptide may comprise two or more polypeptide sequences from the tenth antigen group. Moreover, the hybrid polypeptide may comprise two or more polypeptide sequences from each of the antigens listed above, or two or more variants of the same antigen in the cases in which the sequence has partial variability across strains.

Hybrids consisting of amino acid sequences from two, three, four, five, six, seven, eight, nine, or ten pneumococcal antigens are useful. In particular, hybrids consisting of amino acid sequences from two, three, four, or five pneumococcal antigens are preferred, such as two or three pneumococcal antigens.

Different hybrid polypeptides may be mixed together in a single formulation. Hybrids may be combined with non-hybrid antigens selected from the first, second or third antigen groups. Within such combinations, a pneumococcal antigen may be present in more than one hybrid polypeptide and/or as a non-hybrid polypeptide. It is preferred, however, that an antigen is present either as a hybrid or as a non-hybrid, but not as both.

The hybrid polypeptides can also be combined with conjugates or non-pneumococcal antigens as described above.

Hybrid polypeptides can be represented by the formula NH₂-A-{-X-L-}*ₙ*,-B-COOH, wherein: X is an amino acid sequence of a pneumococcal antigen, as described above; L is an optional linker amino acid sequence; A is an optional N-terminal amino acid sequence; B is an optional C-terminal amino acid sequence; *n* is an integer of 2 or more (*e.g.* 2, 3, 4, 5, 6, *etc*.). Usually *n* is 2 or 3.

If a -X- moiety has a leader peptide sequence in its wild-type form, this may be included or omitted in the hybrid protein. In some embodiments, the leader peptides will be deleted except for that of the -X- moiety located at the N-terminus of the hybrid protein *i.e.* the leader peptide of X₁ will be retained, but the leader peptides of X₂ ... Xₙ will be omitted. This is equivalent to deleting all leader peptides and using the leader peptide of X₁ as moiety -A-.

For each *n* instances of {-X-L-}, linker amino acid sequence -L- may be present or absent. For instance, when *n*=2 the hybrid may be NH₂-X₁-L₁-X₂-L₂-COOH, NH₂-X₁-X₂-COOH, NH₂-X₁-L₁-X₂-COOH, NH₂-X₁-X₂-L₂-COOH, *etc.* Linker amino acid sequence(s) -L- will typically be short (*e.g.* 20 or fewer amino acids *i.e.* 20, 19, 18, 17, 16, 15, 14, 13, 12, 11, 10, 9, 8, 7, 6, 5, 4, 3, 2, 1). Examples comprise short peptide sequences which facilitate cloning, poly-glycine linkers (*i.e*. comprising Gly*ₙ* where *n* = 2, 3, 4, 5, 6, 7, 8, 9, 10 or more), and histidine tags (*i.e.* His*ₙ* where *n* = 3, 4, 5, 6, 7, 8, 9, 10 or more). Other suitable linker amino acid sequences will be apparent to those skilled in the art. A useful linker is GSGGGG (SEQ ID NO:232) or GSGSGGGG (SEQ ID NO:233), with the Gly-Ser dipeptide being formed from a *Bam*HI restriction site, thus aiding cloning and manipulation, and the (Gly)₄ tetrapeptide being a typical poly-glycine linker. Other suitable linkers, particularly for use as the final Lₙ are a Leu-Glu dipeptide or SEQ ID NO: 235.

-A- is an optional N-terminal amino acid sequence. This will typically be short (*e.g.* 40 or fewer amino acids *i.e.* 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct protein trafficking, or short peptide sequences which facilitate cloning or purification (*e.g*. histidine tags *i.e.* His*ₙ* where *n* = 3, 4, 5, 6, 7, 8, 9, 10 or more). Other suitable N-terminal amino acid sequences will be apparent to those skilled in the art. If X₁ lacks its own N-terminus methionine, -A-is preferably an oligopeptide (*e.g*. with 1, 2, 3, 4, 5, 6, 7 or 8 amino acids) which provides a N-terminus methionine *e.g*. Met-Ala-Ser, or a single Met residue.

-B- is an optional C-terminal amino acid sequence. This will typically be short (*e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include sequences to direct protein trafficking, short peptide sequences which facilitate cloning or purification (*e.g*. comprising histidine tags *i.e.* His*ₙ* where *n* = 3, 4, 5, 6, 7, 8, 9, 10 or more, such as SEQ ID NO: 234), or sequences which enhance protein stability. Other suitable C-terminal amino acid sequences will be apparent to those skilled in the art.

Examples of hybrids include polypeptides that comprise an amino acid sequence selected from the group consisting of: spr2021-spr0057 (e.g. SEQ ID NO: 193); spr2021-spr0096 (e.g. SEQ ID NO: 194); spr2021-spr0565 (e.g. SEQ ID NO: 195 or SEQ ID NO: 196 or SEQ ID NO: 197); spr2021-RrgA (e.g. SEQ ID NO: 198); spr0057-spr2021 (e.g. SEQ ID NO: 199); spr0057-spr0096 (e.g. SEQ ID NO: 200); spr0057-RrgA (e.g. SEQ ID NO: 201); spr0057-spr0565 (e.g. SEQ ID NO: 202 or SEQ ID NO: 203 or SEQ ID NO: 204); spr0096-spr2021 (e.g. SEQ ID NO: 205); spr0096-spr0057 (e.g. SEQ ID NO: 206); spr0096-RrgA (e.g. SEQ ID NO: 207); spr0096-spr0565 (e.g. SEQ ID NO: 208 or SEQ ID NO: 209 or SEQ ID NO: 210); RrgA-spr2021 (e.g. SEQ ID NO: 211); RrgA-spr0565 (e.g. SEQ ID NO: 212 or SEQ ID NO: 213 or SEQ ID NO: 214); RrgA-spr0057 (e.g. SEQ ID NO: 215); RrgA-spr0096 (e.g. SEQ ID NO: 216); spr0565-spr0057 (e.g. SEQ ID NO: 217 or SEQ ID NO: 218 or SEQ ID NO: 219); spr0565-spr0096 (e.g. SEQ ID NO: 220 or SEQ ID NO: 221 or SEQ ID NO: 222); spr0565-spr2021 (e.g. SEQ ID NO: 223 or SEQ ID NO: 224 or SEQ ID NO: 225); or spr0565-RrgA (e.g. SEQ ID NO: 226 or SEQ ID NO: 227 or SEQ ID NO: 228).

### Polypeptide used with the invention

Polypeptides used with the invention can take various forms (*e.g*. native, fusions, glycosylated, non-glycosylated, lipidated, non-lipidated, phosphorylated, non-phosphorylated, myristoylated, non-myristoylated, monomeric, multimeric, particulate, denatured, *etc*.).

Polypeptides used with the invention can be prepared by various means (*e.g*. recombinant expression, purification from cell culture, chemical synthesis, *etc*.). Recombinantly-expressed proteins are preferred, particularly for hybrid polypeptides.

Polypeptides used with the invention are preferably provided in purified or substantially purified form *i.e.* substantially free from other polypeptides (*e.g.* free from naturally-occurring polypeptides), particularly from other streptococcal or host cell polypeptides, and are generally at least about 50% pure (by weight), and usually at least about 90% pure *i.e.* less than about 50%, and more preferably less than about 10% (*e.g.* 5%) of a composition is made up of other expressed polypeptides. Thus the antigens in the compositions are separated from the whole organism with which the molecule is expressed.

Polypeptides used with the invention are preferably pneumococcal polypeptides.

The term "polypeptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included are, for example, polypeptides containing one or more analogs of an amino acid (including, for example, unnatural amino acids, *etc*.), as well as other modifications known in the art. Polypeptides can occur as single chains or associated chains.

The invention provides polypeptides comprising a sequence -P-Q- or -Q-P-, wherein: -P- is an amino acid sequence as defined above and -Q- is not a sequence as defined above *i. e.* the invention provides fusion proteins. Where the N-terminus codon of -P- is not ATG, but this codon is not present at the N-terminus of a polypeptide, it will be translated as the standard amino acid for that codon rather than as a Met. Where this codon is at the N-terminus of a polypeptide, however, it will be translated as Met. Examples of -Q- moieties include, but are not limited to, histidine tags (*i.e.* His*ₙ* where *n* = 3, 4, 5, 6, 7, 8, 9, 10 or more), maltose-binding protein, or glutathione-S-transferase (GST).

The disclosure also provides a process for producing a polypeptide of the invention, comprising the step of culturing a host cell transformed with nucleic acid of the invention under conditions which induce polypeptide expression.

Although expression of the polypeptides of the invention may take place in a *Streptococcus,* the invention will usually use a heterologous host for expression (recombinant expression). The heterologous host may be prokaryotic (*e.g.* a bacterium) or eukaryotic. It may be *E.coli,* but other suitable hosts include *Bacillus subtilis, Vibrio cholera, Salmonella typhi, Salmonella typhimurium, Neisseria lactamica, Neisseria cinerea, Mycobacteria* (*e.g. M.tuberculosis*), yeasts, *etc.* Compared to the wild-type pneumococcal genes encoding polypeptides of the invention, it is helpful to change codons to optimise expression efficiency in such hosts without affecting the encoded amino acids.

The disclosure provides a process for producing a polypeptide of the invention, comprising the step of synthesising at least part of the polypeptide by chemical means. ***Nucleic acids,***
The invention also provides nucleic acid encoding hybrid polypeptides of the invention. It also provides nucleic acid comprising a nucleotide sequence that encodes one or more hybrid polypeptides of the invention.

The invention also provides nucleic acid comprising nucleotide sequences having sequence identity to such nucleotide sequences. Identity between sequences is preferably determined by the Smith-Waterman homology search algorithm as described above. Such nucleic acids include those using alternative codons to encode the same amino acid.

The invention also provides nucleic acid which can hybridize to these nucleic acids. Hybridization reactions can be performed under conditions of different "stringency". Conditions that increase stringency of a hybridization reaction of widely known and published in the art (*e.g.* page 7.52 of reference 288). Examples of relevant conditions include (in order of increasing stringency): incubation temperatures of 25°C, 37°C, 50°C, 55°C and 68°C; buffer concentrations of 10 x SSC, 6 x SSC, 1 x SSC, 0.1 x SSC (where SSC is 0.15 M NaCl and 15 mM citrate buffer) and their equivalents using other buffer systems; formamide concentrations of 0%, 25%, 50%, and 75%; incubation times from 5 minutes to 24 hours; 1, 2, or more washing steps; wash incubation times of 1, 2, or 15 minutes; and wash solutions of 6 x SSC, 1 x SSC, 0.1 x SSC, or de-ionized water. Hybridization techniques and their optimization are well known in the art (*e.g.* see refs 82, 83, 288, 290, *etc*.].

In some embodiments, nucleic acid of the invention hybridizes to a target under low stringency conditions; in other embodiments it hybridizes under intermediate stringency conditions; in preferred embodiments, it hybridizes under high stringency conditions. An exemplary set of low stringency hybridization conditions is 50°C and 10 x SSC. An exemplary set of intermediate stringency hybridization conditions is 55°C and 1 x SSC. An exemplary set of high stringency hybridization conditions is 68°C and 0.1 x SSC.

The invention includes nucleic acid comprising sequences complementary to these sequences (*e.g*. for antisense or probing, or for use as primers).

Nucleic acids of the invention can be used in hybridisation reactions (*e.g*. Northern or Southern blots, or in nucleic acid microarrays or 'gene chips') and amplification reactions (*e.g*. PCR, SDA, SSSR, LCR, TMA, NASBA, *etc.*) and other nucleic acid techniques.

Nucleic acid according to the invention can take various forms (*e.g*. single-stranded, double-stranded, vectors, primers, probes, labelled *etc*.). Nucleic acids of the invention may be circular or branched, but will generally be linear. Unless otherwise specified or required, any embodiment of the invention that utilizes a nucleic acid may utilize both the double-stranded form and each of two complementary single-stranded forms which make up the double-stranded form. Primers and probes are generally single-stranded, as are antisense nucleic acids. Nucleic acids of the invention are preferably provided in purified or substantially purified form *i.e.* substantially free from other nucleic acids (*e.g.* free from naturally-occurring nucleic acids), particularly from other pneumococcal or host cell nucleic acids, generally being at least about 50% pure (by weight), and usually at least about 90% pure. Nucleic acids of the invention are preferably pneumococcal nucleic acids.

Nucleic acids of the invention may be prepared in many ways *e.g.* by chemical synthesis (*e.g.* phosphoramidite synthesis of DNA) in whole or in part, by digesting longer nucleic acids using nucleases (*e.g.* restriction enzymes), by joining shorter nucleic acids or nucleotides (*e.g.* using ligases or polymerases), from genomic or cDNA libraries, *etc.*

Nucleic acid of the invention may be attached to a solid support (*e.g*. a bead, plate, filter, film, slide, microarray support, resin, *etc*.). Nucleic acid of the invention may be labelled *e.g.* with a radioactive or fluorescent label, or a biotin label. This is particularly useful where the nucleic acid is to be used in detection techniques *e.g*. where the nucleic acid is a primer or as a probe.

The term "nucleic acid" includes in general means a polymeric form of nucleotides of any length, which contain deoxyribonucleotides, ribonucleotides, and/or their analogs. It includes DNA, RNA, DNA/RNA hybrids. It also includes DNA or RNA analogs, such as those containing modified backbones (*e.g*. peptide nucleic acids (PNAs) or phosphorothioates) or modified bases. Thus the invention includes mRNA, tRNA, rRNA, ribozymes, DNA, cDNA, recombinant nucleic acids, branched nucleic acids, plasmids, vectors, probes, primers, *etc..* Where nucleic acid of the invention takes the form of RNA, it may or may not have a 5' cap.

Nucleic acids of the invention may be part of a vector *i.e*. part of a nucleic acid construct designed for transduction/transfection of one or more cell types. Vectors may be, for example, "cloning vectors" which are designed for isolation, propagation and replication of inserted nucleotides, "expression vectors" which are designed for expression of a nucleotide sequence in a host cell, "viral vectors" which is designed to result in the production of a recombinant virus or virus-like particle, or "shuttle vectors", which comprise the attributes of more than one type of vector. Preferred vectors are plasmids. A "host cell" includes an individual cell or cell culture which can be or has been a recipient of exogenous nucleic acid. Host cells include progeny of a single host cell, and the progeny may not necessarily be completely identical (in morphology or in total DNA complement) to the original parent cell due to natural, accidental, or deliberate mutation and/or change. Host cells include cells transfected or infected *in vivo* or *in vitro* with nucleic acid of the invention.

Where a nucleic acid is DNA, it will be appreciated that "U" in a RNA sequence will be replaced by "T" in the DNA. Similarly, where a nucleic acid is RNA, it will be appreciated that "T" in a DNA sequence will be replaced by "U" in the RNA.

The term "complement" or "complementary" when used in relation to nucleic acids refers to Watson-Crick base pairing. Thus the complement of C is G, the complement of G is C, the complement of A is T (or U), and the complement of T (or U) is A. It is also possible to use bases such as I (the purine inosine) *e.g*. to complement pyrimidines (C or T).

Nucleic acids of the invention can be used, for example: to produce polypeptides; as hybridization probes for the detection of nucleic acid in biological samples; to generate additional copies of the nucleic acids; to generate ribozymes or antisense oligonucleotides; as single-stranded DNA primers or probes; or as triple-strand forming oligonucleotides.

The disclosure provides a process for producing nucleic acid of the invention, wherein the nucleic acid is synthesised in part or in whole using chemical means.

The disclosure provides vectors comprising nucleotide sequences of the invention (*e.g*. cloning or expression vectors) and host cells transformed with such vectors.

Nucleic acid amplification may be quantitative and/or real-time.

For certain embodiments of the invention, nucleic acids are preferably at least 7 nucleotides in length (*e.g.* 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 90, 100, 110, 120, 130, 140, 150, 160, 170, 180, 190, 200, 225, 250, 275, 300 nucleotides or longer).

For certain embodiments of the invention, nucleic acids are preferably at most 500 nucleotides in length *(e.g.* 450, 400, 350, 300, 250, 200, 150, 140, 130, 120, 110, 100, 90, 80, 75, 70, 65, 60, 55, 50, 45, 40, 39, 38, 37, 36, 35, 34, 33, 32, 31, 30, 29, 28, 27, 26, 25, 24, 23, 22, 21, 20, 19, 18, 17, 16, 15 nucleotides or shorter).

Primers and probes and other nucleic acids used for hybridization, are preferably between 10 and 30 nucleotides in length (*e.g*. 10, 11, 12, 13, 14, 15, 16, 17, 18, 19, 20, 21, 22, 23, 24, 25, 26, 27, 28, 29, or 30 nucleotides).

### Strains and variants

Antigens are defined above by reference to "spr" nomenclature. This nomenclature refers to the numbering used in reference 84 for unique identification of open reading frames in the R6 strain of *S.pneumoniae.* The basic reference sequence for any "spr" number can easily be found in public gene databases. For instance, GenBank accession number NC_003098 (GI:15902044) is the complete R6 genome sequence (2,038,615 bp), and the individual spr sequences are given as "locus_tag" entries in the genome sequence's "features" section. Thus the amino acid sequence for any given spr number, and its natural coding sequence, can be established unambiguously for strain R6. Functional annotations are also given in the databases.

The invention is not limited to sequences from the R6 strain. Genome sequences of several other strains of *S.pneumoniae* are available, including those of 23F [85], 670 [86] and TIGR4 [87,88,89]. Standard search and alignment techniques can be used to identify in any of these (or other) further genome sequences the homolog of any particular spr sequence from R6. Moreover, the available R6 (and other) sequences can be used to design primers for amplification of homologous sequences from other strains. Thus the invention is not limited to R6 sequences, but rather encompasses such variants and homologs from other strains of *S.pneumoniae,* as well as non-natural variants. In general, suitable variants of a particular SEQ ID NO include its allelic variants, its polymorphic forms, its homologs, its orthologs, its paralogs, its mutants, *etc.*

Thus, for instance, polypeptides used with the invention may, compared to the R6 reference sequence, include one or more (*e.g.* 1, 2, 3, 4, 5, 6, 7, 8, 9, *etc*.) amino acid substitutions, such as conservative substitutions (*i.e*. substitutions of one amino acid with another which has a related side chain). Genetically-encoded amino acids are generally divided into four families: (1) acidic *i.e.* aspartate, glutamate; (2) basic *i. e.* lysine, arginine, histidine; (3) non-polar *i. e.* alanine, valine, leucine, isoleucine, proline, phenylalanine, methionine, tryptophan; and (4) uncharged polar *i.e.* glycine, asparagine, glutamine, cysteine, serine, threonine, tyrosine. Phenylalanine, tryptophan, and tyrosine are sometimes classified jointly as aromatic amino acids. In general, substitution of single amino acids within these families does not have a major effect on the biological activity. The polypeptides may also include one or more (*e.g.* 1, 2, 3, 4, 5, 6, 7, 8, 9, *etc*.*)* single amino acid deletions relative to the R6 sequences. The polypeptides may also include one or more (*e.g.* 1, 2, 3, 4, 5, 6, 7, 8, 9, *etc.*) insertions (*e.g.* each of 1, 2, 3, 4 or 5 amino acids) relative to the R6 sequences.

Similarly, a polypeptide used with the invention may comprise an amino acid sequence that:
(a) is identical (*i.e*. 100% identical) to a sequence disclosed in the sequence listing;
(b) shares sequence identity (*e.g*. 60%, 65%, 70%, 75%, 80%, 85%, 90%, 91%, 92%, 93%, 94%, 95%, 96%, 97%, 98%, 99%, 99.5% or more) with a sequence disclosed in the sequence listing;
(c) has 1, 2, 3, 4, 5, 6, 7, 8, 9 or 10 (or more) single amino acid alterations (deletions, insertions, substitutions), which may be at separate locations or may be contiguous, as compared to the sequences of (a) or (b); and
(d) when aligned with a particular sequence from the sequence listing using a pairwise alignment algorithm, each moving window of x amino acids from N-terminus to C-terminus (such that for an alignment that extends to *p* amino acids, where *p>x,* there are *p*-*x*+*1* such windows) has at least *x·y* identical aligned amino acids, where: x is selected from 20, 25, 30, 35, 40, 45, 50, 60, 70, 80, 90, 100, 150, 200; *y* is selected from 0.50, 0.60, 0.70, 0.75, 0.80, 0.85, 0.90, 0.91, 0.92, 0.93, 0.94, 0.95, 0.96, 0.97, 0.98, 0.99; and if *x·y* is not an integer then it is rounded up to the nearest integer. The preferred pairwise alignment algorithm is the Needleman-Wunsch global alignment algorithm [90], using default parameters (*e.g*. with Gap opening penalty = 10.0, and with Gap extension penalty = 0.5, using the EBLOSUM62 scoring matrix). This algorithm is conveniently implemented in the *needle* tool in the EMBOSS package [91].
Where hybrid polypeptides are used, the individual antigens within the hybrid (*i.e*. individual -X-moieties) may be from one or more strains. Where *n*=2, for instance, X₂ may be from the same strain as X₁ or from a different strain. Where *n*=3, the strains might be (i) X₁=X₂=X₃ (ii) X₁=X₂≠X₃ (iii) X₁≠X₂=X₃ (iv) X₁≠X₂≠X₃ or (v) X₁=X₃≠X₂, *etc.*

Within group (c), deletions or substitutions may be at the N-terminus and/or C-terminus, or may be between the two termini. Thus a truncation is an example of a deletion. Truncations may involve deletion of up to 40 (or more) amino acids at the N-terminus and/or C-terminus.

In general, when a polypeptide of the invention comprises a sequence that is not identical to a complete pneumococcal sequence from the sequence listing (*e.g*. when it comprises a sequence listing with <100% sequence identity thereto, or when it comprises a fragment thereof) it is preferred in each individual instance that the polypeptide can elicit an antibody that recognises the complete pneumococcal sequence.

### Mutant bacteria

The disclosure also provides a pneumococcus in which one or more of the antigens from the various antigen groups of the invention has/have been knocked out. Techniques for producing knockout bacteria are well known, and knockout pneumococci have been reported. A knockout mutation may be situated in the coding region of the gene or may lie within its transcriptional control regions (*e.g*. within its promoter). A knockout mutation will reduce the level of mRNA encoding the antigen to <1% of that produced by the wild-type bacterium, preferably <0.5%, more preferably <0.1%, and most preferably to 0%.

The disclosure also provides a pneumococcus in which one or more of the antigens from the various antigen groups of the invention has a mutation which inhibits its activity. The gene encoding the antigen will have a mutation that changes the encoded amino acid sequence. Mutation may involve deletion, substitution, and/or insertion, any of which may be involve one or more amino acids.

### Immunogenic compositions and medicaments

Immunogenic compositions of the invention may be useful as vaccines. Vaccines according to the invention may either be prophylactic (*i.e.* to prevent infection) or therapeutic (*i.e.* to treat infection), but will typically be prophylactic.

Compositions may thus be pharmaceutically acceptable. They will usually include components in addition to the antigens *e.g*. they typically include one or more pharmaceutical carrier(s) and/or excipient(s). A thorough discussion of such components is available in reference 285.

Compositions will generally be administered to a mammal in aqueous form. Prior to administration, however, the composition may have been in a non-aqueous form. For instance, although some vaccines are manufactured in aqueous form, then filled and distributed and administered also in aqueous form, other vaccines are lyophilised during manufacture and are reconstituted into an aqueous form at the time of use. Thus a composition of the invention may be dried, such as a lyophilised formulation. The composition may include preservatives such as thiomersal or 2-phenoxyethanol. It is preferred, however, that the vaccine should be substantially free from (*i. e.* less than 5µg/ml) mercurial material *e.g*. thiomersal-free. Vaccines containing no mercury are more preferred. Preservative-free vaccines are particularly preferred.

To improve thermal stability, a composition may include a temperature protective agent. Further details of such agents are provided below.

To control tonicity, it is preferred to include a physiological salt, such as a sodium salt. Sodium chloride (NaCl is preferred, which may be present at between 1 and 20 mg/ml *e.g*. about 10±2mg/ml NaCl. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg.

Compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range. The pH of a composition will generally be between 5.0 and 8.1, and more typically between 6.0 and 8.0 *e.g.* 6.5 and 7.5, or between 7.0 and 7.8.

The composition is preferably sterile. The composition is preferably non-pyrogenic e.g. containing <1 EU (endotoxin unit, a standard measure) per dose, and preferably <0.1 EU per dose. The composition is preferably gluten free.

The composition may include material for a single immunisation, or may include material for multiple immunisations (*i.e*. a 'multidose' kit). The inclusion of a preservative is preferred in multidose arrangements. As an alternative (or in addition) to including a preservative in multidose compositions, the compositions may be contained in a container having an aseptic adaptor for removal of material.

Human vaccines are typically administered in a dosage volume of about 0.5ml, although a half dose *(i. e.* about 0.25ml) may be administered to children.

Immunogenic compositions of the invention may also comprise one or more immunoregulatory agents. Preferably, one or more of the immunoregulatory agents include one or more adjuvants. The adjuvants may include a TH1 adjuvant and/or a TH2 adjuvant, further discussed below.

Adjuvants which may be used in compositions of the invention include, but are not limited to: A. Mineral-containing compositions

Mineral containing compositions suitable for use as adjuvants in the invention include mineral salts, such as aluminium salts and calcium salts (or mixtures thereof). Calcium salts include calcium phosphate (*e.g*. the "CAP" particles disclosed in ref. 92). Aluminum salts include hydroxides, phosphates, sulfates, *etc.,* with the salts taking any suitable form (*e.g.* gel, crystalline, amorphous, *etc*.). Adsorption to these salts is preferred. The mineral containing compositions may also be formulated as a particle of metal salt [93].

The adjuvants known as aluminum hydroxide and aluminum phosphate may be used. These names are conventional, but are used for convenience only, as neither is a precise description of the actual chemical compound which is present *(e.g.* see chapter 9 of reference 94)). The invention can use any of the "hydroxide" or "phosphate" adjuvants that are in general use as adjuvants. The adjuvants known as "aluminium hydroxide" are typically aluminium oxyhydroxide salts, which are usually at least partially crystalline. The adjuvants known as "aluminium phosphate" are typically aluminium hydroxyphosphates, often also containing a small amount of sulfate (*i.e*. aluminium hydroxyphosphate sulfate). They may be obtained by precipitation, and the reaction conditions and concentrations during precipitation influence the degree of substitution of phosphate for hydroxyl in the salt.

A fibrous morphology (*e.g.* as seen in transmission electron micrographs) is typical for aluminium hydroxide adjuvants. The pI of aluminium hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminium hydroxide adjuvants.

Aluminium phosphate adjuvants generally have a PO₄/Al molar ratio between 0.3 and 1.2, preferably between 0.8 and 1.2, and more preferably 0.95±0.1. The aluminium phosphate will generally be amorphous, particularly for hydroxyphosphate salts. A typical adjuvant is amorphous aluminium hydroxyphosphate with PO₄/Al molar ratio between 0.84 and 0.92, included at 0.6mg Al³⁺/ml. The aluminium phosphate will generally be particulate (*e.g.* plate-like morphology as seen in transmission electron micrographs). Typical diameters of the particles are in the range 0.5-20µm *(e.g.* about 5-10µm) after any antigen adsorption. Adsorptive capacities of between 0.7-1.5 mg protein per mg Al⁺⁺⁺ at pH 7.4 have been reported for aluminium phosphate adjuvants.

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g.* about 5.7. Suspensions of aluminium salts used to prepare compositions of the invention may contain a buffer *(e.g.* a phosphate or a histidine or a Tris buffer), but this is not always necessary. The suspensions are preferably sterile and pyrogen-free. A suspension may include free aqueous phosphate ions *e.g*. present at a concentration between 1.0 and 20 mM, preferably between 5 and 15 mM, and more preferably about 10 mM. The suspensions may also comprise sodium chloride.

The invention can use a mixture of both an aluminium hydroxide and an aluminium phosphate. In this case there may be more aluminium phosphate than hydroxide *e.g*. a weight ratio of at least 2:1 *e.g.* ≥5:1, ≥6:1, ≥7:1, ≥8:1, ≥9:1, *etc.*

The concentration of Al⁺⁺⁺ in a composition for administration to a patient is preferably less than 10mg/ml *e.g.* ≤5 mg/ml, ≤4 mg/ml, ≤3 mg/ml, ≤2 mg/ml, ≤1 mg/ml, *etc.* A preferred range is between 0.3 and 1mg/ml. A maximum of 0.85mg/dose is preferred.

Aluminium phosphates are particularly preferred, particularly in compositions which include a *H.influenzae* saccharide antigen, and a typical adjuvant is amorphous aluminium hydroxyphosphate with PO₄/Al molar ratio between 0.84 and 0.92, included at 0.6mg Al³⁺/ml. Adsorption with a low dose of aluminium phosphate may be used *e.g*. between 50 and 100µg Al³⁺ per conjugate per dose. Where there is more than one conjugate in a composition, not all conjugates need to be adsorbed.

### B. Oil Emulsions

Oil emulsion compositions suitable for use as adjuvants in the invention include squalene-water emulsions, such as MF59 [Chapter 10 of ref. 94; see also ref. 95] (5% Squalene, 0.5% Tween 80, and 0.5% Span 85, formulated into submicron particles using a microfluidizer). Complete Freund's adjuvant (CFA) and incomplete Freund's adjuvant (IFA) may also be used.

Various oil-in-water emulsion adjuvants are known, and they typically include at least one oil and at least one surfactant, with the oil(s) and surfactant(s) being biodegradable (metabolisable) and biocompatible. The oil droplets in the emulsion are generally less than 5µm in diameter, and ideally have a sub-micron diameter, with these small sizes being achieved with a microfluidiser to provide stable emulsions. Droplets with a size less than 220nm are preferred as they can be subjected to filter sterilization.

The emulsion can comprise oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used e.g. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. Preferred surfactants for including in the emulsion are Tween 80 (polyoxyethylene sorbitan monooleate), Span 85 (sorbitan trioleate), lecithin and Triton X-100.

Mixtures of surfactants can be used *e.g*. Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%. Preferred emulsion adjuvants have an average droplets size of <1µm *e.g.* ≤750nm, ≤500nm, ≤400nm, ≤300nm, ≤250nm, ≤220nm, ≤200nm, or smaller. These droplet sizes can conveniently be achieved by techniques such as microfluidisation.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [96-98], as described in more detail in Chapter 10 of ref. 99 and chapter 12 of ref. 100. The MF59 emulsion advantageously includes citrate ions *e.g.* 10mM sodium citrate buffer.
- An emulsion of squalene, a tocopherol, and polysorbate 80 (Tween 80). The emulsion may include phosphate buffered saline. It may also include Span 85 *(e.g.* at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. Squalene and Tween 80 may be present volume ratio of about 5:2 or at a weight ratio of about 11:5. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g*. with an average diameter of between 100 and 250nm, preferably about 180nm. The emulsion may also include a 3-de-O-acylated monophosphoryl lipid A (3d-MPL). Another useful emulsion of this type may comprise, per human dose, 0.5-10 mg squalene, 0.5-11 mg tocopherol, and 0.1-4 mg polysorbate 80 [101].
- An emulsion of squalene, a tocopherol, and a Triton detergent (*e.g*. Triton X-100). The emulsion may also include a 3d-MPL (see below). The emulsion may contain a phosphate buffer.
- An emulsion comprising a polysorbate *(e.g.* polysorbate 80), a Triton detergent *(e.g.* Triton X-100) and a tocopherol (*e.g*. an α-tocopherol succinate). The emulsion may include these three components at a mass ratio of about 75:11:10 (*e.g*. 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [102] (0.05-1% Thr-MDP, 5% squalane, 2.5% Pluronic L121 and 0.2% polysorbate 80). It can also be used without the Thr-MDP, as in the "AF" adjuvant [103] (5% squalane, 1.25% Pluronic L121 and 0.2% polysorbate 80). Microfluidisation is preferred.
- An emulsion comprising squalene, an aqueous solvent, a polyoxyethylene alkyl ether hydrophilic nonionic surfactant (*e.g*. polyoxyethylene (12) cetostearyl ether) and a hydrophobic nonionic surfactant (*e.g*. a sorbitan ester or mannide ester, such as sorbitan monoleate or 'Span 80'). The emulsion is preferably thermoreversible and/or has at least 90% of the oil droplets (by volume) with a size less than 200 nm [104]. The emulsion may also include one or more of: alditol; a cryoprotective agent *(e.g.* a sugar, such as dodecylmaltoside and/or sucrose); and/or an alkylpolyglycoside. The emulsion may include a TLR4 agonist [105]. Such emulsions may be lyophilized.
- An emulsion of squalene, poloxamer 105 and Abil-Care [106]. The final concentration (weight) of these components in adjuvanted vaccines are 5% squalene, 4% poloxamer 105 (pluronic polyol) and 2% Abil-Care 85 (Bis-PEG/PPG-16/16 PEG/PPG-16/16 dimethicone; caprylic/capric triglyceride).
- An emulsion having from 0.5-50% of an oil, 0.1-10% of a phospholipid, and 0.05-5% of a non-ionic surfactant. As described in reference 107, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 108, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin *(e.g.* QuilA or QS21) and a sterol *(e.g.* a cholesterol) are associated as helical micelles [109].
- An emulsion comprising a mineral oil, a non-ionic lipophilic ethoxylated fatty alcohol, and a non-ionic hydrophilic surfactant (*e.g*. an ethoxylated fatty alcohol and/or polyoxyethylene-polyoxypropylene block copolymer) [110].
- An emulsion comprising a mineral oil, a non-ionic hydrophilic ethoxylated fatty alcohol, and a non-ionic lipophilic surfactant (*e.g*. an ethoxylated fatty alcohol and/or polyoxyethylene-polyoxypropylene block copolymer) [110].

In some embodiments an emulsion may be mixed with antigen extemporaneously, at the time of delivery, and thus the adjuvant and antigen may be kept separately in a packaged or distributed vaccine, ready for final formulation at the time of use. In other embodiments an emulsion is mixed with antigen during manufacture, and thus the composition is packaged in a liquid adjuvanted form,. The antigen will generally be in an aqueous form, such that the vaccine is finally prepared by mixing two liquids. The volume ratio of the two liquids for mixing can vary (*e.g*. between 5:1 and 1:5) but is generally about 1:1. Where concentrations of components are given in the above descriptions of specific emulsions, these concentrations are typically for an undiluted composition, and the concentration after mixing with an antigen solution will thus decrease.

Where a composition includes a tocopherol, any of the α, β, γ, δ, ε or ξ tocopherols can be used, but α-tocopherols are preferred. The tocopherol can take several forms *e.g*. different salts and/or isomers. Salts include organic salts, such as succinate, acetate, nicotinate, *etc.* D-α-tocopherol and DL-α-tocopherol can both be used. Tocopherols are advantageously included in vaccines for use in elderly patients (*e.g*. aged 60 years or older) because vitamin E has been reported to have a positive effect on the immune response in this patient group [111]. They also have antioxidant properties that may help to stabilize the emulsions [112]. A preferred α-tocopherol is DL-α-tocopherol, and the preferred salt of this tocopherol is the succinate. The succinate salt has been found to cooperate with TNF-related ligands *in vivo.*

### C. Saponin formulations [chatter 22 of ref. 94]

Saponin formulations may also be used as adjuvants in the invention. Saponins are a heterogeneous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™.

Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 113. Saponin formulations may also comprise a sterol, such as cholesterol [114].

Combinations of saponins and cholesterols can be used to form unique particles called immunostimulating complexs (ISCOMs) [chapter 23 of ref. 94]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 114-116. Optionally, the ISCOMS may be devoid of additional detergent [117].

A review of the development of saponin based adjuvants can be found in refs. 118 & 119. D. Virosomes and virus-like particles

Virosomes and virus-like particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein p1). VLPs are discussed further in refs. 120-125. Virosomes are discussed further in, for example, ref. 126

### E. Bacterial or microbial derivatives

Adjuvants suitable for use in the invention include bacterial or microbial derivatives such as non-toxic derivatives of enterobacterial lipopolysaccharide (LPS), Lipid A derivatives, immunostimulatory oligonucleotides and ADP-ribosylating toxins and detoxified derivatives thereof.

Non-toxic derivatives of LPS include monophosphoryl lipid A (MPL) and 3-O-deacylated MPL (3dMPL). 3dMPL is a mixture of 3 de-O-acylated monophosphoryl lipid A with 4, 5 or 6 acylated chains. A preferred "small particle" form of 3 De-O-acylated monophosphoryl lipid A is disclosed in ref. 127. Such "small particles" of 3dMPL are small enough to be sterile filtered through a 0.22µm membrane [127]. Other non-toxic LPS derivatives include monophosphoryl lipid A mimics, such as aminoalkyl glucosaminide phosphate derivatives *e.g*. RC-529 [128,129].

Lipid A derivatives include derivatives of lipid A from *Escherichia coli* such as OM-174. OM-174 is described for example in refs. 130 & 131.

Immunostimulatory oligonucleotides suitable for use as adjuvants in the invention include nucleotide sequences containing a CpG motif (a dinucleotide sequence containing an unmethylated cytosine linked by a phosphate bond to a guanosine). Double-stranded RNAs and oligonucleotides containing palindromic or poly(dG) sequences have also been shown to be immunostimulatory.

The CpG's can include nucleotide modifications/analogs such as phosphorothioate modifications and can be double-stranded or single-stranded. References 132, 133 and 134 disclose possible analog substitutions *e.g*. replacement of guanosine with 2'-deoxy-7-deazaguanosine. The adjuvant effect of CpG oligonucleotides is further discussed in refs. 135-140.

The CpG sequence may be directed to TLR9, such as the motif GTCGTT or TTCGTT [141]. The CpG sequence may be specific for inducing a Th1 immune response, such as a CpG-A ODN, or it may be more specific for inducing a B cell response, such a CpG-B ODN. CpG-A and CpG-B ODNs are discussed in refs. 142-144. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, refs. 141 & 145-147.

A useful CpG adjuvant is CpG7909, also known as ProMune™ (Coley Pharmaceutical Group, Inc.). Another is CpG1826. As an alternative, or in addition, to using CpG sequences, TpG sequences can be used [148], and these oligonucleotides may be free from unmethylated CpG motifs. The immunostimulatory oligonucleotide may be pyrimidine-rich. For example, it may comprise more than one consecutive thymidine nucleotide (*e.g*. TTTT, as disclosed in ref. 148), and/or it may have a nucleotide composition with >25% thymidine *(e.g.* >35%, >40%, >50%, >60%, >80%, *etc.).* For example, it may comprise more than one consecutive cytosine nucleotide *(e.g.* CCCC, as disclosed in ref. 148), and/or it may have a nucleotide composition with >25% cytosine *(e.g.* >35%, >40%, >50%, >60%, >80%, *etc.).* These oligonucleotides may be free from unmethylated CpG motifs. Immunostimulatory oligonucleotides will typically comprise at least 20 nucleotides. They may comprise fewer than 100 nucleotides.

A particularly useful adjuvant based around immunostimulatory oligonucleotides is known as IC-31™ [149]. Thus an adjuvant used with the invention may comprise a mixture of (i) an oligonucleotide (*e.g*. between 15-40 nucleotides) including at least one (and preferably multiple) CpI motifs (*i.e.* a cytosine linked to an inosine to form a dinucleotide), and (ii) a polycationic polymer, such as an oligopeptide (*e.g*. between 5-20 amino acids) including at least one (and preferably multiple) Lys-Arg-Lys tripeptide sequence(s). The oligonucleotide may be a deoxynucleotide comprising 26-mer sequence 5'-(IC)₁₃-3' (SEQ ID NO: 230). The polycationic polymer may be a peptide comprising 11-mer amino acid sequence KLKLLLLLKLK (SEQ ID NO: 231). The oligonucleotide and polymer can form complexes *e.g*. as disclosed in references 150 & 151.

Bacterial ADP-ribosylating toxins and detoxified derivatives thereof may be used as adjuvants in the invention. Preferably, the protein is derived from *E.coli* (*E.coli* heat labile enterotoxin "LT"), cholera ("CT"), or pertussis ("PT"). The use of detoxified ADP-ribosylating toxins as mucosal adjuvants is described in ref. 152 and as parenteral adjuvants in ref. 153. The toxin or toxoid is preferably in the form of a holotoxin, comprising both A and B subunits. Preferably, the A subunit contains a detoxifying mutation; preferably the B subunit is not mutated. Preferably, the adjuvant is a detoxified LT mutant such as LT-K63, LT-R72, and LT-G192. The use of ADP-ribosylating toxins and detoxified derivatives thereof, particularly LT-K63 and LT-R72, as adjuvants can be found in refs. 154-161. A useful CT mutant is or CT-E29H [162]. Numerical reference for amino acid substitutions is preferably based on the alignments of the A and B subunits of ADP-ribosylating toxins set forth in ref. 163.

### F. Human immunomodulators

Human immunomodulators suitable for use as adjuvants in the invention include cytokines, such as interleukins *(e.g.* IL-1, IL-2, IL-4, IL-5, IL-6, IL-7, IL-12 [164], *etc.)* [165], interferons *(e.g.* interferon-γ), macrophage colony stimulating factor, and tumor necrosis factor. A preferred immunomodulator is IL-12.

### G. Bioadhesives and Mucoadhesives

Bioadhesives and mucoadhesives may also be used as adjuvants in the invention. Suitable bioadhesives include esterified hyaluronic acid microspheres [166] or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention [167].

### H. Microparticles

Microparticles may also be used as adjuvants in the invention. Microparticles (*i.e*. a particle of ∼100nm to ∼150µm in diameter, more preferably ∼200nm to ∼30µm in diameter, and most preferably ∼500nm to ∼10µm in diameter) formed from materials that are biodegradable and non-toxic (*e.g*. a poly(α-hydroxy acid), a polyhydroxybutyric acid, a polyorthoester, a polyanhydride, a polycaprolactone, *etc.),* with poly(lactide-co-glycolide) are preferred, optionally treated to have a negatively-charged surface *(e.g.* with SDS) or a positively-charged surface *(e.g.* with a cationic detergent, such as CTAB).

### I. Liposomes (Chapters 13 & 14 of ref. 94)

Examples of liposome formulations suitable for use as adjuvants are described in refs. 168-170.

### J. Polyoxyethylene ether and polyoxyethylene ester formulations

Adjuvants suitable for use in the invention include polyoxyethylene ethers and polyoxyethylene esters [171]. Such formulations further include polyoxyethylene sorbitan ester surfactants in combination with an octoxynol [172] as well as polyoxyethylene alkyl ethers or ester surfactants in combination with at least one additional non-ionic surfactant such as an octoxynol [173]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### K. Phosphazenes

A phosphazene, such as poly[di(carboxylatophenoxy)phosphazene] ("PCPP") as described, for example, in references 174 and 175, may be used.

### L. Muramyl peptides

Examples of muramyl peptides suitable for use as adjuvants in the invention include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-*sn-*glycero-3-hydroxyphosphoryloxy)-ethylamine MTP-PE). M. Imidazoquinolone Compounds.

Examples of imidazoquinolone compounds suitable for use adjuvants in the invention include Imiquimod ("R-837") [176,177], Resiquimod ("R-848") [178], and their analogs; and salts thereof *(e.g.* the hydrochloride salts). Further details about immunostimulatory imidazoquinolines can be found in references 179 to 183.

### N. Substituted ureas

Substituted ureas useful as adjuvants include compounds of formula I, II or III, or salts thereof: 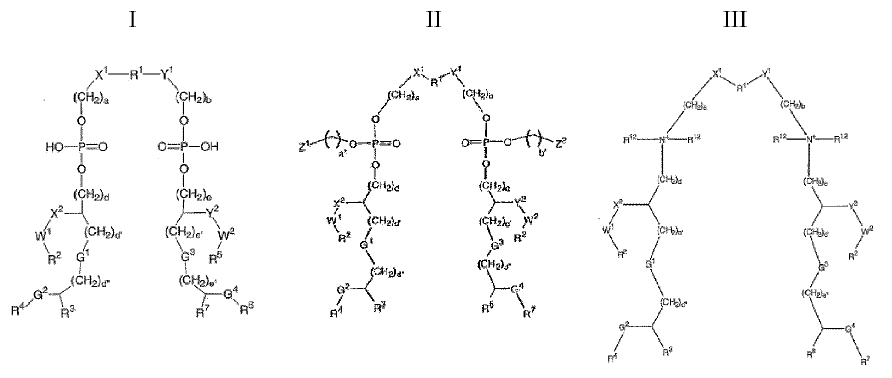 as defined in reference 184, such as 'ER 803058', 'ER 803732', 'ER 804053', ER 804058', 'ER 804059', 'ER 804442', 'ER 804680', 'ER 804764', ER 803022 or 'ER 804057' e.g.:

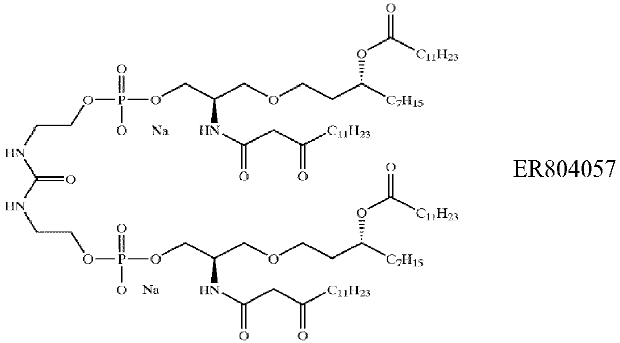

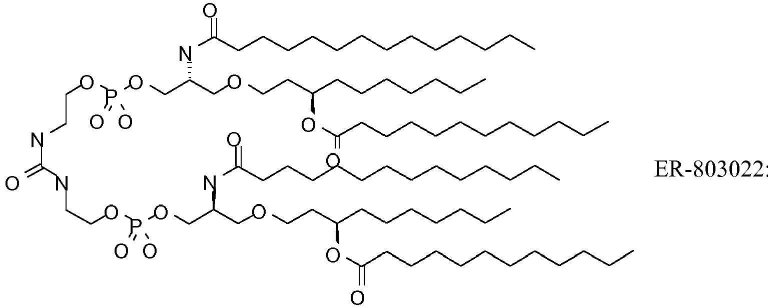

### O. Further adjuvants

Further adjuvants that may be used with the invention include:
- An aminoalkyl glucosaminide phosphate derivative, such as RC-529 [185,186].
- A thiosemicarbazone compound, such as those disclosed in reference 187. Methods of formulating, manufacturing, and screening for active compounds are also described in reference 187. The thiosemicarbazones are particularly effective in the stimulation of human peripheral blood mononuclear cells for the production of cytokines, such as TNF-α.
- A tryptanthrin compound, such as those disclosed in reference 188. Methods of formulating, manufacturing, and screening for active compounds are also described in reference 188. The thiosemicarbazones are particularly effective in the stimulation of human peripheral blood mononuclear cells for the production of cytokines, such as TNF-α.
- A nucleoside analog, such as: (a) Isatorabine (ANA-245; 7-thia-8-oxoguanosine): 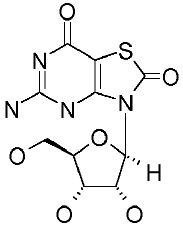 and prodrugs thereof; (b) ANA975; (c) ANA-025-1; (d) ANA380; (e) the compounds disclosed in references 189 to 191Loxoribine (7-allyl-8-oxoguanosine) [192].
- Compounds disclosed in reference 193, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [194,195], Hydrapthalamide compounds, Benzophenone compounds, Isoxazole compounds, Sterol compounds, Quinazilinone compounds, Pyrrole compounds [196], Anthraquinone compounds, Quinoxaline compounds, Triazine compounds, Pyrazalopyrimidine compounds, and Benzazole compounds [197].
- Compounds containing lipids linked to a phosphate-containing acyclic backbone, such as the TLR4 antagonist E5564 [198,199]:
- A polyoxidonium polymer [200,201] or other N-oxidized polyethylene-piperazine derivative.
- Methyl inosine 5'-monophosphate ("MIMP") [202].
- A polyhydroxlated pyrrolizidine compound [203], such as one having formula: 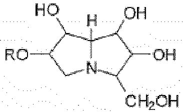 where R is selected from the group comprising hydrogen, straight or branched, unsubstituted or substituted, saturated or unsaturated acyl, alkyl (*e.g*. cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi*-casuarine, 7-*epi*-casuarine, 3,7-di*epi*-casuarine, *etc.*
- A CD1d ligand, such as an α-glycosylceramide [204-211] (*e.g*. α-galactosylceramide), phytosphingosine-containing α-glycosylceramides, OCH, KRN7000 [(2S,3S,4R)-1-O-(α-D-galactopyranosyl)-2-(N-hexacosanoylamino)-1,3,4-octadecanetriol], CRONY-101, 3"-O-sulfo-galactosylceramide, *etc.*
- A gamma inulin [212] or derivative thereof, such as algammulin.

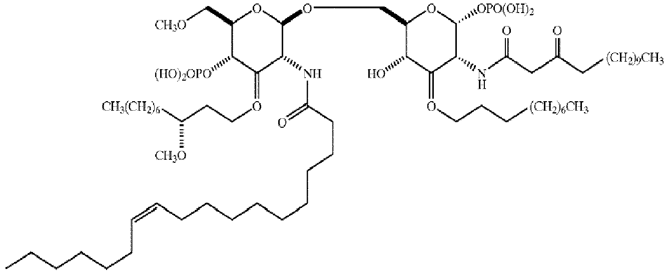

### Adjuvant combinations

The invention may also comprise combinations of aspects of one or more of the adjuvants identified above. For example, the following adjuvant compositions may be used in the invention: (1) a saponin and an oil-in-water emulsion [213]; (2) a saponin *(e.g.* QS21) + a non-toxic LPS derivative *(e.g.* 3dMPL) [214]; (3) a saponin *(e.g.* QS21) + a non-toxic LPS derivative *(e.g.* 3dMPL) + a cholesterol; (4) a saponin (e.g. QS21) + 3dMPL + IL-12 (optionally + a sterol) [215]; (5) combinations of 3dMPL with, for example, QS21 and/or oil-in-water emulsions [216]; (6) SAF, containing 10% squalane, 0.4% Tween 80™, 5% pluronic-block polymer L121, and thr-MDP, either microfluidized into a submicron emulsion or vortexed to generate a larger particle size emulsion. (7) Ribi™ adjuvant system (RAS), (Ribi Immunochem) containing 2% squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and (8) one or more mineral salts (such as an aluminum salt) + a non-toxic derivative of LPS (such as 3dMPL).

Other substances that act as immunostimulating agents are disclosed in chapter 7 of ref. 94.

The use of an aluminium hydroxide and/or aluminium phosphate adjuvant is particularly preferred, and antigens are generally adsorbed to these salts. Calcium phosphate is another preferred adjuvant. Other preferred adjuvant combinations include combinations of Th1 and Th2 adjuvants such as CpG & alum or resiquimod & alum. A combination of aluminium phosphate and 3dMPL maybe used, as this has been reported as effective in pneumococcal immunisation [325]. The compositions of the invention may elicit both a cell mediated immune response as well as a humoral immune response. This immune response will preferably induce long lasting (*e.g*. neutralising) antibodies and a cell mediated immunity that can quickly respond upon exposure to pnuemococcus.

Two types of T cells, CD4 and CD8 cells, are generally thought necessary to initiate and/or enhance cell mediated immunity and humoral immunity. CD8 T cells can express a CD8 co-receptor and are commonly referred to as Cytotoxic T lymphocytes (CTLs). CD8 T cells are able to recognized or interact with antigens displayed on MHC Class I molecules.

CD4 T cells can express a CD4 co-receptor and are commonly referred to as T helper cells. CD4 T cells are able to recognize antigenic peptides bound to MHC class II molecules. Upon interaction with a MHC class II molecule, the CD4 cells can secrete factors such as cytokines. These secreted cytokines can activate B cells, cytotoxic T cells, macrophages, and other cells that participate in an immune response. Helper T cells or CD4+ cells can be further divided into two functionally distinct subsets: TH1 phenotype and TH2 phenotypes which differ in their cytokine and effector function.

Activated TH1 cells enhance cellular immunity (including an increase in antigen-specific CTL production) and are therefore of particular value in responding to intracellular infections. Activated TH1 cells may secrete one or more of IL-2, IFN-γ, and TNF-β. A TH1 immune response may result in local inflammatory reactions by activating macrophages, NK (natural killer) cells, and CD8 cytotoxic T cells (CTLs). A TH1 immune response may also act to expand the immune response by stimulating growth of B and T cells with IL-12. TH1 stimulated B cells may secrete IgG2a.

Activated TH2 cells enhance antibody production and are therefore of value in responding to extracellular infections. Activated TH2 cells may secrete one or more of IL-4, IL-5, IL-6, and IL-10. A TH2 immune response may result in the production of IgG1, IgE, IgA and memory B cells for future protection.

An enhanced immune response may include one or more of an enhanced TH1 immune response and a TH2 immune response.

A TH1 immune response may include one or more of an increase in CTLs, an increase in one or more of the cytokines associated with a TH1 immune response (such as IL-2, IFN-γ, and TNF-β), an increase in activated macrophages, an increase in NK activity, or an increase in the production of IgG2a. Preferably, the enhanced TH1 immune response will include an increase in IgG2a production.

A TH1 immune response may be elicited using a TH1 adjuvant. A TH1 adjuvant will generally elicit increased levels of IgG2a production relative to immunization of the antigen without adjuvant. TH1 adjuvants suitable for use in the invention may include for example saponin formulations, virosomes and virus like particles, non-toxic derivatives of enterobacterial lipopolysaccharide (LPS), immunostimulatory oligonucleotides. Immunostimulatory oligonucleotides, such as oligonucleotides containing a CpG motif, are preferred TH1 adjuvants for use in the invention.

A TH2 immune response may include one or more of an increase in one or more of the cytokines associated with a TH2 immune response (such as IL-4, IL-5, IL-6 and IL-10), or an increase in the production of IgG1, IgE, IgA and memory B cells. Preferably, the enhanced TH2 immune resonse will include an increase in IgG1 production.

A TH2 immune response may be elicited using a TH2 adjuvant. A TH2 adjuvant will generally elicit increased levels of IgG1 production relative to immunization of the antigen without adjuvant. TH2 adjuvants suitable for use in the invention include, for example, mineral containing compositions, oil-emulsions, and ADP-ribosylating toxins and detoxified derivatives thereof. Mineral containing compositions, such as aluminium salts are preferred TH2 adjuvants for use in the invention.

Preferably, the invention includes a composition comprising a combination of a TH1 adjuvant and a TH2 adjuvant. Preferably, such a composition elicits an enhanced TH1 and an enhanced TH2 response, i.e., an increase in the production of both IgG1 and IgG2a production relative to immunization without an adjuvant. Still more preferably, the composition comprising a combination of a TH1 and a TH2 adjuvant elicits an increased TH1 and/or an increased TH2 immune response relative to immunization with a single adjuvant (*i.e.,* relative to immunization with a TH1 adjuvant alone or immunization with a TH2 adjuvant alone).

The immune response may be one or both of a TH1 immune response and a TH2 response. Preferably, immune response provides for one or both of an enhanced TH1 response and an enhanced TH2 response.

The enhanced immune response may be one or both of a systemic and a mucosal immune response. Preferably, the immune response provides for one or both of an enhanced systemic and an enhanced mucosal immune response. Preferably the mucosal immune response is a TH2 immune response. Preferably, the mucosal immune response includes an increase in the production of IgA.

Pneumococcal infections can affect various areas of the body and so the compositions of the invention may be prepared in various forms. For example, the compositions may be prepared as injectables, either as liquid solutions or suspensions. Solid forms suitable for solution in, or suspension in, liquid vehicles prior to injection can also be prepared *(e.g.* a lyophilised composition or a spray-freeze dried composition). The composition may be prepared for topical administration *e.g.* as an ointment, cream or powder. The composition may be prepared for oral administration *e.g.* as a tablet or capsule, as a spray, or as a syrup (optionally flavoured). The composition may be prepared for pulmonary administration *e.g*. as an inhaler, using a fine powder or a spray. The composition may be prepared as a suppository or pessary. The composition may be prepared for nasal, aural or ocular administration *e*.*g*. as drops. The composition may be in kit form, designed such that a combined composition is reconstituted just prior to administration to a patient. Such kits may comprise one or more antigens in liquid form and one or more lyophilised antigens.

Where a composition is to be prepared extemporaneously prior to use (*e.g*. where a component is presented in lyophilised form) and is presented as a kit, the kit may comprise two vials, or it may comprise one ready-filled syringe and one vial, with the contents of the syringe being used to reactivate the contents of the vial prior to injection.

Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated *(e.g.* non-human primate, primate, *etc.),* the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Where more than one antigen is included in a composition then two antigens may be present at the same dose as each other or at different doses.

As mentioned above, a composition may include a temperature protective agent, and this component may be particularly useful in adjuvanted compositions (particularly those containing a mineral adjuvant, such as an aluminium salt). As described in reference 217, a liquid temperature protective agent may be added to an aqueous vaccine composition to lower its freezing point *e.g.* to reduce the freezing point to below 0°C. Thus the composition can be stored below 0°C, but above its freezing point, to inhibit thermal breakdown. The temperature protective agent also permits freezing of the composition while protecting mineral salt adjuvants against agglomeration or sedimentation after freezing and thawing, and may also protect the composition at elevated temperatures *e.g*. above 40°C. A starting aqueous vaccine and the liquid temperature protective agent may be mixed such that the liquid temperature protective agent forms from 1-80% by volume of the final mixture. Suitable temperature protective agents should be safe for human administration, readily miscible/soluble in water, and should not damage other components (*e.g*. antigen and adjuvant) in the composition. Examples include glycerin, propylene glycol, and/or polyethylene glycol (PEG). Suitable PEGs may have an average molecular weight ranging from 200-20,000 Da. In a preferred embodiment, the polyethylene glycol can have an average molecular weight of about 300 Da ('PEG-300').

The invention provides an immunogenic composition comprising: (i) one or more antigen(s) selected from the first, second, third, fourth, fifth, sixth, seventh, eighth, ninth or tenth antigen groups; and (ii) a temperature protective agent. This composition may be formed by mixing (i) an aqueous composition comprising one or more antigen(s) selected from the first, second, third, fourth, fifth, sixth, seventh, eighth, ninth or tenth antigen groups, with (ii) a temperature protective agent. The mixture may then be stored e.g. below 0°C, from 0-20°C, from 20-35°C, from 35-55°C, or higher. It may be stored in liquid or frozen form. The mixture may be lyophilised. The composition may alternatively be formed by mixing (i) a dried composition comprising one or more antigen(s) selected from the first, second, third, fourth, fifth, sixth, seventh, eighth, ninth or tenth antigen groups, with (ii) a liquid composition comprising the temperature protective agent. Thus component (ii) can be used to reconstitute component (i).

### Methods of treatment, and administration of the vaccine

The invention also provides an immunogenic composition of the invention for use in a method for raising an immune response in a mammal. The immune response is preferably protective and preferably involves antibodies and/or cell-mediated immunity. The method may raise a booster response.

The invention also provides at least two antigens of the invention for combined use as a medicament *e.g.* for use in raising an immune response in a mammal.

The invention also provides the at least two antigens of the invention for use in the manufacture of a medicament for raising an immune response in a mammal.

By raising an immune response in the mammal by these uses and methods, the mammal can be protected against pneumococcal infection. More particularly, the mammal may be protected against pneumococcal meningitis. The invention is effective against pneumococci of various different serotypes, but can be particularly useful in protecting against disease resulting from pneumococcal infection by strains in serotype 1, 5, 6 and 19A.

The disclosure also provides a kit comprising a first component and a second component wherein neither the first component nor the second component is a composition of the invention as described above, but wherein the first component and the second component can be combined to provide a composition of the invention as described above. The kit may further include a third component comprising one or more of the following: instructions, syringe or other delivery device, adjuvant, or pharmaceutically acceptable formulating solution.

The disclosure also provides a delivery device pre-filled with an immunogenic composition of the invention.

The mammal is preferably a human. Where the vaccine is for prophylactic use, the human is preferably a child *(e.g.* a toddler or infant) or a teenager; where the vaccine is for therapeutic use, the human is preferably a teenager or an adult. A vaccine intended for children may also be administered to adults *e.g.* to assess safety, dosage, immunogenicity, *etc.*

One way of checking efficacy of therapeutic treatment involves monitoring pneumococcal infection after administration of the compositions of the invention. One way of checking efficacy of prophylactic treatment involves monitoring immune responses, systemically (such as monitoring the level of IgG1 and IgG2a production) and/or mucosally (such as monitoring the level of IgA production), against the antigens in the compositions of the invention after administration of the composition. Typically, antigen-specific serum antibody responses are determined post-immunisation but pre-challenge whereas antigen-specific mucosal antibody responses are determined post-immunisation and post-challenge.

Another way of assessing the immunogenicity of the compositions of the present invention is to express the proteins recombinantly for screening patient sera or mucosal secretions by immunoblot and/or microarrays. A positive reaction between the protein and the patient sample indicates that the patient has mounted an immune response to the protein in question. This method may also be used to identify immunodominant antigens and/or epitopes within antigens.

The efficacy of vaccine compositions can also be determined *in vivo* by challenging animal models of pneumococcal infection, *e.g*., guinea pigs or mice, with the vaccine compositions. One such model is described in reference 218.

Compositions of the invention will generally be administered directly to a patient. Direct delivery may be accomplished by parenteral injection (*e.g*. subcutaneously, intraperitoneally, intravenously, intramuscularly, or to the interstitial space of a tissue), or mucosally, such as by rectal, oral *(e.g.* tablet, spray), vaginal, topical, transdermal or transcutaneous, intranasal, ocular, aural, pulmonary or other mucosal administration.

Compositions of the invention may be used to elicit systemic and/or mucosal immunity, preferably to elicit an enhanced systemic and/or mucosal immunity.

Preferably the enhanced systemic and/or mucosal immunity is reflected in an enhanced TH1 and/or TH2 immune response. Preferably, the enhanced immune response includes an increase in the production of IgG1 and/or IgG2a and/or IgA.

Dosage can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Multiple doses will typically be administered at least 1 week apart (e.g. about 2 weeks, about 3 weeks, about 4 weeks, about 6 weeks, about 8 weeks, about 10 weeks, about 12 weeks, about 16 weeks, *etc.).*

Vaccines of the invention may be for use in treating both children and adults. Thus a human patient maybe less than 1 year old, 1-5 years old, 5-15 years old, 15-55 years old, or at least 55 years old. Preferred patients for receiving the vaccines are the elderly (*e.g.* ≥50 years old, ≥60 years old, and preferably ≥65 years), the young (*e.g.* ≤5 years old), hospitalised patients, healthcare workers, armed service and military personnel, pregnant women, the chronically ill, or immunodeficient patients. The vaccines are not suitable solely for these groups, however, and may be used more generally in a population.

Vaccines produced by the invention may be administered to patients at substantially the same time as (*e.g*. during the same medical consultation or visit to a healthcare professional or vaccination centre) other vaccines *e.g*. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, *etc.*

### Mucosal immunisation

The invention provides an immunogenic composition comprising (i) a polypeptide antigen of the invention. The invention also provides an immunogenic composition of the invention for use in a method for raising an immune response in a mammal. The composition is preferably administered mucosally (to a mucosal surface) *e.g*. it may be administered intranasally.

The polypeptide antigen may be, for example, part of the seventh antigen group. The polypeptide antigen may be a pilus antigen, such as a RrgA or RrgB polypeptide.

The toxin of component (i) may be, for example, derived from *E.coli* heat labile enterotoxin ("LT"). The derivative may have a detoxifying mutation in its A subunit *e.g*. it may be LT-K63 or LT-R72.

Intranasal administration of a RrgB polypeptide and a LT-K63 adjuvant is preferred. In mice this has been shown to decrease bacterial load of an invasive pneumococcal strain in the nasopharynx, lungs and blood and to give a 5-fold increase in survival rate.

### Nucleic acid immunisation

The immunogenic compositions described above include polypeptide antigens from pneumococcus. In all cases, however, the polypeptide antigens can be replaced by nucleic acids (typically DNA) encoding those polypeptides, to give compositions, methods and uses based on nucleic acid immunisation. Nucleic acid immunisation is now a developed field *(e.g.* see references 219 to 226 *etc.),* and has been applied to pneumococcal vaccines *(e.g.* ref. 227).

The nucleic acid encoding the immunogen is expressed *in vivo* after delivery to a patient and the expressed immunogen then stimulates the immune system. The active ingredient will typically take the form of a nucleic acid vector comprising: (i) a promoter; (ii) a sequence encoding the immunogen, operably linked to the promoter; and optionally (iii) a selectable marker. Preferred vectors may further comprise (iv) an origin of replication; and (v) a transcription terminator downstream of and operably linked to (ii). In general, (i) & (v) will be eukaryotic and (iii) & (iv) will be prokaryotic.

Preferred promoters are viral promoters *e.g*. from cytomegalovirus (CMV). The vector may also include transcriptional regulatory sequences (*e.g*. enhancers) in addition to the promoter and which interact functionally with the promoter. Preferred vectors include the immediate-early CMV enhancer/promoter, and more preferred vectors also include CMV intron A. The promoter is operably linked to a downstream sequence encoding an immunogen, such that expression of the immunogen-encoding sequence is under the promoter's control.

Where a marker is used, it preferably functions in a microbial host *(e.g.* in a prokaryote, in a bacteria, in a yeast). The marker is preferably a prokaryotic selectable marker (*e.g*. transcribed under the control of a prokaryotic promoter). For convenience, typical markers are antibiotic resistance genes.

The vector of the invention is preferably an autonomously replicating episomal or extrachromosomal vector, such as a plasmid.

The vector of the invention preferably comprises an origin of replication. It is preferred that the origin of replication is active in prokaryotes but not in eukaryotes.

Preferred vectors thus include a prokaryotic marker for selection of the vector, a prokaryotic origin of replication, but a *eukaryotic* promoter for driving transcription of the immunogen-encoding sequence. The vectors will therefore (a) be amplified and selected in prokaryotic hosts without polypeptide expression, but (b) be expressed in eukaryotic hosts without being amplified. This arrangement is ideal for nucleic acid immunization vectors.

The vector of the invention may comprise a eukaryotic transcriptional terminator sequence downstream of the coding sequence. This can enhance transcription levels. Where the coding sequence does not have its own, the vector of the invention preferably comprises a polyadenylation sequence. A preferred polyadenylation sequence is from bovine growth hormone.

The vector of the invention may comprise a multiple cloning site

In addition to sequences encoding the immunogen and a marker, the vector may comprise a second eukaryotic coding sequence. The vector may also comprise an IRES upstream of said second sequence in order to permit translation of a second eukaryotic polypeptide from the same transcript as the immunogen. Alternatively, the immunogen-coding sequence may be downstream of an IRES.

The vector of the invention may comprise unmethylated CpG motifs *e.g*. unmethylated DNA sequences which have in common a cytosine preceding a guanosine, flanked by two 5' purines and two 3' pyrimidines. In their unmethylated form these DNA motifs have been demonstrated to be potent stimulators of several types of immune cell. Vectors may be delivered in a targeted way. Receptor-mediated DNA delivery techniques are described in, for example, references 228 to 233. Therapeutic compositions containing a nucleic acid are administered in a range of about 100ng to about 200mg of DNA for local administration in a gene therapy protocol. Concentration ranges of about 500 ng to about 50 mg, about 1 µg to about 2 mg, about 5µg to about 500µg, and about 20µg to about 100µg of DNA can also be used during a gene therapy protocol. Factors such as method of action (e.g. for enhancing or inhibiting levels of the encoded gene product) and efficacy of transformation and expression are considerations which will affect the dosage required for ultimate efficacy. Where greater expression is desired over a larger area of tissue, larger amounts of vector or the same amounts re-administered in a successive protocol of administrations, or several administrations to different adjacent or close tissue portions may be required to effect a positive therapeutic outcome. In all cases, routine experimentation in clinical trials will determine specific ranges for optimal therapeutic effect.

Vectors can be delivered using gene delivery vehicles. The gene delivery vehicle can be of viral or non-viral origin (see generally references 234 to 237).

Viral-based vectors for delivery of a desired nucleic acid and expression in a desired cell are well known in the art. Exemplary viral-based vehicles include, but are not limited to, recombinant retroviruses *(e.g.* references 238 to 248), alphavirus-based vectors *(e.g.* Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532); hybrids or chimeras of these viruses may also be used), poxvirus vectors (e.g. vaccinia, fowlpox, canarypox, modified vaccinia Ankara, *etc.),* adenovirus vectors, and adeno-associated virus (AAV) vectors (*e.g*. see refs. 249 to 254). Administration of DNA linked to killed adenovirus [255] can also be employed.

Non-viral delivery vehicles and methods can also be employed, including, but not limited to, polycationic condensed DNA linked or unlinked to killed adenovirus alone [*e.g.* 255], ligand-linked DNA [256], eukaryotic cell delivery vehicles cells [*e.g.* refs. 257 to 261] and nucleic charge neutralization or fusion with cell membranes. Naked DNA can also be employed. Exemplary naked DNA introduction methods are described in refs. 262 and 263. Liposomes (*e.g*. immunoliposomes) that can act as gene delivery vehicles are described in refs. 264 to 268. Additional approaches are described in references 269 & 270.

Further non-viral delivery suitable for use includes mechanical delivery systems such as the approach described in ref. 270. Moreover, the coding sequence and the product of expression of such can be delivered through deposition of photopolymerized hydrogel materials or use of ionizing radiation [*e.g.* refs. 271 & 272]. Other conventional methods for gene delivery that can be used for delivery of the coding sequence include, for example, use of hand-held gene transfer particle gun [273] or use of ionizing radiation for activating transferred genes [271 & 272]. Delivery DNA using PLG {poly(lactide-co-glycolide)} microparticles is a particularly preferred method *e.g*. by adsorption to the microparticles, which are optionally treated to have a negatively-charged surface (*e.g*. treated with SDS) or a positively-charged surface (*e.g*. treated with a cationic detergent, such as CTAB).

### Antibodies

Antibodies against pneumococcal antigens can be used for passive immunisation [274]. Thus the disclosure provides an antibody that is specific for an antigen in the first, second or third antigen groups. The disclosure also provides an antibody that is specific for an antigen in the fourth, fifth, sixth, seventh, eighth, ninth or tenth antigen groups. The disclosure also provides the use of such antibodies in therapy. The disclosure also provides the use of such antibodies in the manufacture of a medicament. The disclosure also provides a method for treating a mammal comprising the step of administering an effective amount of an antibody of the invention. As described above for immunogenic compositions, these methods and uses allow a mammal to be protected against pneumococcal infection.

The term "antibody" includes intact immunoglobulin molecules, as well as fragments thereof which are capable of binding an antigen. These include hybrid (chimeric) antibody molecules [275, 276]; F(ab')2 and F(ab) fragments and Fv molecules; non-covalent heterodimers [277, 278]; single-chain Fv molecules (sFv) [279]; dimeric and trimeric antibody fragment constructs; minibodies [280, 281]; humanized antibody molecules [282-284]; and any functional fragments obtained from such molecules, as well as antibodies obtained through non-conventional processes such as phage display. Preferably, the antibodies are monoclonal antibodies. Methods of obtaining monoclonal antibodies are well known in the art. Humanised or fully-human antibodies are preferred.

### General

The practice of the present invention will employ, unless otherwise indicated, conventional methods of chemistry, biochemistry, molecular biology, immunology and pharmacology, within the skill of the art. Such techniques are explained fully in the literature. See, *e.g.,* references 285-292, *etc.*

"GI" numbering is used above. A GI number, or "GenInfo Identifier", is a series of digits assigned consecutively to each sequence record processed by NCBI when sequences are added to its databases. The GI number bears no resemblance to the accession number of the sequence record. When a sequence is updated (*e.g*. for correction, or to add more annotation or information) then it receives a new GI number. Thus the sequence associated with a given GI number is never changed.

Where the invention concerns an "epitope", this epitope may be a B-cell epitope and/or a T-cell epitope. Such epitopes can be identified empirically (*e.g*. using PEPSCAN [293,294] or similar methods), or they can be predicted (*e.g*. using the Jameson-Wolf antigenic index [295], matrix-based approaches [296], MAPITOPE [297], TEPITOPE [298,299], neural networks [300], OptiMer & EpiMer [301, 302], ADEPT [303], Tsites [304], hydrophilicity [305], antigenic index [306] or the methods disclosed in references 307-311, *etc.).* Epitopes are the parts of an antigen that are recognised by and bind to the antigen binding sites of antibodies or T-cell receptors, and they may also be referred to as "antigenic determinants".

Where an antigen "domain" is omitted, this may involve omission of a signal peptide, of a cytoplasmic domain, of a transmembrane domain, of an extracellular domain, *etc.*

The term "comprising" encompasses "including" as well as "consisting" *e.g*. a composition "comprising" X may consist exclusively of X or may include something additional *e.g*. X + Y.

The term "about" in relation to a numerical value x means, for example, *x*±10%.

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of ref. 312. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in ref. 313.

### BRIEF DESCRIPTION OF DRAWINGS

Figure 1 shows the mortality course of mice after bacterial challenge, followed for up to 10 days, after immunisation with spr0565 or spr1431, compared to the mortality course of mice in a control group. Figure 2 shows similar data for combined antigens.
Figure 3 shows bacterial numbers (CFU/ml) in the blood of mice, comparing test groups with control (ctrl) groups. Circles are individual animals, and the bar is the geometric mean. Data are from animal groups: (3A) 0; (3B) 1; (3C) 4; (3D) 6. Figure 4 shows survival data (days) for the same groups. Diamonds are individual animals and bars shown the median.
Figure 5 shows mortality course as in Figure 2, but with different antigen combinations.
Figure 6 shows results of bacteremia experiments. The y-axis shows CFU/ml and the numbers beneath the x-axis show P values calculated by the U-test.
Figure 7 shows results of mortality experiments. The y-axis shows the survival time (days) and the numbers beneath the x-axis show P values calculated by the U-test.
Figures 8-25 and 28-31 and 33-36 follow the same pattern as figures 6 and 7.
Figure 26 shows the results of an OPKA assay at two serum dilutions (1/12 or 1/36). Each triplet shows data with a PBS control (left), combo-1 (middle) or combo-2 (right). Y-axis shows % killing.
Figure 27 shows results from OPKA experiments with diluted serum.
Figure 32 shows purification gels for hybrids of spr2021 and spr0096. The left-hand bands are the hybrid proteins and the right-hand bands are a BSA standard (64kDa). Figure 32A shows spr2021-spr0096 and Figure 32B shows spr0096-spr2021. **MODES FOR CARRYING OUT THE INVENTION**

### Antigen identification

Twelve pneumococcal polypeptides were selected for immunological investigation. Numbered according to the R6 genome [84], these twelve are: spr0057; spr0286; spr0565; spr0867; spr1098; spr1345; spr1416; spr1418; spr1431; spr1739; and spr2021.

The spr0057 antigen is annotated as β-N-acetylhexosaminidase (strH), which cleaves N-acetylglucosamine on human glycoproteins [314]. Thus the enzyme might facilitate pathogenesis/ colonisation of humans, and blocking it might be useful for vaccination purposes. Moreover, the protein is surface-located, being LPxTG-anchored, and immunoreactive sera have been seen in convalescent pneumonia and meningitis patients [315]. The spr0057 sequence is 98.6% conserved between 22 different pneumococcal strains, and may thus offer broad protection. It is absent from *S.mitis* and *S.mutans.* There are no published studies on the protective efficacy of spr0057 as an immunogen. The wild-type spr0057 gene is 3939 nucleotides long, but for immunisation purposes a 3741-mer fragment was used (encoding SEQ ID NO: 180). When expressed as a His-tagged recombinant protein, enzymatic activity is seen *in vitro* when using a 4-nitrophenyl-N-acetyl-β-D-glucosaminide substrate, but not when using a 2-nitrophenyl-β-D-galactopyranoside substrate. The *in vivo* expression of spr0057 by pneumococci was monitored in nasal and lung washes, and was strongly upregulated.

The spr0096 antigen is annotated as a hypothetical protein that contains a LysM motif. The spr0096 sequence is 99% conserved between 22 different pneumococcal strains, and may thus offer broad protection. The wild-type spr0096 gene is 504 nucleotides long, but for immunisation purposes a 423-mer fragment (encoding SEQ ID NO: 229) was used.

The spr0286 antigen is annotated as hyaluronidase (Hyl), which degrades hyaluronic acid, a component of the extracellular matrix. The protein is surface-located, being LPxTG-anchored. The spr0286 sequence is 98.8% conserved between 22 different pneumococcal strains, and may thus offer broad protection. Although the hyaluronidase is a virulence factor, reference 316 reported that hyaluronidase neither influenced the clinical course of disease nor had any effect on systemic bacterial spread in the authors' disease model. The wild-type spr0286 gene is 3201 nucleotides long, but for immunisation purposes a 1884-mer (encoding SEQ ID NO: 182) or 1356-mer fragment (encoding SEQ ID NO: 183) was used.

The spr0565 antigen is annotated as β-galactosidase (BgaA), which cleaves galactose on human glycoproteins [314]. Thus the enzyme might facilitate pathogenesis/colonisation of humans, and blocking it might be useful for vaccination purposes. Moreover, the protein is surface-located, being LPxTG-anchored, and immunoreactive sera have been seen in convalescent pneumonia and meningitis patients. The spr0565 sequence is 97.9% conserved between 22 different pneumococcal strains, and may thus offer broad protection. There are no published studies on the protective efficacy of spr0565 as an immunogen. The wild-type spr0565 gene is 6687 nucleotides long, but for immunisation purposes a 6444-mer fragment was used (encoding SEQ ID NO: 184). When expressed as a His-tagged recombinant protein, enzymatic activity is seen *in vitro* when using 2-nitrophenyl-β-D-galactopyranoside substrate, but not when using a 4-nitrophenyl-N-acetyl-β-D-glucosaminide substrate. The *in vivo* expression of spr0565 by pneumococci was monitored in nasal and lung washes, and was strongly upregulated.

The spr0867 antigen is annotated as endo-β-N-acetylglucosaminidase (LytB), which mediates cell separation during bacterial replication. The protein is surface-located, being a choline-binding protein [317], and immunoreactive sera have been seen in convalescent patients. The spr0867 sequence is 98.8% conserved between 22 different pneumococcal strains, and may thus offer broad protection. It has been reported as a protective immunogen when used alone [318]. Allelic variations are discussed in reference 319. The wild-type spr0867 gene is 2109 nucleotides long, but for immunisation purposes a 2040-mer fragment was used (encoding SEQ ID NO: 185).

The spr1098 antigen is annotated as sortase A (srtA), which anchors LPXTG motif proteins to the pneumococcal surface. The protein is membrane-located. The spr1098 sequence is 100% conserved between 22 different pneumococcal strains, and may thus offer broad protection. Further details on the polypeptide are given in references 320 & 321. There are no published studies on the protective efficacy of spr1098 as an immunogen. The wild-type spr1098 gene is 744 nucleotides long, but for immunisation purposes a 654-mer fragment was used (encoding SEQ ID NO: 187).

The spr1345 antigen is annotated as cell wall surface anchor family protein, an adhesin that binds to mucins. The protein is surface-located, being LPxTG-anchored. The spr1345 sequence is 100% conserved between 22 different pneumococcal strains, although different strains have different numbers of repeat sequences, and may thus offer broad protection. Further details on the polypeptide are given in reference 322. There are no published studies on the protective efficacy of spr1345 as an immunogen. The wild-type spr1345 gene is 609 nucleotides long, but for immunisation purposes a 495-mer fragment was used (encoding SEQ ID NO: 188).

The spr1416 antigen is annotated as a hypothetical protein, and seems to be cytoplasmic. It has an unknown function, but the spr1416 sequence is 100% conserved between 22 different pneumococcal strains, and may thus offer broad protection. There are no published studies on spr1416. The wild-type spr1416 gene is 387 nucleotides long, but for immunisation purposes a 381-mer fragment was used.

The spr1418 antigen is annotated as a conserved hypothetical protein. The protein is surface-located, based on the presence of a leader peptide. It has an unknown function, buy immunoreactive sera have been seen in convalescent pneumonia and meningitis patients. The spr1418 sequence is 99.8% conserved between 22 different pneumococcal strains, and may thus offer broad protection. There are no published studies on spr1418. The wild-type spr0481 gene is 780 nucleotides long, but for immunisation purposes a 705-mer fragment was used.

The spr1431 antigen is annotated as lysozyme (LytC), which mediates autolysis. The protein is surface-located, being LPxTG-anchored, and immunoreactive sera have been seen in convalescent pneumonia and meningitis patients. The spr1431 sequence is 98% conserved between 22 different pneumococcal strains, and may thus offer broad protection. Further details on the polypeptide are given in references 323 & 324. Some protection data with LytC are reported in reference 318. The wild-type spr1431 gene is 1506 nucleotides long, but for immunisation purposes a 1407-mer fragment was used (encoding SEQ ID NO: 189).

The spr1739 antigen is pneumolysin, a secreted cytoplasmic pore-forming toxin. Immunoreactive sera have been seen in convalescent pneumonia and meningitis patients. The spr1739 sequence is 100% conserved between 22 different pneumococcal strains, and may thus offer broad protection. Pneumolysin has previously been used in combination with CbpA (PspC) [325] or other antigens [2] for immunisation.

The spr2021 antigen is annotated as a secreted 45kDa protein (PcsB). It is an essential hydrolase involved in the separation of dividing cells, and was initially identified because immunoreactive were seen in convalescent pneumonia and meningitis patients. The spr2021 sequence is 100% conserved between 22 different pneumococcal strains, and may thus offer broad protection. It has been confirmed as a leading vaccine candidate in reference 78. Further details on the PcsB polypeptide are given in references 326 & 327. The wild-type spr2021 gene is 1179 nucleotides long, but for immunisation purposes a 1098-mer fragment was used (encoding SEQ ID NO: 190).

One criterion for selecting these genes is their high level of conservation across a panel of 22 pneumococcal strain that includes representative strains from different pathogenic serogroups. There are several pneumococcal genes that are present in all of the current published genome sequences, with a high level of sequence identity, but that are absent in at least one strain in the panel. Thus current genomes can give a misleading impression that a particular gene is conserved. For example, a dicarboxylate/amino acid:cation (Na+ or H+) symporter (DAACS) family protein is present in the genomes of strains SP3-BS71, SP6-BS73, SP9-BS68, SP11-BS70, SP14-BS69, SP18-BS74, SP19-BS75, SP23-BS72, CGSP14, D39, R6 and TIGR4, but it is not seen in strains JJA and P1031.

Both spr0057 and spr0565 are surface-exposed exoglycosidases that deglycosylate host proteins. Both antigens are enzymatically active *in vitro* when expressed in His-tagged form, as shown using a substrate such as 4-nitrophenyl-N-acetyl-β-D-glucosaminide.

The spr0096, spr0867, spr1431 and spr2021 antigens may all be peptidoglycan hydrolases.

### Efficacy testing

Various model systems of pneumococcal disease were used for testing efficacy of the immunogens. In a mouse model of intraperitoneal infection, antigens were administered intraperitoneally and the challenge was intraperitoneal. Six-week-old, specific-pathogen-free female BALB/c mice were immunized intraperitoneally on days 0, 14, and 28. Immunizations were done using single recombinant proteins (20 µg/mouse) or with a combination of them (10 µg each/mouse), along with aluminium hydroxide or Freund's adjuvant. Controls received identical courses of saline plus adjuvant. Mice were then challenged intraperitoneally with a lethal dose of a homologous or heterologous strain (D39, TIGR4, SP-PD, PT131, Strep-5, 35Bsme15). The bacterial dosages used were generally: 5x10⁵ CFU/mouse of D39, about 10² CFU/mouse of TIGR4, or aout 5.4x10⁴ CFU/mouse of SP-PD. In some cases, similar experiments were carried out in CD1 mice, adjusting the challenging dose to achieve infection and mortality. Efficacy of immunisation is tested by evaluating the effect of vaccination on bacteremia (at 5 and/or 24 hours post infection) and mortality (monitored for 10 days following bacterial challenge or longer, depending on the infecting strain).

In a passive transfer model, sera against the recombinant proteins were administered intraperitoneally and challenge was intraperitoneal. Antisera were developed in mice immunized intraperitoneally as described above. 10-week-old BALB/c mice received 50 µl of immune serum intraperitoneally 15 min before challenge. Controls received identical courses of saline plus adjuvant. Mice were then challenged intraperitoneally.

In a model of intravenous infection, antigens were administered intraperitoneally and the challenge was intravenous. Five-week-old CD-1 mice were immunized intraperitoneally on days 0, 14, and 28. Immunizations were done using recombinant proteins individually (20 µg/mouse) or with a combination of them (10 µg each/mouse), along with Freund's adjuvant. Controls received identical courses of saline plus adjuvant. Mice were then challenged intravenously with a lethal dose of a homologous or heterologous strain (D39 or TIGR-4). The bacterial dosages used were: about 10⁵ CFU/mouse of D39, 2x10⁶ CFU/mouse of TIGR-4, about 10⁷ CFU/mouse of PT131, about 10⁴ CFU/mouse of Strep-5, about 2x10⁷ CFU/mouse of 35Bsme15. Efficacy of vaccine candidates is tested by evaluating the effect of vaccination on bacteremia (at 48 hours post-infection) and mortality (monitored for 10 days following bacterial challenge or longer, depending on the infecting strain)).

In a model of intranasal infection, antigens were administered intranasally, and the challenge was intranasal. Six-week-old, C57BL/6 mice were immunized intranasally on days 0, 16 and 32. Immunizations were done using recombinant proteins (20 µg/mouse) along with LTK63 adjuvant. Controls received identical courses of LTK63 or PBS. Mice were challenged with 10⁶ CFU TIGR4. Efficacy of vaccine candidates is tested by evaluating the effect of vaccination on mortality (monitored for 2-3 weeks following bacterial challenge) and on nasopharyngeal colonization and lung infection. Other experiments were carried out with different mouse and bacterial strains and administering the antigens either mucosally or systemically. ***Immunogenicity and protection studies with individual antigens***

The following antigens were individually tested in a mouse model: spr0057; spr0286; spr0565; spr0867; spr1098; spr1345; spr1416; spr1418; spr1431; and spr2021. The spr2086 antigen was split into two domains and tested as spr2086A and spr2086B. The antigens were adjuvanted with Freund's adjuvant and tested in CD1 and/or BALB/c mice. Challenge was with either the D39 or TIGR4 strain of pneumococcus, by either an intravenous (i.v.) or intraperitoneal (i.p.) route. Each group included at least 8 mice. A control group received adjuvant in phosphate-buffered saline. The TIGR4 strain is very virulent, and so protection against challenge by this strain indicates a high level of efficacy.

In terms of bacteremia, results were determined by measuring CFU/ml in the blood of immunised and control animals, 24 hours after challenge. In addition, the numbers of infected animals in both groups were compared using the one-tailed Mann-Whitney U-test. Results were as follows:

| **Ag** | **Mice** | **Strain** | **Challenge Route** | **CFU/ml (geometric mean)** | | | **Number infected** | | |
|---|---|---|---|---|---|---|---|---|---|
| | | | | **Test** | **Control** | **Ratio** | **Test** | **Control** | **P** |
| 0057 | CD1 | D39 | i.v. | 6.15E+03 | 9.22E+04 | 15.0 | 11/9 | 5/15 | 0.064 |
| 0057 | BALB/c | D39 | i.p. | 1.77E+05 | 5.76E+06 | 32.5 | 2/8 | 0/9 | 0.056 |
| 0286A | CD1 | D39 | i.v. | 1.25E+02 | 1.70E+04 | 135.2 | 19/1 | 10/10 | 0.005 |
| 0286B | BALB/c | D39 | i.p. | 6.26E+02 | 5.38E+04 | 85.9 | 3/5 | 1/6 | 0.027 |
| 0565 | CD1 | D39 | i.v. | 2.44E+02 | 8.00E+04 | 327.6 | 15/5 | 5/15 | 0.0002 |
| 0867 | CD1 | D39 | i.v. | 2.97E+03 | 9.16E+04 | 30.9 | 6/4 | 3/7 | 0.095 |
| 1098 | CD1 | D39 | i.v. | 2.49E+03 | 3.47E+05 | 139.6 | 6/4 | 3/7 | 0.062 |
| 1098 | BALB/c | D39 | i.p. | 9.91 E+03 | 4.18E+06 | 421.3 | 3/4 | 0/8 | 0.007 |
| 1098 | BALB/c | TIGR4 | i.p. | 1.06E+03 | 1.91E+05 | 180.9 | 4/4 | 0/8 | 0.007 |
| 1345 | CD1 | D39 | i.v. | 4.24E+02 | 1.77E+05 | 417.7 | 7/3 | 4/6 | 0.038 |
| 1345 | BALB/c | D39 | i.p. | 5.87E+03 | 7.99E+05 | 136.1 | 1/7 | 0/16 | 0.011 |
| 1416 | BALB/c | TIGR4 | i.p. | 5.56E+04 | 1.66E+06 | 29.8 | 0/8 | 1/15 | 0.077 |
| 1418 | BALB/c | D39 | i.p. | 6.53E+03 | 7.99E+05 | 122.4 | 2/6 | 0/16 | 0.004 |
| 1431 | CD1 | D39 | i.v. | 4.68E+02 | 2.74E+05 | 585.5 | 13/7 | 6/14 | 0.002 |
| 2021 | CD1 | D39 | i.v. | 1.63E+04 | 6.05E+04 | 3.7 | 10/10 | 6/14 | > 0.1 |
| 2021 | BALB/c | TIGR4 | i.p. | 1.69E+05 | 1.19E+06 | 7.1 | 0/8 | 1/15 | 0.040 |

In terms of survival, results were determined by measuring the median survival (in days) per group. In addition, the numbers of surviving animals in both groups were compared using a one-tailed Mann-Whitney U-test. Results are in the following table. In addition, the number of mice surviving was followed over time, and an example of such data is shown in Figure 1.

| **Ag** | **Mice** | **Strain** | **Route** | **Survival (days)** | | **Alive/dead** | | |
|---|---|---|---|---|---|---|---|---|
| | | | | **Test** | **Control** | **Test** | **Control** | **P** |
| 0057 | CD1 | D39 | i.v. | >10.5 | 5.5 | 11/9 | 5/15 | 0.064 |
| 0057 | BALB/c | D39 | i.p. | 4 | 2.5 | 3/7 | 0/9 | 0.067 |
| 0286A | CD1 | D39 | i.v. | >10.5 | 7.5 | 17/3 | 9/11 | 0.006 |
| 0286B | BALB/c | D39 | i.p. | 8 | 5.5 | 4/4 | 2/5 | >0.1 |
| 0565 | CD1 | D39 | i.v. | >10.5 | 5 | 14/6 | 6/14 | 0.002 |
| 0867 | CD1 | D39 | i.v. | >11.5 | 6.5 | 6/4 | 3/7 | >0.1 |
| 1098 | CD1 | D39 | i.v. | >10.5 | 6 | 6/4 | 3/7 | >0.1 |
| 1098 | BALB/c | D39 | i.p. | 5.5 | 4.5 | 3/4 | 1/7 | >0.1 |
| 1098 | BALB/c | TIGR4 | i.p. | 6.5 | 2.5 | 3/5 | 1/7 | 0.032 |
| 1345 | CD1 | D39 | i.v. | >10.5 | 4.5 | 8/2 | 3/7 | 0.018 |
| 1345 | BALB/c | D39 | i.p. | 4.5 | 4 | 3/5 | 3/13 | >0.1 |
| 1416 | BALB/c | TIGR4 | i.p. | 3 | 1.5 | 2/6 | 1/15 | 0.086 |
| 1418 | BALB/c | D39 | i.p. | 5.5 | 4 | 3/5 | 3/13 | >0.1 |
| 1431 | CD1 | D39 | i.v. | >10.5 | 4.5 | 15/5 | 6/14 | 0.002 |
| 2021 | CD1 | D39 | i.v. | >10.5 | 4.5 | 11/9 | 4/17 | 0.061 |
| 2021 | BALB/c | TIGR4 | i.p. | 2 | 1.5 | 2/6 | 0/16 | >0.1 |

For all tested antigens, therefore, there was a substantial reduction in bacteremia and/or an increase in survival.

In summary, four preferred antigens are effective against serotype 2, 3, 4 and 35B strains as follows:

| | **OPKA** | **Type 2** | **Type 3** | **Type 4** | **Type 35** |
|---|---|---|---|---|---|
| spr0057 | +/- | ++ | - | + | - |
| spr0096 | + | + | ++ | + | ++ |
| spr0565 | + | ++ | ++ | ++ | - |
| spr2021 | ++ | +/- | - | +/- | ? |

Although serotype 4 is covered by current conjugate vaccines, serotypes 2, 3 and 35B are not.

### Immunogenicity and protection studies with combined antigens

The following combinations of antigens were tested in the same mouse model as individual antigens: (1) spr0057+spr0096+spr2021; (2) spr0057+spr0096+spr2021; (3) spr0057+spr0096+spr2021; (4) spr0057+spr2021; (5) spr0057+spr0565+spr2021. In addition, a control group (0) was immunised with a combination of PspC and a detoxified pneumolysin ('Ply-detox') [325], and a group (6) received a combination of spr0565+spr2021+Ply-detox. Results were as follows:

| | **CFU/ml (geometric mean)** | | | **Number infected** | | | **Survival** | | **Alive/dead** | | |
|---|---|---|---|---|---|---|---|---|---|---|---|
| | **Test** | **Control** | **Ratio** | **Test** | **Ctrl** | **P** | **Test** | **Ctrl** | **Test** | **Ctrl** | **P** |
| 0 | 1.70E+05 | 9.60E+06 | 56.5 | 4/26 | 0/30 | 0.009 | 4.5 | 1 | 12/18 | 2/28 | <0.001 |
| 1 | 4.21E+03 | 1.19E+06 | 283.4 | 0/8 | 1/15 | 0.001 | >10.5 | 4 | 6/2 | 0/16 | <0.001 |
| 2 | 3.87E+04 | 1.40E+06 | 36.2 | 2/6 | 1/7 | 0.065 | 5 | 1.5 | 3/5 | 2/6 | >0.1 |
| 3 | 1.18E+03 | 4.21E+04 | 35.5 | 7/3 | 3/7 | 0.062 | >10.5 | 7 | 7/3 | 3/7 | >0.1 |
| 4 | 1.07E+04 | 3.44E+05 | 32.0 | 1/7 | 1/15 | 0.014 | 5.5 | 1.5 | 2/6 | 3/13 | 0.019 |
| 5 | 3.48E+02 | 4.21E+04 | 120.8 | 7/3 | 3/7 | 0.026 | >10.5 | 7 | 8/2 | 3/7 | 0.018 |
| 6 | 1.62E+03 | 3.16E+05 | 194.6 | 7/3 | 1/9 | 0.014 | >10.5 | 3.5 | 7/3 | 1/9 | 0.014 |

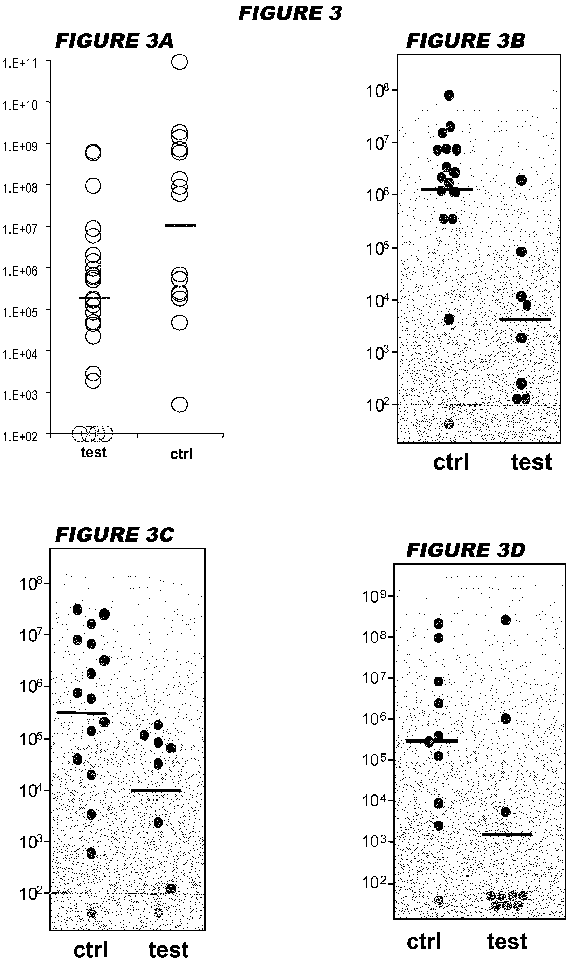

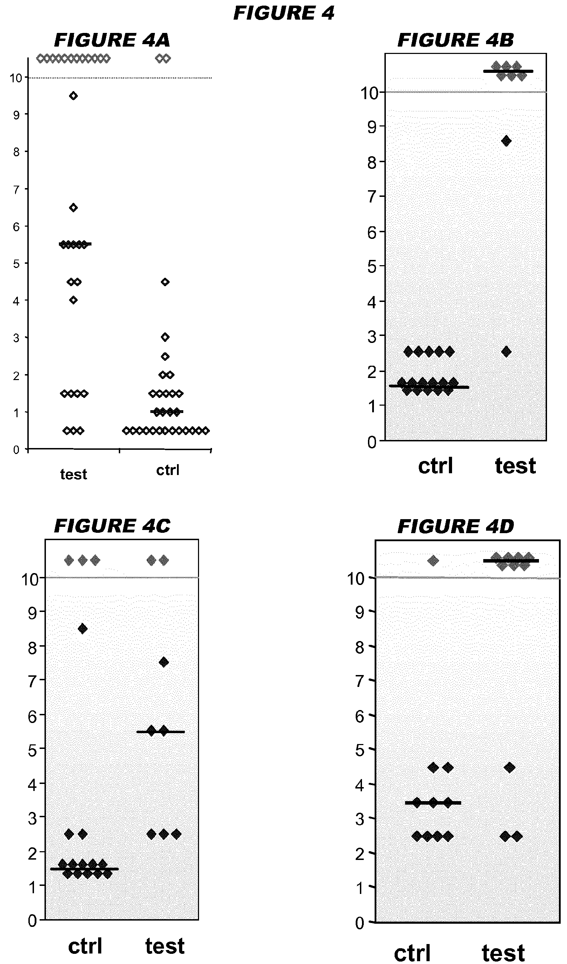

Bacteremia data for groups 0, 1, 4 and 6 are shown in Figure 3. Survival data for the same groups are shown in Figure 4. Survival data for groups 1 and 5 are shown in Figure 2.

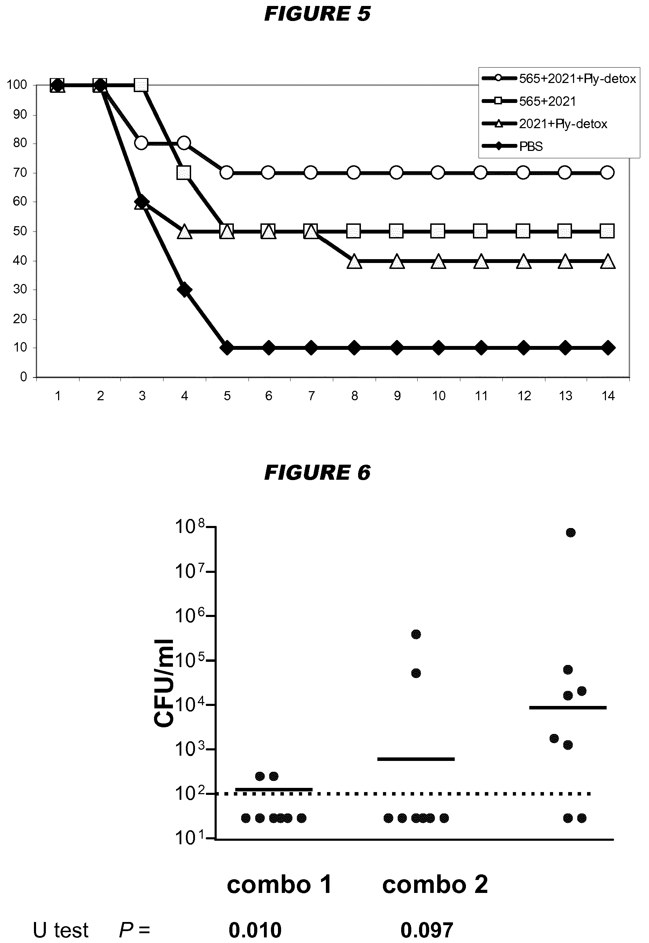

In further experiments, mice received (7) spr0565+spr2021 or (8) spr2021+Ply-detox. Survival data for groups 6, 7 and 8 are in Figure 5.

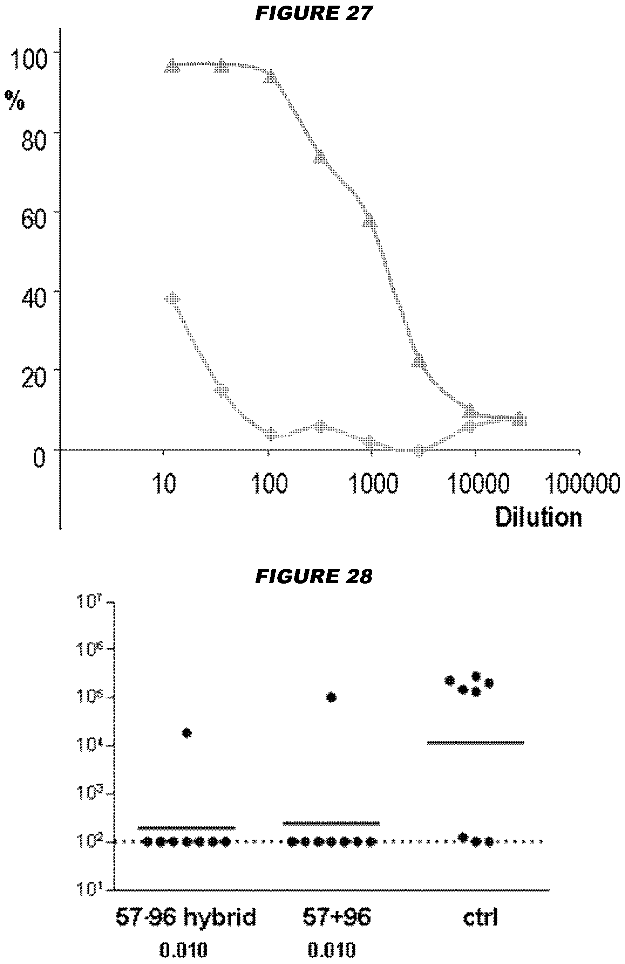

In further experiments, mice received a combination of spr0565, PmP and spr2021. Sera were tested in the opsonophagocytic killing assay (OPKA). Results are in Figure 27. A 10,000-fold dilution was required before the immune serum's activity declined to control levels.

### Combo-1 to Combo-4

Four different antigen combinations were prepared:
Combo-1: spr0057 + spr0096 + spr2021.
Combo-2: spr0057 + spr0565 + spr2021.
Combo-3: spr0057 + spr0096 + spr0565.
Combo-4: spr0057 + spr0096 + spr0565 + spr2021.

Rabbit were immunised and their immune sera were transferred into BALB/c mice to test for passive protection against the TIGR4 strain.

Mice were also immunised and then tested in challenge models using strains including TIGR4 (i.p. challenge) and D39, PT131 & TREP6A (i.v. challenge). The TIGR4 strain is serotype 4, which is covered by Prevnar™ ('VT' strains), but the other 3 strains are serotypes 2, 3 and 6A, which are not covered by Prevnar™ ('NVT' strains). Serotypes 3, 4 and 6A are covered by proposed 13-valent saccharide vaccines [328], but serotype 2 strains are not.

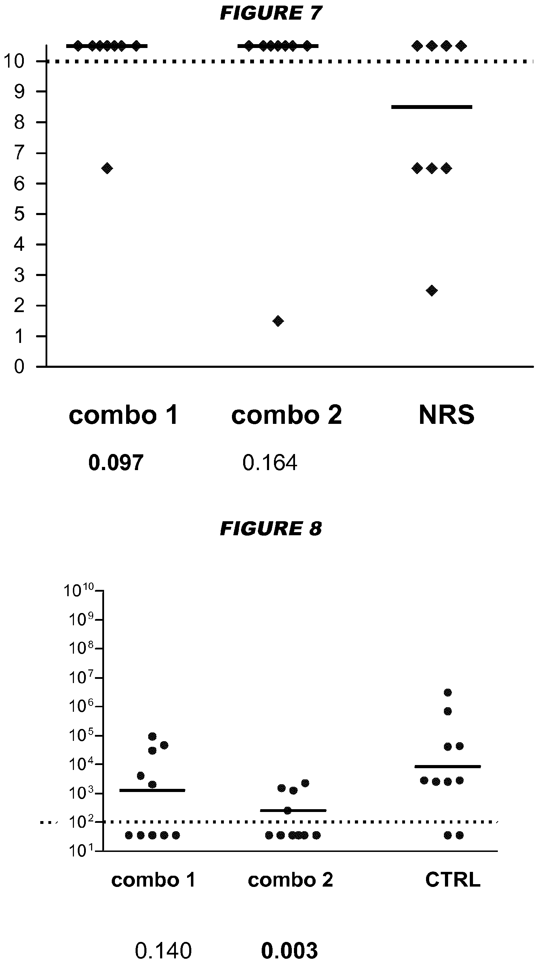

Figures 6 and 7 show the results obtained in the passive transfer test. Combo-1 and combo-2 gave at least a log reduction in bacteremia (Fig. 6) and resulted in a survival time of at least 10 days (Fig. 7). Efficacy was not improved by increasing dosing to 50µg of each antigen.

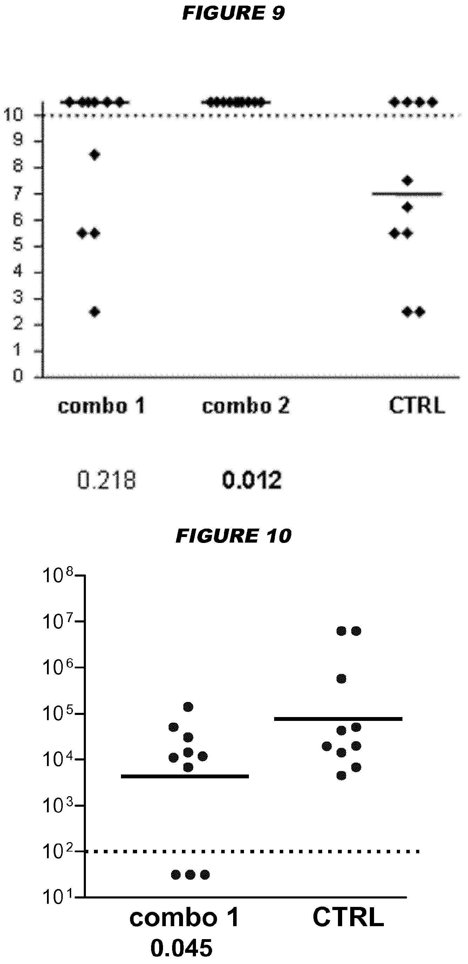

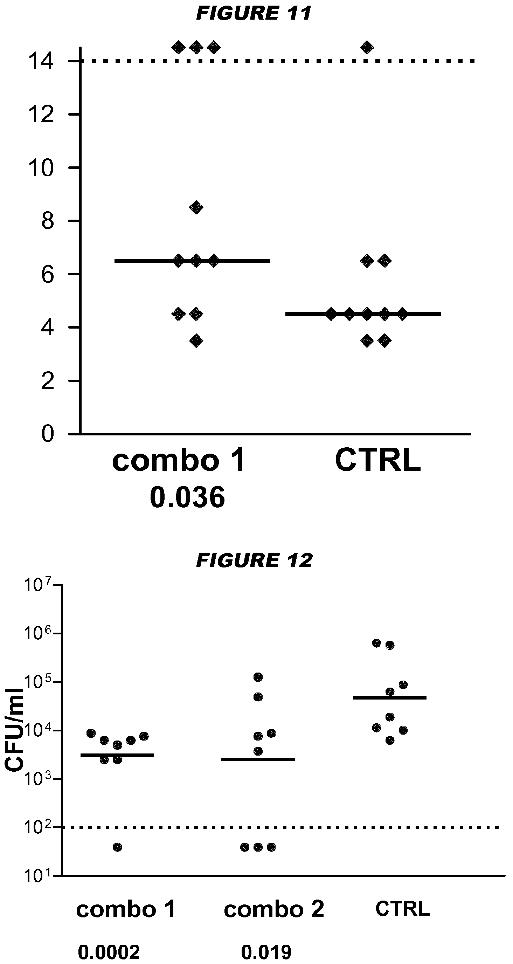

Figures 8 & 9 show results obtained in the challenge model using strain PT131. Combo-1 and combo-2 were again effective, with combo-2 being superior. Figures 10 & 11 show results obtained in the challenge model using 1.1x10⁴ CFU of strain STREP-5 after immunisation with combo-1.

Figure 12 shows the reduction in CFU/ml 24 hours after challenge with OREP-3. Combo-1 and combo-2 are both effective.

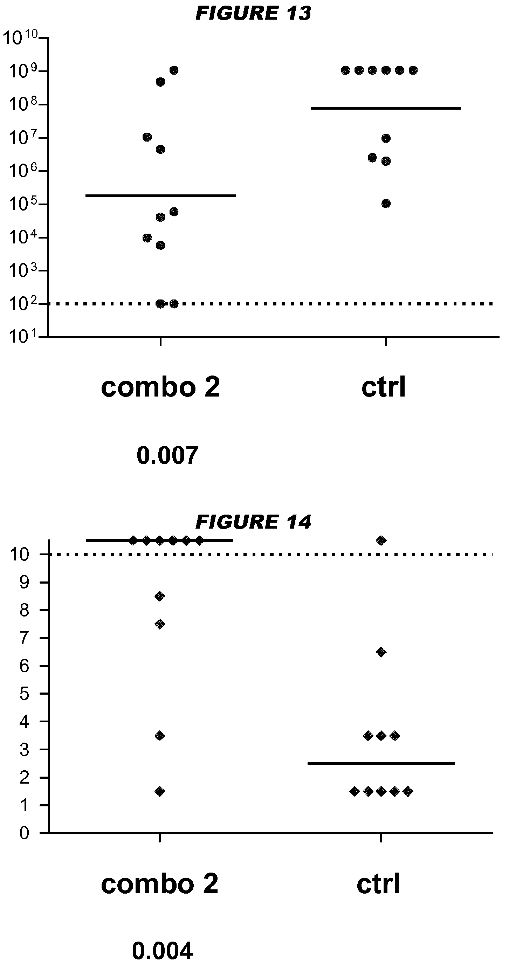

Figures 13 & 14 shows results obtained after challenge of combo-2-immunised mice with TREP-6A.

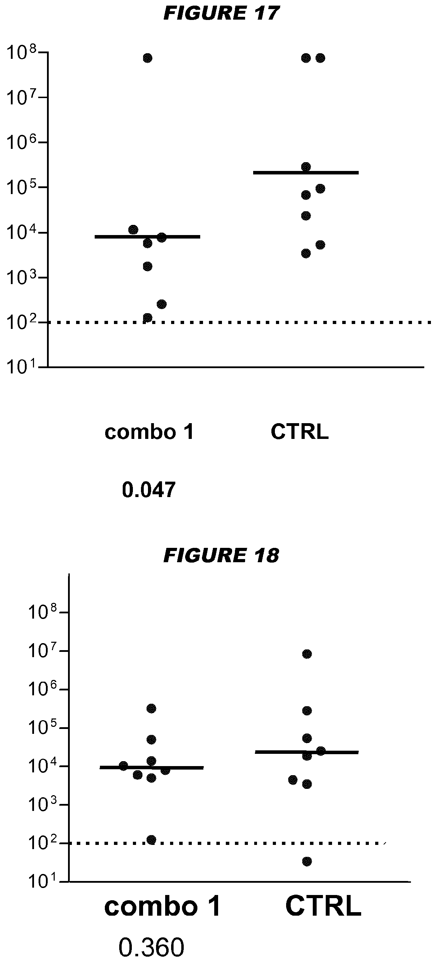

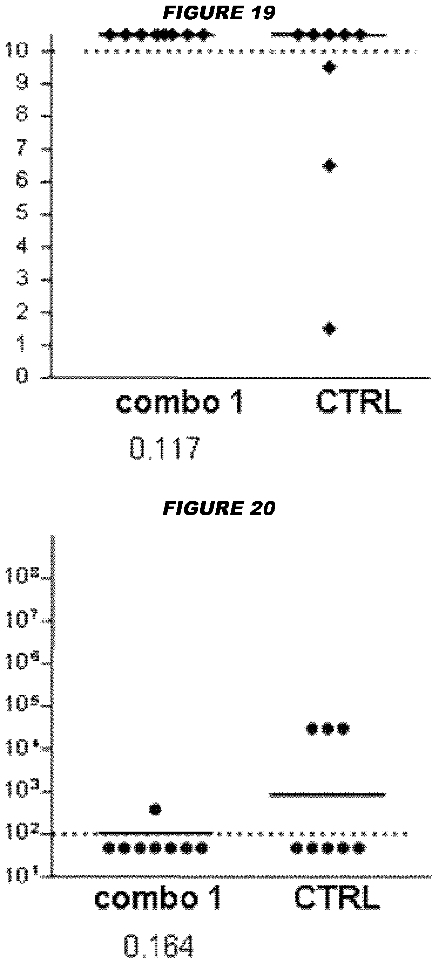

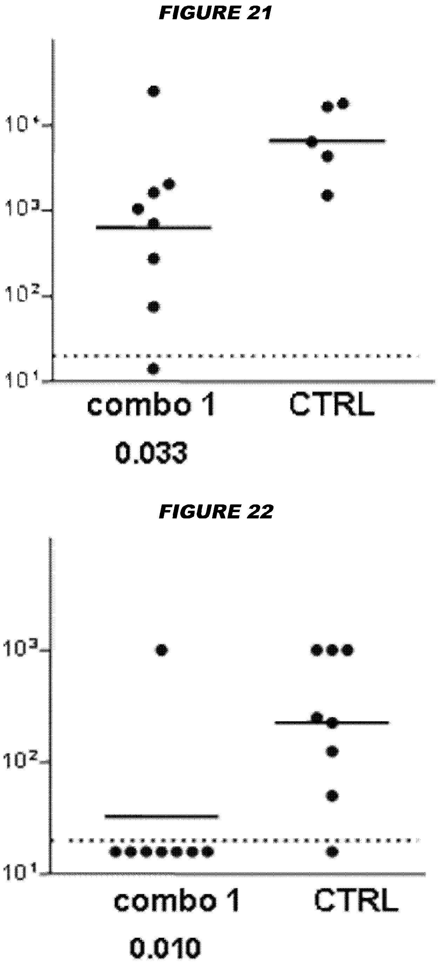

As shown in Figure 17, combo-1 decreases bacteremia after a high-dose (1.1x10⁷ cfu) intranasal challenge with TIGR4, although nasal colonisation and lung infection were not affected. Using a sublethal intranasal challenge at late stage of infection, figures 18-22 show that combo-1 is effective.

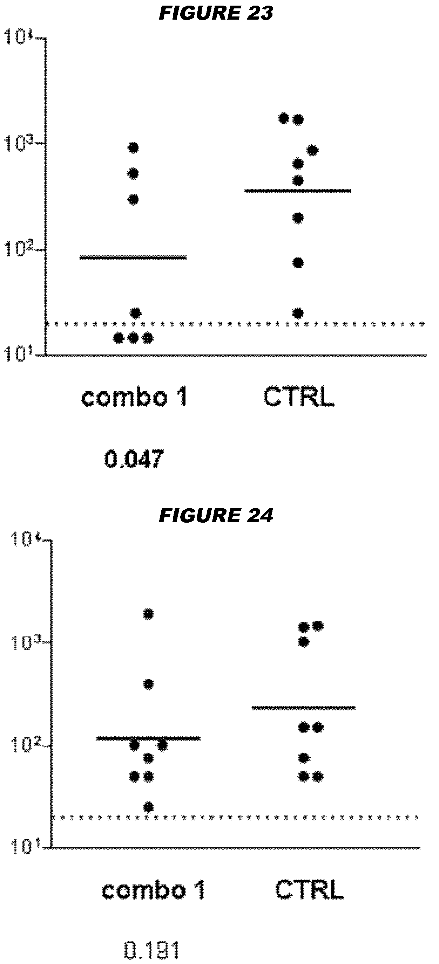

Efficacy against intranasal challenge with 14-Spain-15 strain is shown in Figures 23 (nasal wash, 48 hours) & 24 (lung wash, 48 hours).

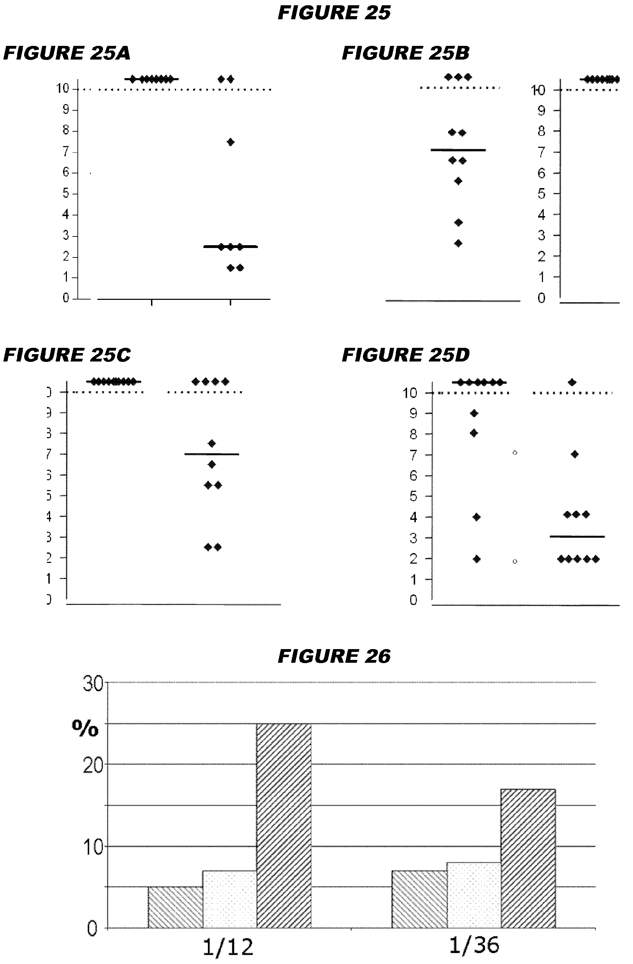

Figure 25 summarises mortality after challenge with TIGR-4 (i.p. challenge; Fig. 25A) or with D39, PT131 or TREP6A (i.v. challenge; Fig. 25B) following immunisation with combo-2.

Thus immunisation with combo-1 or combo-2 is effective against infection by a wide variety of pneumococcal strains.

Figure 26 shows OPKA results obtained in the using serum from control animals or from animals immunised with combo-1 or combo-2. Large increases in OPKA activity are seen using combo-2 at both a 1/12 and 1/36 dilution.

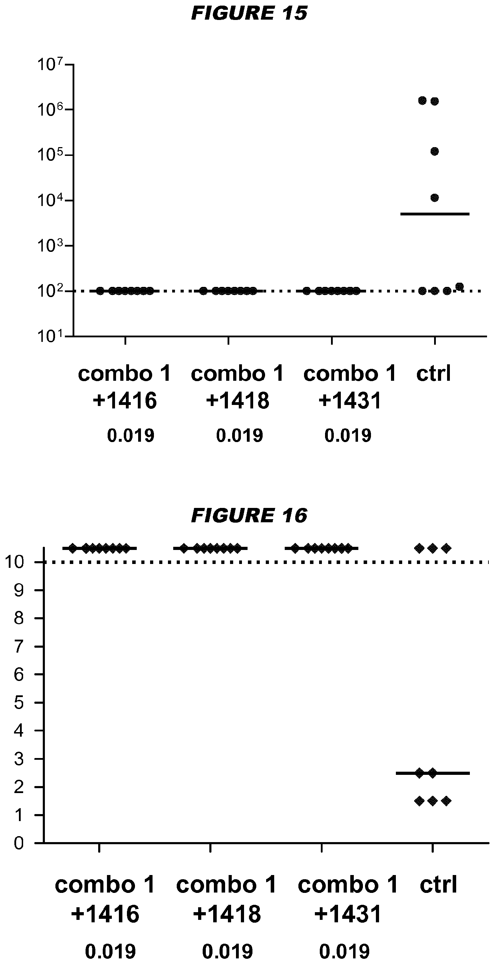

Combo-1 was modified by addition of spr1416, spr1418 or spr1431. As shown in Figures 15 & 16, combo-1 maintains its efficacy against TIGR4 challenge even when these three antigens are added.

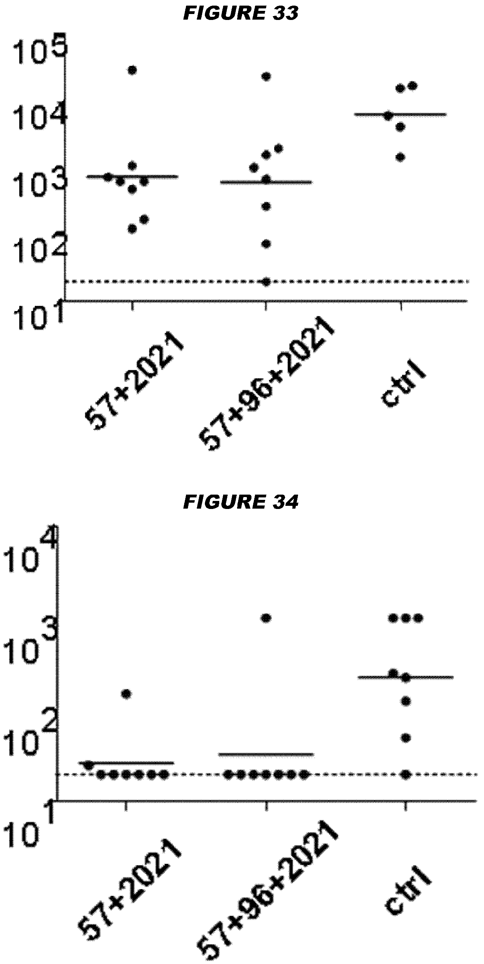

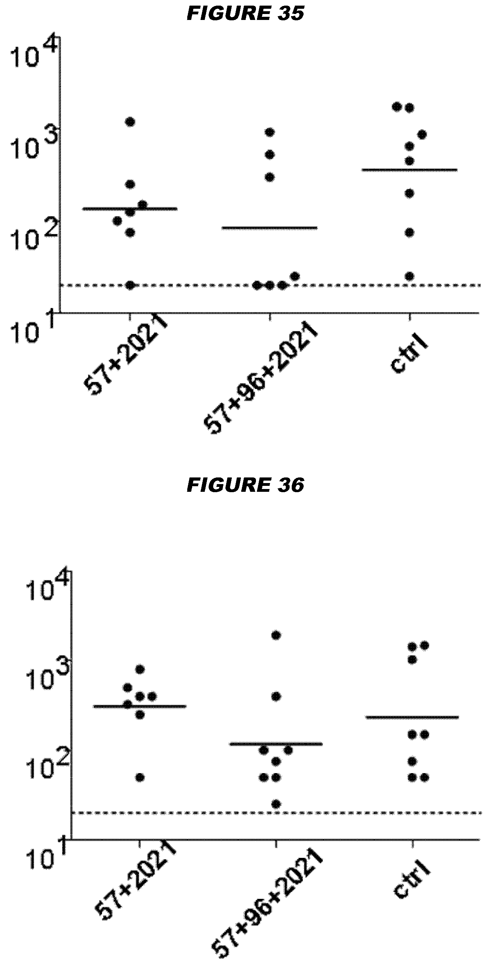

Combo-1, which includes three antigens, was also compared to a combination of two of those three antigens (spr0057+spr2021) using intranasal immunisation and intranasal challenge. Although the two combinations showed little difference in terms of mortality or bacteremia (after both 24 hours and 10 days) using TIGR4 as a challenge strain, nasal and lung washes after 10 days showed significant reductions (Figures 33 and 34). Good results were also seen when using 14-Spain15 as the challenge strain (Figures 35 and 36).

Combo-1 was also compared to spr0565 alone. Although spr0565 reduced bacteremia after 24 hours, after 48 hours the effect was not seen. In contrast, the efficacy of combo-1 became apparent only after 48 hours. No efficacy was seen against nasal colonization or lung infection. In summary, the best representative results with combo-1 were as follows:

| **Challenge** | | | **Bacteremia** | | | **Survival** | | |
|---|---|---|---|---|---|---|---|---|
| | | | **Geom. mean CFU** | | **U**-**test** | **median days** | | **U**-**test** |
| **Serotype**-**strain** | **route** | **dose** | **Combo** | **Control** | ***P*** | **Combo** | **Control** | ***P*** |
| 02-D39 | ip | 1.1E+03 | 1.9E+04 | 1.1E+05 | 0.253 | 6.25 | 1.5 | 0.097 |
| 02-D39 | iv | 2.1E+05 | 3.4E+04 | 4.5E+06 | 0.038 | 9 | 2.5 | 0.022 |
| 03-0REP3 | ip | 4.5E+03 | 1.7E+03 | 2.5E+04 | <0.001 | 2.5 | 2.5 | 0.117 |
| 04-TIGR4 | in | 4.5E+06 | 1.2E+02 | 8.5E+02 | 0.164 | - | - | - |
| 04-TIGR4 | in | 1.1E+07 | 7.9E+03 | 2.1E+05 | 0.047 | - | - | - |
| 04-TIGR4 | ip | 1.1E+02 | 4.2E+03 | 1.2E+06 | 0.001 | 10.5 | 1.5 | <0.001 |
| 04-TIGR4 | ip | 1.5E+02 | 1.6E+04 | 3.4E+05 | 0.053 | 5.5 | 1.5 | 0.096 |
| 04-TIGR5 | ip | 7.0E+01 | 1.5E+02 | 2.6E+05 | <0.001 | 10.5 | 1.5 | 0.025 |
| 04-TIGR4 | iv | 3.2E+06 | 1.1E+04 | 4.1E+06 | 0.007 | 11.5 | 5 | 0.018 |
| 05-STREP5 | iv | 1.1E+04 | 4.4E+03 | 7.7E+04 | 0.045 | 6.5 | 4.5 | 0.036 |
| 06B-Finland12 | ip | 5.0E+04 | 6.6E+03 | 5.2E+06 | 0.001 | 5.5 | 1.5 | 0.164 |
| 19F-5167 | iv | 7.0E+07 | 7.8E+03 | 1.5E+05 | 0.032 | 7.5 | 3.5 | 0.062 |
| 35B-SME15 | iv | 4.5E+07 | 1.3E+04 | 1.8E+05 | 0.083 | 9 | 5.5 | 0.095 |

In summary, the best representative results with combo-2 were as follows:

| **Challenge** | | | **Bacteremia** | | | **Survival** | | |
|---|---|---|---|---|---|---|---|---|
| | | | **Geom. mean CFU** | | **U**-**test** | **median days** | | **U**-**test** |
| **Serotype**-**strain** | **route** | **Dose** | **Combo** | **Control** | ***P*** | **Combo** | **Control** | ***P*** |
| 02-D39 | iv | 1.6E+05 | 3.5E+02 | 4.2E+04 | 0.026 | 10.5 | 7 | 0.018 |
| 03-0REP3 | ip | 4.5E+03 | 2.0E+03 | 2.5E+04 | 0.019 | 3.5 | 2.5 | 0.005 |
| 03-PT131 | iv | 9.0E+06 | 2.5E+02 | 8.3E+03 | 0.003 | 10.5 | 7 | 0.012 |
| 04-TIGR4 | ip | 1.5E+02 | 6.3E+02 | 6.5E+04 | 0.007 | 10.5 | 2.5 | 0.005 |
| 04-TIGR5 | ip | 7.0E+01 | 1.1E+03 | 3.9E+04 | 0.032 | 10.5 | 9 | 0.139 |
| 04-TIGR4 | iv | 2.8E+06 | 2.3E+03 | 1.8E+04 | 0.176 | 9.5 | 5.5 | 0.158 |
| 05-STREP5 | iv | 9.0E+03 | 1.7E+04 | 1.1E+05 | 0.083 | 6.5 | 5.5 | 0.072 |
| 06A-TREP-6A | iv | 3.5E+07 | 1.8E+05 | 7.7E+07 | 0.007 | 10.5 | 2.5 | 0.004 |
| 06B-Finland12 | ip | 5.0E+04 | 5.7E+05 | 5.2E+06 | 0.014 | 1.5 | 1.5 | 0.191 |
| 35B-SME15 | iv | 6.8E+07 | 2.7E+04 | 1.1E+06 | 0.045 | 6.5 | 5 | 0.095 |

In summary, the best representative results with combo-4 were as follows:

| **Challenge** | | | **Bacteremia** | | | **Survival** | | |
|---|---|---|---|---|---|---|---|---|
| | | | **Geom. mean CFU** | | **U**-**test** | **median days** | | **U**-**test** |
| **Serotype**-**strain** | **route** | **dose** | **Combo** | **Control** | ***P*** | **Combo** | **Control** | ***P*** |
| 02-D39 | iv | 2.3E+05 | 1.9E+04 | 3.3E+06 | 0.032 | 8.5 | 3.5 | 0.053 |
| 03-0REP3 | ip | 4.5E+03 | 9.3E+02 | 2.5E+04 | 0.032 | 3.5 | 2.5 | 0.005 |
| 03-PT131 | iv | 9.0E+06 | 5.8E+02 | 5.1E+03 | 0.045 | 10.5 | 6.5 | 0.176 |
| 04-TIGR4 | ip | 1.4E+02 | 2.6E+02 | 7.9E+03 | 0.041 | 10.5 | 7 | 0.052 |
| 04-TIGR4 | iv | 3.5E+06 | 4.8E+03 | 1.7E+05 | 0.072 | 10 | 6.5 | 0.095 |
| 05-STREP5 | iv | 8.5E+03 | 2.2E+03 | 3.7E+04 | 0.083 | 9 | 6.5 | 0.109 |

Thus the combinations provide good protection from bacteremia and increase survival.

### Intranasal immunisation of mice with pilus antigens

A mouse model was used to characterize the protective potential of pneumococcal pilus RrgA, RrgB and RrgC proteins. Intranasal immunization of recombinant pilus proteins combined with the non-toxic *E.coli* heat-labile enterotoxin mutant LTK63 as adjuvant evoked IgA and IgG-responses in serum. Furthermore, immunization with the major pilus component (RrgB) led to decreased bacterial load of the invasive TIGR4 strain in the nasopharynx, lungs and blood and yielded a 5-fold increase in survival. There was no effect on asymptomatic nasopharyngeal carriage of a non-invasive strain, pointing to restriction of the observed protective effect to a fulminant infection.

Six-week old female C57BL/6 mice were intranasally immunized 3 times (2-week interval) with 10µl PBS containing either 20µg of recombinant RrgA, RrgB or RrgC or a mix of 10µg of each. Proteins had an affinity tag and used the sequence from the TIGR4 strain. All protein solutions were administered together with 2µg of LTK63. Controls received 2µg of LTK63 in PBS, or only PBS. One week after the final immunization serum samples were taken in order to determine antibody titres by ELISA. Mice were challenged intranasally with 0.5-1x10⁷ cfu/mouse of invasive TIGR4 pneumococci or the colonizing serotype 19F strain. Mice were sacrificed after exceeding a defined clinical score as approved by the local committee for animal experiments. Seven to nine days post-infection the surviving mice were sacrificed. Blood samples, the lungs and a nasopharyngeal-tracheal flush sample were retrieved. Viable bacteria were quantified by serial plating of blood samples. The lungs were weighted, homogenized in PBS containing protease inhibitor cocktail and used for the quantification of viable bacteria. To determine the number of bacteria in the upper respiratory tract, the nasopharyngeal-trachea was lavaged post mortem with 30 µl of PBS by inserting a 20-gauge catheter into the proximal trachea and collecting the flush from the nostrils and performed serial plating. A camera was used to determine the colonization in a non-invasive manner by determining the luminescence intensity in the nasopharynx, lungs, ears and bloodstream of anesthetized mice. A threshold of 300 p/sec/cm²/sr in the nasal area was considered as being sufficient for indicating successful colonization.

Intranasal immunization with pilus proteins evoked a systemic IgA and IgG response. Serum titres of IgA showed that mice immunized with individual pilus proteins mounted an IgA response to the respective antigens. No response to the other two proteins was observed, showing that individual pilus proteins do not elicit cross-reactive antibodies. Mice immunized with all 3 proteins had serum IgA levels comparable to the single immunizations although antibody levels to RrgA were lower. Similar results were obtained for IgG serum levels. In short, an immune response, comprising both IgA and IgG, is mounted after intranasal immunization with any of the three pilus proteins but not with the controls.

After immunization mice were infected intranasally with a high dose of the invasive TIGR4 strain. Analysing the survival rates showed significant differences between the different groups. Only 10% of the PBS-immunized and 20% of the LTK63-immunized mice survived the challenge. Mice had bacterial counts of 1x10⁵-10⁹ bacteria per ml of blood and most of those mice succumbed to pneumococcal disease within 96 hours post-infection. Immunization with RrgB plus LTK63 increased survival from 10% (in the control group of PBS-immunized mice) to 55% (*p*=0.0001). Also survival compared to the group receiving only the adjuvant was significantly increased (*p*= 0.016). A vaccine mixture of RrgA, RrgB and RrgC showed the same protection as vaccination with RrgB alone (*p*=0.002, increased survival from 10% to 45% compared to PBS-immunized mice). Furthermore bacterial counts in the bloodstream, lungs and nasopharynx were significantly reduced in RrgB-immunized mice compared to the control groups. Compared to PBS-immunized mice, median bacterial numbers were reduced from 1.5x10⁶cfu/ml to 1.0x10⁰cfu/ml (*p*=0.067) in the bloodstream, from 5x10⁴cfu/mg tissue to 1.5x10¹cfu/mg tissue (*p*=0.001) in the lungs and from 7x10³ to 4.5x10² (*p*=0.0002) in the nasopharynx. Thus mice immunized with TIGR4-based RrgB proteins together with the mucosal adjuvant LTK63 showed a significant increase in survival after intranasal challenge with TIGR4 as compared to non-vaccinated control mice.

The immunization did not lead to enhanced clearance of colonizing pneumococci from the nasopharynx. Unlike TIGR4, a serotype 19F strain of sequence type 162 was previously shown to be non-invasive even after a high dose intranasal challenge. This hyper-colonizing strain expresses pili belonging to the same clade as TIGR4. Only 1 amino acid (A645V) differs in the RrgB protein between the two strains. RrgB-immunized mice were challenged with this 19F strain, also engineered to express luciferase. Control groups received LTK63 or PBS, only. Serum IgG and IgA against RrgB was determined by ELISA and values were similar to those seen in the previous experiments. Mice became colonized with intermittent occurrence of mostly subclinical otitis media or pneumonia. Colonization was determined 24-216 hours post infection. For the PBS-treated control group the percentage of colonized mice varied between 25% (48h p.i) and 70% (96h p.i.). The initial low colonization rate increased over time with a final clearance event taking effect after 96 hours. The same observations were made for the LTK63- and RrgB-immunized mice, but with a shift towards a ∼24-hour earlier clearance. No significant difference in colonization- and/or clearance-events could be observed between control and RrgB-immunized mice. Occurrence of otitis media and pneumonia was generally low, with a maximum of 10% occurrence in each group. No significant difference between the groups could be established. Finally bacterial counts in the nasopharynx were determined upon sacrifice of the animals 9 days post-infection. Mice of all three groups showed similar amounts of bacteria in the nasopharynx (2.8 - 4.4x10³ per wash). A peak of colonization was observed around 96 hours post-infection.

### Hybrid polypeptides

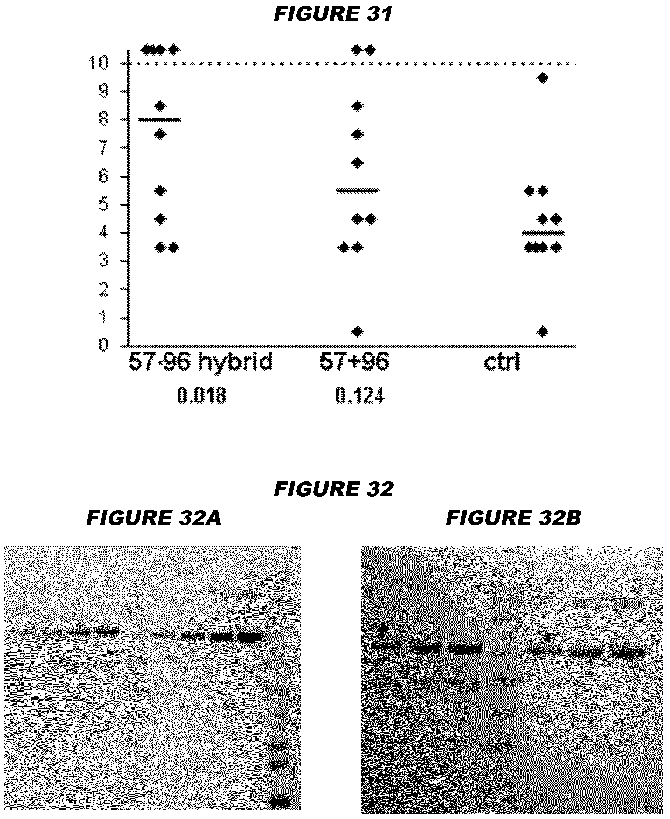

Hybrids have been made with pairs of spr0057, spr0096, spr0565 (optionally in the spr0565A or spr0565B form), spr2021 and RrgA. Various pairings have been constructed, expressed and purified. For example, Figure 32 includes two gels showing purified (>1mg/ml , ≥80% pure) spr2021-spr0096 and spr0096-spr2021, both with a molecular weight of ∼61kDa. Although both hybrids could be expressed and purified in soluble form, the spr2021-spr0096 hybrid was much more soluble than the spr0096-spr2021 hybrid.

The following hybrid sequences (having formula NH₂-A-{-X-L-}₂-B-COOH) have been prepared:

| **A** | **X₁** | **L₁** | **X₂** | **L₂** | **B** | **SEQ ID** |
|---|---|---|---|---|---|---|
| M | spr2021 | SEQ ID 233 | spr0057 | LE | His₆ | 193 |
| M | spr2021 | SEQ ID 233 | spr0096 | LE | His₆ | 194 |
| M | spr2021 | SEQ ID 233 | spr0565 | SEQ ID 235 | His₆ | 195 |
| M | spr2021 | SEQ ID 233 | spr0565A | SEQ ID 235 | His₆ | 196 |
| M | spr2021 | SEQ ID 233 | spr0565B | SEQ ID 235 | His₆ | 197 |
| MAS | spr2021 | SEQ ID 233 | RrgA | LE | His₆ | 198 |
| M | spr0057 | SEQ ID 233 | spr2021 | SEQ ID 235 | His₆ | 199 |
| M | spr0057 | SEQ ID 233 | spr0096 | LE | His₆ | 200 |
| M | spr0057 | SEQ ID 233 | RrgA | LE | His₆ | 201 |
| M | spr0057 | SEQ ID 233 | spr0565 | SEQ ID 235 | His₆ | 202 |
| M | spr0057 | SEQ ID 233 | spr0565A | SEQ ID 235 | His₆ | 203 |
| M | spr0057 | SEQ ID 233 | spr0565B | SEQ ID 235 | His₆ | 204 |
| M | spr0096 | SEQ ID 233 | spr2021 | SEQ ID 235 | His₆ | 205 |
| M | spr0096 | SEQ ID 233 | spr0057 | LE | His₆ | 206 |
| M | spr0096 | SEQ ID 233 | RrgA | LE | His₆ | 207 |
| M | spr0096 | SEQ ID 233 | spr0565 | SEQ ID 235 | His₆ | 208 |
| M | spr0096 | SEQ ID 233 | spr0565A | SEQ ID 235 | His₆ | 209 |
| M | spr0096 | SEQ ID 233 | spr0565B | SEQ ID 235 | His₆ | 210 |
| MAS | RrgA | SEQ ID 233 | spr2021 | SEQ ID 235 | His₆ | 211 |
| MAS | RrgA | SEQ ID 233 | spr0565 | SEQ ID 235 | His₆ | 212 |
| MAS | RrgA | SEQ ID 233 | spr0565A | SEQ ID 235 | His₆ | 213 |
| MAS | RrgA | SEQ ID 233 | spr0565B | SEQ ID 235 | His₆ | 214 |
| MAS | RrgA | SEQ ID 233 | spr0057 | LE | His₆ | 215 |
| MAS | RrgA | SEQ ID 233 | spr0096 | LE | His₆ | 216 |
| MAS | spr0565 | SEQ ID 233 | spr0057 | SEQ ID 235 | His₆ | 217 |
| MAS | spr0565A | SEQ ID 233 | spr0057 | SEQ ID 235 | His₆ | 218 |
| MAS | spr0565B | SEQ ID 233 | spr0057 | LE | His₆ | 219 |
| MAS | spr0565 | SEQ ID 233 | spr0096 | LE | His₆ | 220 |
| MAS | spr0565A | SEQ ID 233 | spr0096 | LE | His₆ | 221 |
| MAS | spr0565B | SEQ ID 233 | spr0096 | LE | His₆ | 222 |
| MAS | spr0565 | SEQ ID 233 | spr2021 | SEQ ID 235 | His₆ | 223 |
| MAS | spr0565A | SEQ ID 233 | spr2021 | SEQ ID 235 | His₆ | 224 |
| MAS | spr0565B | SEQ ID 233 | spr2021 | SEQ ID 235 | His₆ | 225 |
| MAS | spr0565 | SEQ ID 233 | RrgA | LE | His₆ | 226 |
| MAS | spr0565A | SEQ ID 233 | RrgA | LE | His₆ | 227 |
| MAS | spr0565B | SEQ ID 233 | RrgA | LE | His₆ | 228 |

The X₁ and X₂ moieties of these hybrids can be used in further hybrids.

As mentioned above, spr0057 polypeptide shows enzymatic activity using a 4-nitrophenyl-N-acetyl-β-D-glucosaminide substrate. This enzymatic activity was retained in various hybrid polypeptides (spr0096-spr0057; spr2021-spr0057; spr0057-spr2021; and spr0057-spr0096).

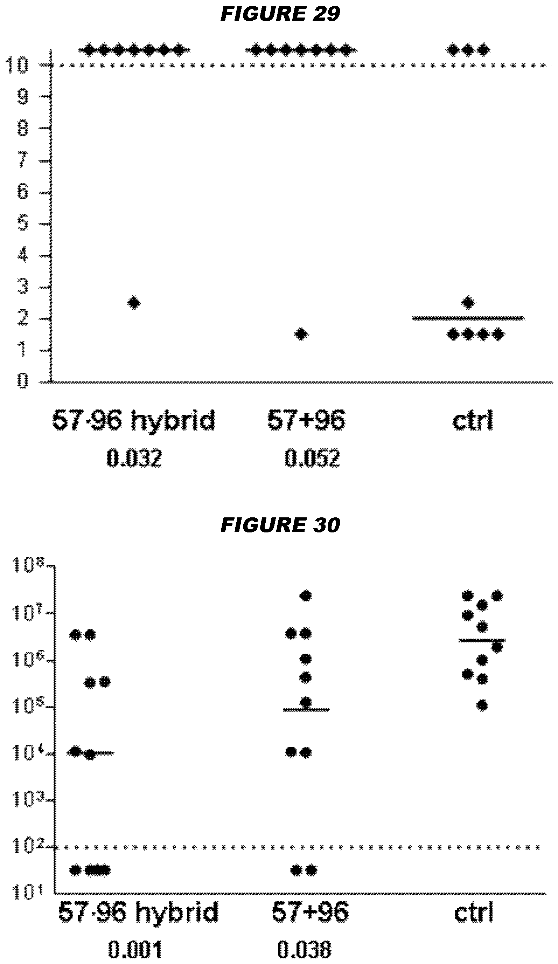

A combination of spr0057 and spr0096 was compared to a hybrid polypeptide spr0057-spr0096 in immunological tests. As shown in Figures 28-31, the hybrid showed equal or better efficacy than the combination. The biggest difference was seen in mortality after i.p. immunisation and subsequent i.v. challenge with TIGR4 using 5.8x10⁶ cfu (Figure 31).

### REFERENCES

[1] WO02/22168.
[2] Ogunniyi et al. (2007) Infect Immun. 75(1):350-7.
[3] WO02/079241
[4] WO02/34773.
[5] WO00/06737.
[6] WO00/06738.
[7] WO00/58475.
[8] WO2003/082183..
[9] Bagnoli et al. (2008) J Bacteriol. 190(15):5480-92.
[10] WO2004/092209.
[11] Research Disclosure, 453077 (Jan 2002).
[12] EP-A-0372501.
[13] EP-A-0378881.
[14] EP-A-0427347.
[15] WO93/17712.
[16] WO94/03208.
[17] WO98/58668.
[18] EP-A-0471177.
[19] WO91/01146.
[20] Falugi et al. (2001) Eur J Immunol 31:3816-3824.
[21] Baraldo et al. (2004) Infect Immun 72(8):4884-7.
[22] EP-A-0594610.
[23] Ruan et al. (1990) J Immunol 145:3379-3384.
[24] WO00/56360.
[25] Kuo et al. (1995) Infect Immun 63:2706-13.
[26] Michon et al. (1998) Vaccine. 16:1732-41.
[27] WO02/091998.
[28] WO01/72337.
[29] WO00/61761.
[30] WO00/33882
[31] WO2007/071707
[32] WO99/42130.
[33] US patent 4,761,283.
[34] US patent 4,356,170.
[35] US patent 4,882,317.
[36] US patent 4,695,624.
*[37]* Mol. Immunol., 1985, 22, 907-919
[38] EP-A-0208375.
[39] Bethell G.S. et al., J. Biol. Chem., 1979, 254, 2572-4
[40] Hearn M.T.W., J. Chromatogr., 1981, 218, 509-18
[41] WO00/10599.
[42] Gever et al., Med. Microbiol. Immunol, 165 : 171-288 (1979).
[43] US patent 4,057,685.
[44] US patents 4,673,574; 4,761,283; 4,808,700.
[45] US patent 4,459,286.
[46] US patent 4,965,338.
[47] US patent 4,663,160.
[48] WO2007/000343.
[49] Vaccines. (eds. Plotkin & Orenstein). 4th edition, 2004, ISBN: 0-7216-9688-0.
[50] Rappuoli et al. (1991) TIBTECH 9:232-238.
[51] Kirkham et al. (2006) Infect Immun. 74(1):586-93.
[52] WO2005/108580.
[53] Berry et al. (1999) Infect Immun 67(2):981-5.
[54] US-6716432.
[55] WO90/06951.
[56] WO99/03884.
[57] Baba et al. (2002) Infect Immun 70: 107-113.
[58] US-7217791
[59] WO2008/061953.
[60] Cao et al. (2007) Vaccine 25(27):4996-5005.
[61] WO2005/063283.
[62] WO2003/104272.
[63] WO00/37105.
[64] Adamou et al. (2001) Infect Immun. 69(2):949-58.
[65] WO98/18930.
[66] WO99/53940.
[67] WO02/22167.
[68] WO02/08426.
[69] WO01/12219.
[70] Briles et al. (2000) J Infect Dis 182:1694-1701.
[71] Talkington et al. (1996) Microb Pathog. 21(1):17-22.
[72] WO00/76540.
[73] Bethe et al. (2001) FEMS Microbiol Lett. 205(1):99-104.
[74] WO01/81380.
[75] Brown et al. (2001) Infect Immun 69:6702-6.
[76] Whalan et al. (2005) FEMS Immunol Med Microbiol 43:73-80.
[77] Jomaa et al. (2006) Vaccine. 24(24):5133-9.
[78] Giefing et al. (2008) J Exp Med 205:117-131.
[79] WO2007/116322.
[80] LeMieux et al. (2006) Infect Immun 74:2453-6.
[81] Nelson et al. (2007) Mol Microbiol 66:329-40.
[82] US patent 5,707,829
[83] Current Protocols in Molecular Biology (F.M. Ausubel et al. eds., 1987) Supplement 30.
[84] Hoskins et al. (2001) J.Bacteriol. 183:5709-5717.
[85] GenBank NC_004512.
[86] GenBank NC_003440.
[87] GenBank NC_003028.
[88] Tettelin et al. (2001) Science 293:498-506.
[89] WO02/077021.
[90] Needleman & Wunsch (1970) J. Mol. Biol. 48, 443-453.
[91] Rice et al. (2000) Trends Genet 16:276-277.
[92] US patent 6355271.
[93] WO00/23105.
[94] Vaccine Design... (1995) eds. Powell & Newman. ISBN: 030644867X. Plenum.
[95] WO90/14837.
[96] WO90/14837.
[97] Podda & Del Giudice (2003) Expert Rev Vaccines 2:197-203.
[98] Podda (2001) Vaccine 19: 2673-2680.
[99] *V*accine Design: The Subunit and Adjuvant Approach (eds. Powell & Newman) Plenum Press 1995 (ISBN 0-306-44867-X).
[100] Vaccine Adjuvants: Preparation Methods and Research Protocols (Volume 42 of Methods in Molecular Medicine series). ISBN: 1-59259-083-7. Ed. O'Hagan.
[101] WO2008/043774.
[102] Allison & Byars (1992) Res Immunol 143:519-25.
[103] Hariharan et al. (1995) Cancer Res 55:3486-9.
[104] US-2007/014805.
[105] US-2007/0191314.
[106] Suli et al. (2004) Vaccine 22(25-26):3464-9.
[107] WO95/11700.
[108] US patent 6,080,725.
[109] WO2005/097181.
[110] WO2006/113373.
[111] Han et al. (2005) Impact of Vitamin E on Immune Function and Infectious Diseases in the Aged at Nutrition, Immune functions and Health EuroConference, Paris, 9-10 June 2005.
[112] US- 6630161.
[113] US 5,057,540.
[114] WO96/337
[115] EP-A-0109942.
[116] WO96/11711.
[117] WO00/07621.
[118] Barr et al. (1998) Advanced Drug Delivery Reviews 32:247-271.
[119] Sjolanderet et al. (1998) Advanced Drug Delivery Reviews 32:321-338.
[120] Niikura et al. (2002) Virology 293:273-280.
[121] Lenz et al. (2001) J Immunol 166:5346-5355.
[122] Pinto et al. (2003) J Infect Dis 188:327-338.
[123] Gerber et al. (2001) J Virol 75:4752-4760.
[124] WO03/024480.
[125] WO03/024481.
[126] Gluck et al. (2002) Vaccine 20:B10-B16.
[127] EP-A-0689454.
[128] Johnson et al. (1999) Bioorg Med Chem Lett 9:2273-2278.
[129] Evans et al. (2003) Expert Rev Vaccines 2:219-229.
[130] Meraldi et al. (2003) Vaccine 21:2485-2491.
[131] Pajak et al. (2003) Vaccine 21:836-842.
[132] Kandimalla et al. (2003) Nucleic Acids Research 31:2393-2400.
[133] WO02/26757.
[134] WO99/62923.
[135] Krieg (2003) Nature Medicine 9:831-835.
[136] McCluskie et al. (2002) FEMS Immunology and Medical Microbiology 32:179-185.
[137] WO98/40100.
[138] US 6,207,646.
[139] US 6,239,116.
[140] US 6,429,199.
[141] Kandimalla et al. (2003) Biochemical Society Transactions 31 (part 3):654-658.
[142] Blackwell et al. (2003) J Immunol 170:4061-4068*.*
[143] Krieg (2002) Trends Immunol 23:64-65.
[144] WO01/9593
[145] Kandimalla et al. (2003) BBRC 306:948-953.
[146] Bhagat et al. (2003) BBRC 300:853-861.
[147] WO03/035836.
[148] WO01/2297
[149] Schellack et al. (2006) Vaccine 24:5461-72.
[150] Kamath et al. (2008) Eur J Immunol 38:1247-56*.*
[151] Riedl et al. (2008) Vaccine 26:3461-8.
[152] WO95/17211.
[153] WO98/42375.
[154] Beignon et al. (2002) Infect Immun 70:3012-3019.
[155] Pizza et al. (2001) Vaccine 19:2534-2541.
[156] Pizza et al. (2000) Int J Med Microbiol 290:455-461.
[157] Scharton-Kersten et al. (2000) Infect Immun 68:5306-5313.
[158] Ryan et al. (1999) Infect Immun 67:6270-6280.
[159] Partidos et al. (1999) Immunol Lett 67:209-216.
[160] Peppoloni et al. (2003) Expert Rev Vaccines 2:285-293.
[161] Pine et al. (2002) J Control Release 85:263-270.
[162] Tebbey et al. (2000) Vaccine 18:2723-34.
[163] Domenighini et al. (1995) Mol Microbiol 15:1165-1167*.*
[164] WO99/40936.
[165] WO99/44636.
[166] Singh et al] (2001) J Cont Release 70:267-276.
[167] WO99/27960.
[168] US 6,090,406.
[169] US 5,916,588.
[170] EP-A-0626169.
[171] WO99/52549.
[172] WO01/21207.
[173] WO01/21152.
[174] Andrianov et al. (1998) Biomaterials 19:109-115.
[175] Payne et al. (1998) Adv Drug Delivery Review 31:185-196.
[176] US 4,680,338.
[177] US 4,988,815.
[178] WO92/15582.
[179] Stanley (2002) Clin Exp Dermatol 27:571-577.
[180] Wu et al. (2004) Antiviral Res. 64(2):79-83.
[181] Vasilakos et al. (2000) Cell Immunol. 204(1):64-74.
[182] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[183] Jones (2003) Curr Opin Investig Drugs 4:214-218.
[184] WO03/011223.
[185] Johnson et al. (1999) Bioorg Med Chem Lett 9:2273-2278.
[186] Evans et al. (2003) Expert Rev Vaccines 2:219-229.
[187] WO2004/060308.
[188] WO2004/064759.
[189] US 6,924,271.
[190] US2005/0070556.
[191] US 5,658,731.
[192] US patent 5,011,828.
[193] WO2004/87153.
[194] US 6,605,617.
[195] WO02/18383.
[196] WO2004/018455.
[197] WO03/082272.
[198] Wong et al. (2003) J Clin Pharmacol 43(7):735-42*.*
[199] US2005/0215517.
[200] Dyakonova et al. (2004) Int Immunopharmacol 4(13):1615-23.
[201] FR-2859633.
[202] Signorelli & Hadden (2003) Int Immunopharmacol 3(8): 1177-86.
[203] WO2004/064715.
[204] De Libero et al, Nature Reviews Immunology, 2005, 5: 485-496
[205] US patent 5,936,076.
[206] Oki et al, J. Clin. Investig., 113: 1631-1640
[207] US2005/0192248
[208] Yang et al, Angew. Chem. Int. Ed., 2004, 43: 3818-3822
[209] WO2005/102049
[210] Goff et al, J. Am. Chem., Soc., 2004, 126: 13602-13603
[211] WO03/105769
[212] Cooper (1995) Pharm Biotechnol 6:559-80.
[213] WO99/11241.
[214] WO94/00153.
[215] WO98/57659.
[216] European patent applications 0835318, 0735898 and 0761231.
[217] WO2006/11060
[218] Zwijnenburg et al. (2001) J Infect Dis 183:1143-6.
[219] Donnelly et al. (1997) Annu Rev Immunol 15:617-648*.*
[220] Strugnell et al. (1997) Immunol Cell Biol 75(4):364-369*.*
[221] Cui (2005) Adv Genet 54:257-89.
[222] Robinson & Torres (1997) Seminars in Immunol 9:271-283.
[223] Brunham et al. (2000) J Infect Dis 181 Suppl 3:S538-43.
[224] Svanholm et al. (2000) Scand J Immunol 51(4):345-53.
*[225]* DNA Vaccination - Genetic Vaccination (1998) eds. Koprowski et al. (ISBN 3540633928).
*[226]* Gene Vaccination : Theory and Practice (1998) ed. Raz (ISBN 3540644288).
[227] Ferreira et al. (2006) J Med Microbiol. 55(Pt 4):375-8.
[228] Findeis et al., Trends Biotechnol. (1993) 11:202
[229] Chiou et al. (1994) Gene Therapeutics: Methods And Applications Of Direct Gene Transfer. ed. Wolff
[230] Wu et al., J. Biol. Chem. (1988) 263:621
[231] Wu et al., J. Biol. Chem. (1994) 269:542
[232] Zenke et al., Proc. Natl. Acad. Sci. (USA) (1990) 87:3655
[233] Wu et al., J. Biol. Chem. (1991) 266:338
[234] Jolly, Cancer Gene Therapy (1994) 1:51
[235] Kimura, Human Gene Therapy (1994) 5:845
[236] Connelly, Human Gene Therapy (1995) 1:185
[237] Kaplitt, Nature Genetics (1994) 6:148
[238] WO 90/07936.
[239] WO 94/03622.
[240] WO 93/25698.
[241] WO 93/25234.
[242] US patent 5,219,740.
[243] WO 93/11230.
[244] WO 93/10218.
[245] US patent 4,777,127.
[246] GB Patent No. 2,200,651.
[247] EP-A-0345242.
[248] WO 91/02805.
[249] WO 94/12649.
[250] WO 93/03769.
[251] WO 93/19191.
[252] WO 94/28938.
[253] WO 95/11984.
[254] WO 95/00655.
[255] Curiel, Hum. Gene Ther. (1992) 3:147
[256] Wu, J. Biol. Chem. (1989) 264:16985
[257] US patent 5,814,482.
[258] WO 95/07994.
[259] WO 96/17072.
[260] WO 95/30763.
[261] WO 97/42338.
[262] WO 90/11092.
[263] US patent 5,580,859
[264] US patent 5,422,120
[265] WO 95/13796.
[266] WO 94/23697.
[267] WO 91/14445.
[268] EP-0524968.
[269] Philip, Mol. Cell Biol. (1994) 14:2411
[270] Woffendin, Proc. Natl. Acad. Sci. (1994) 91:11581
[271] US patent 5,206,152.
[272] WO 92/11033.
[273] US patent 5,149,655.
[274] Brandt et al. (2006) J Antimicrob Chemother. 58(6):1291-4. Epub 2006 Oct 26
[275] Winter et al., (1991) Nature 349:293-99
[276] US 4,816,567.
[277] Inbar et al., (1972) Proc. Natl. Acad. Sci. U.S.A. 69:2659-62.
[278] Ehrlich et al., (1980) Biochem 19:4091-96.
[279] Huston et al., (1988) Proc. Natl. Acad. Sci. U.S.A. 85:5897-83.
[280] Pack et al., (1992) Biochem 31, 1579-84.
[281] Cumber et al., (1992) J. Immunology 149B, 120-26.
[282] Riechmann et al., (1988) Nature 332, 323-27.
[283] Verhoeyan et al., (1988) Science 239, 1534-36.
[284] GB 2,276,169.
[285] Gennaro (2000) Remington: The Science and Practise of Pharmacy. 20th edition, ISBN: 0683306472.
[286] Methods In Enzymology (S. Colowick and N. Kaplan, eds., Academic Press, Inc.)
*[287]* Handbook of Experimental Immunology, Vols. I-IV (D.M. Weir and C.C. Blackwell, eds, 1986, Blackwell Scientific Publications)
[288] Sambrook et al. (2001) Molecular Cloning: A Laboratory Manual, 3rd edition (Cold Spring Harbor Laboratory Press).
[289] Handbook of Surface and Colloidal Chemistry (Birdi, K.S. ed., CRC Press, 1997)
[290] Ausubel et al. (eds) (2002) Short protocols in molecular biology, 5th edition (Current Protocols).
[291] Molecular Biology Techniques: An Intensive Laboratory Course, (Ream et al., eds., 1998, Academic Press)
[292] PCR (Introduction to Biotechniques Series), 2nd ed. (Newton & Graham eds., 1997, Springer Verlag)
[293] Geysen et al. (1984) PNAS USA 81:3998-4002.
[294] Carter (1994) Methods Mol Biol 36:207-23.
[295] Jameson, BA et al. 1988, CABIOS 4(1):181-186.
[296] Raddrizzani & Hammer (2000) Brief Bioinform 1(2):179-89.
[297] Bublil et al. (2007) Proteins 68(1):294-304.
[298] De Lalla et al. (1999) J. Immunol. 163:1725-29.
[299] Kwok et al. (2001) Trends Immunol 22:583-88.
[300] Brusic et al. (1998) Bioinformatics 14(2):121-30
[301] Meister et al. (1995) Vaccine 13(6):581-91.
[302] Roberts et al. (1996) AIDS Res Hum Retroviruses 12(7):593-610.
[303] Maksyutov & Zagrebelnaya (1993) Comput Appl Biosci 9(3):291-7.
[304] Feller & de la Cruz (1991) Nature 349(6311):720-1.
[305] Hopp (1993) Peptide Research 6:183-190.
[306] Welling et al. (1985) FEBS Lett. 188:215-218.
[307] Davenport et al. (1995) Immunogenetics 42:392-297.
[308] Tsurui & Takahashi (2007) J Pharmacol Sci. 105(4):299-316.
[309] Tong et al. (2007) Brief Bioinform. 8(2):96-108.
[310] Schirle et al. (2001) J Immunol Methods. 257(1-2):1-16.
[311] Chen et al. (2007) Amino Acids 33(3):423-8.
[312] Current Protocols in Molecular Biology (F.M. Ausubel et al., eds., 1987) Supplement 30
[313] Smith & Waterman (1981) Adv. Appl. Math. 2: 482-489.
[314] King et al. (2006) Mol Microbiol 59(3):961-74.
[315] Zysk et al. (2000) Infect Immun 68:3740-3.
[316] Wellmer et al. (2002) Infect Immun 70:6504-8.
[317] Gosink et al. (2000) Infect Immun 68(10):5690-5.
[318] Wizemann et al. (2001) Infect Immun 69:1593-8.
[319] Moscoso et al. (2005) Appl Environ Microbiol 71:8706-13..
[320] Kharat & Tomasz (2003) Infect Immun 71:2758-65.
[321] Chen et al. (2005) FEMS Microbiol Lett. 253(1):151-4.
[322] Bumbaca et al. (2007) Proteins. 66(3):547-58.
[323] Monterroso et al. (2005) Biochem J. 391:41-9.
[324] Moscoso et al. (2005) J Bacteriology 187:6238-41.
[325] Ogunniyi et al. (2001) Infect Immun 69:5997-6003.
[326] Ng et al. (2005) J Bacteriol. 187(21):7444-59.
[327] Mills et al. (2007) J Bacteriol. 189:4544-6.
[328] WO2008/079732.

## Claims

1. An immunogenic composition comprising a combination of *S.pneumoniae* antigens, said combination comprising a spr0096 antigen and a spr2021 antigen, wherein:
said spr0096 antigen is a polypeptide that comprises an amino acid sequence: (a) having 80% or more identity to SEQ ID NO: 12; and/or (b) comprising a fragment of at least 10 consecutive amino acids, comprising an epitope of SEQ ID NO: 12; and
said spr2021 antigen is a polypeptide that comprises an amino acid sequence: (a) having 80% or more identity to SEQ ID NO: 11; and/or (b) comprising a fragment of at least 10 consecutive amino acids, comprising an epitope of SEQ ID NO: 11.

2. An immunogenic composition according to claim 1 comprising one or more antigens selected from the group consisting of:
(1) a spr1739 antigen comprising an amino acid sequence: (a) having 80% or more identity to SEQ ID NO: 10; and/or (b) comprising a fragment of at least 10 consecutive amino acids, comprising an epitope of SEQ ID NO: 10;
(2) a spr0867 antigen comprising an amino acid sequence: (a) having 80% or more identity to SEQ ID NO: 8; and/or (b) comprising a fragment of at least 10 consecutive amino acids, comprising an epitope of SEQ ID NO: 8;
(3) a spr1431 antigen comprising an amino acid sequence: (a) having 80% or more identity to SEQ ID NO: 9; and/or (b) comprising a fragment of at least 10 consecutive amino acids, comprising an epitope of SEQ ID NO: 9;
(4) a spr1433 antigen comprising an amino acid sequence: (a) having 80% or more identity to SEQ ID NO: 13; and/or (b) comprising a fragment of at least 10 consecutive amino acids, comprising an epitope of SEQ ID NO: 13;
(5) a spr1707 antigen comprising an amino acid sequence: (a) having 80% or more identity to SEQ ID NO: 14; and/or (b) comprising a fragment of at least 10 consecutive amino acids, comprising an epitope of SEQ ID NO: 14;
(6) a spr0057 antigen comprising an amino acid sequence: (a) having 80% or more identity to SEQ ID NO: 1; and/or (b) comprising a fragment of at least 10 consecutive amino acids, comprising an epitope of SEQ ID NO: 1;
(7) a spr0565 antigen comprising an amino acid sequence: (a) having 80% or more identity to SEQ ID NO: 3; and/or (b) comprising a fragment of at least 10 consecutive amino acids, comprising an epitope of SEQ ID NO: 3;
(8) a spr1345 antigen comprising an amino acid sequence: (a) having 80% or more identity to SEQ ID NO: 5; and/or (b) comprising a fragment of at least 10 consecutive amino acids, comprising an epitope of SEQ ID NO: 5;
(9) a spr1416 antigen comprising an amino acid sequence: (a) having 80% or more identity to SEQ ID NO: 6; and/or (b) comprising a fragment of at least 10 consecutive amino acids, comprising an epitope of SEQ ID NO: 6;
(10) a spr1418 antigen comprising an amino acid sequence: (a) having 80% or more identity to SEQ ID NO: 7; and/or (b) comprising a fragment of at least 10 consecutive amino acids, comprising an epitope of SEQ ID NO: 8;
(11) a spr1098 antigen comprising an amino acid sequence: (a) having 80% or more identity to SEQ ID NO: 4; and/or (b) comprising a fragment of at least 10 consecutive amino acids, comprising an epitope of SEQ ID NO: 4; and/or
(12) a spr0286 antigen comprising an amino acid sequence: (a) having 80% or more identity to SEQ ID NO: 2; and/or (b) comprising a fragment of at least 10 consecutive amino acids, comprising an epitope of SEQ ID NO: 2;
wherein preferably, the one or more antigens is/are selected from the group consisting of: (1) spr1739 antigen; (2) a spr0867 antigen; (3) a spr1431 antigen; (4) a spr1433 antigen; and/or (5) a spr1707 antigen.

3. An immunogenic composition according to claim 1, comprising a spr0057 antigen and/or a spr0565 antigen.

4. The composition of claim 3, further comprising one or more of:
(a) a *S.pneumoniae* RrgA and/or RrgB pilus antigen;
(b) a *S.pneumoniae* Pmp antigen; and/or
(c) one or more conjugates of a pneumococcal capsular saccharide and a carrier protein, wherein (i) the capsular saccharide is from one or more of the following pneumococcal serotypes: 1, 3, 4, 5, 6B, 7F, 9V, 14, 18C, 19F and 23F; and (ii) the carrier protein is a bacterial toxin or toxoid, or is protein D from *H.influenzae.*
wherein:
said RrgA antigen comprises an amino acid sequence: (a) having 80% or more identity to SEQ ID NO: 172 or 179; and/or (b) comprises a fragment of at least 10 consecutive amino acids, comprising an epitope of SEQ ID NO: 172 or 179;
said RrgB antigen comprises an amino acid sequence: (a) having 80% or more identity to SEQ ID NO: 173, 174 or 175; and/or (b) comprises a fragment of at least 10 consecutive amino acids, comprising an epitope of SEQ ID NO: 173, 174, or 175; and/or
said Pmp antigen comprises an amino acid sequence: (a) having 80% or more identity to SEQ ID NO: 28; and/or (b) comprises a fragment of at least 10 consecutive amino acids, comprising an epitope of SEQ ID NO: 28.

5. An immunogenic composition according to claim 1 or claim 2 comprising:
(a) one or more conjugates of a pneumococcal capsular saccharide and a carrier protein;
(b) one or more antigen(s) selected from the group consisting of: diphtheria toxoid; tetanus toxoid; hepatitis B virus surface antigen; an inactivated poliovirus antigen; one or more acellular pertussis antigens; a conjugate of the capsular saccharide antigen from *H.influenzae* type B; a conjugate of the capsular saccharide antigen from serogroup C of *N.meningitidis*; a conjugate of the capsular saccharide antigen from serogroup Y of *N.meningitidis;* a conjugate of the capsular saccharide antigen from serogroup W135 of *N.meningitidis*; and a conjugate of the capsular saccharide antigen from serogroup A of *N.meningitidis;* or
(c) a temperature protective agent that improves the thermal stability of the composition.

6. A polypeptide of formula NH₂-A-{-X-L-}*ₙ*-B-COOH, wherein:
X is an amino acid sequence of a pneumococcal antigen, selected from the group consisting of: (1) a spr0057 antigen; (2) a spr0096 antigen; (3) a spr0565 antigen; and (4) a spr2021 antigen; (5) a RrgA antigen; and (6) a RrgB antigen;
L is an optional linker amino acid sequence;
A is an optional N-terminal amino acid sequence;
B is an optional C-terminal amino acid sequence,
*n* is an integer of 2 or more; and
at least one instance of X is spr0096 and at least one instance of X is spr2021;
wherein:
said spr0057 antigen comprises an amino acid sequence: (a) having 80% or more identity to SEQ ID NO: 1; and/or (b) comprises a fragment of at least 10 consecutive amino acids, comprising an epitope of SEQ ID NO: 1;
said spr0096 antigen comprises an amino acid sequence: (a) having 80% or more identity to SEQ ID NO: 12; and/or (b) comprises a fragment of at least 10 consecutive amino acids, comprising an epitope of SEQ ID NO: 12;
said spr0565 antigen comprises an amino acid sequence: (a) having 80% or more identity to SEQ ID NO: 3; and/or (b) comprises a fragment of at least 10 consecutive amino acids, comprising an epitope of SEQ ID NO: 3;
said spr2021 antigen comprises an amino acid sequence: (a) having 80% or more identity to SEQ ID NO: 11; and/or (b) comprises a fragment of at least 10 consecutive amino acids, comprising an epitope of SEQ ID NO: 11;
said RrgA antigen comprises an amino acid sequence: (a) having 80% or more identity to SEQ ID NO: 172 or 179; and/or (b) comprises a fragment of at least 10 consecutive amino acids, comprising an epitope of SEQ ID NO: 172 or 179; and/or
said RrgB antigen comprises an amino acid sequence: (a) having 80% or more identity to SEQ ID NO: 173, 174 or 175; and/or (b) comprises a fragment of at least 10 consecutive amino acids, comprising an epitope of SEQ ID NO: 173, 174, or 175.

7. A polypeptide according to claim 6, having the formula NH₂-A-X₁-L₁-X₂-L₂-B-COOH, wherein:
(a) X₁ is an amino acid sequence of a spr0096 pneumococcal antigen and X₂ is an amino acid sequence of a spr2021 pneumococcal antigen; or
(b) X₁ is an amino acid sequence of a spr2021 pneumococcal antigen and X₂ is an amino acid sequence of a spr0096 pneumococcal antigen;
L is an optional linker amino acid sequence;
A is an optional N-terminal amino acid sequence;
B is an optional C-terminal amino acid sequence;
and wherein:
said spr0096 antigen comprises an amino acid sequence: (a) having 80% or more identity to SEQ ID NO: 12; and/or (b) comprises a fragment of at least 10 consecutive amino acids, comprising an epitope of SEQ ID NO: 12; and/or
said spr2021 antigen comprises an amino acid sequence: (a) having 80% or more identity to SEQ ID NO: 11; and/or (b) comprises a fragment of at least 10 consecutive amino acids, comprising an epitope of SEQ ID NO: 11.

8. The polypeptide of claim 6 or claim 7, wherein at least one L sequence comprises Glyₓ, where x = 2, 3, 4, 5, 6, 7, 8, 9 or 10.

9. The polypeptide of claim 6 or claim 7, comprising an amino acid sequence selected from the group consisting of SEQ ID NOs: 194 and 205.

10. A polypeptide having 75% or more sequence identity to an amino acid sequence selected from the group consisting of SEQ ID NOs: 194 and 205.

11. An immunogenic composition comprising the polypeptide of any one of claims 6 to 10.

12. Nucleic acid encoding the polypeptide of any one of claims 6 to 10.

13. The immunogenic composition of any one of claims 1-5 or 11 for use in a method of raising an immune response in a mammal.

14. The immunogenic composition of claim 13 for use in protecting said mammal against pneumococcal infection.

## Patentansprüche

1. Immunogene Zusammensetzung, umfassend eine Kombination von S. pneumoniae-Antigenen, wobei die Kombination ein spr0096-Antigen und ein spr2021-Antigen umfasst, wobei:
das spr0096-Antigen ein Polypeptid ist, das eine Aminosäuresequenz umfasst: (a) mit 80 % oder mehr Identität zu SEQ ID NO: 12; und/oder (b) umfassend ein Fragment von zumindest 10 aufeinanderfolgenden Aminosäuren, umfassend ein Epitop von SEQ ID NO: 12; und
das spr2021-Antigen ein Polypeptid ist, das eine Aminosäuresequenz umfasst: (a) mit 80 % oder mehr Identität zu SEQ ID NO: 11; und/oder (b) umfassend ein Fragment von zumindest 10 aufeinanderfolgenden Aminosäuren, umfassend ein Epitop von SEQ ID NO: 11.

2. Immunogene Zusammensetzung gemäss Anspruch 1, umfassend ein oder mehrere Antigen(e), ausgewählt aus der Gruppe bestehend aus:
(1) einem spr1739-Antigen, umfassend eine Aminosäuresequenz: (a) mit 80 % oder mehr Identität zu SEQ ID NO: 10; und/oder (b) umfassend ein Fragment von zumindest 10 aufeinanderfolgenden Aminosäuren, umfassend ein Epitop von SEQ ID NO: 10;
(2) einem spr0867-Antigen, umfassend eine Aminosäuresequenz: (a) mit 80 % oder mehr Identität zu SEQ ID NO: 8; und/oder (b) umfassend ein Fragment von zumindest 10 aufeinanderfolgenden Aminosäuren, umfassend ein Epitop von SEQ ID NO: 8;
(3) einem spr1431-Antigen, umfassend eine Aminosäuresequenz: (a) mit 80 % oder mehr Identität zu SEQ ID NO: 9; und/oder (b) umfassend ein Fragment von zumindest 10 aufeinanderfolgenden Aminosäuren, umfassend ein Epitop von SEQ ID NO: 9;
(4) einem spr1433-Antigen, umfassend eine Aminosäuresequenz: (a) mit 80 % oder mehr Identität zu SEQ ID NO: 13; und/oder (b) umfassend ein Fragment von zumindest 10 aufeinanderfolgenden Aminosäuren, umfassend ein Epitop von SEQ ID NO: 13;
(5) einem spr1707-Antigen, umfassend eine Aminosäuresequenz: (a) mit 80 % oder mehr Identität zu SEQ ID NO: 14; und/oder (b) umfassend ein Fragment von zumindest 10 aufeinanderfolgenden Aminosäuren, umfassend ein Epitop von SEQ ID NO: 14;
(6) einem spr0057-Antigen, umfassend eine Aminosäuresequenz: (a) mit 80 % oder mehr Identität zu SEQ ID NO: 1; und/oder (b) umfassend ein Fragment von zumindest 10 aufeinanderfolgenden Aminosäuren, umfassend ein Epitop von SEQ ID NO: 1;
(7) einem spr0565-Antigen, umfassend eine Aminosäuresequenz: (a) mit 80 % oder mehr Identität zu SEQ ID NO: 3; und/oder (b) umfassend ein Fragment von zumindest 10 aufeinanderfolgenden Aminosäuren, umfassend ein Epitop von SEQ ID NO: 3;
(8) einem spr1345-Antigen, umfassend eine Aminosäuresequenz: (a) mit 80 % oder mehr Identität zu SEQ ID NO: 5; und/oder (b) umfassend ein Fragment von zumindest 10 aufeinanderfolgenden Aminosäuren, umfassend ein Epitop von SEQ ID NO: 5;
(9) einem spr1416-Antigen, umfassend eine Aminosäuresequenz: (a) mit 80 % oder mehr Identität zu SEQ ID NO: 6; und/oder (b) umfassend ein Fragment von zumindest 10 aufeinanderfolgenden Aminosäuren, umfassend ein Epitop von SEQ ID NO: 6;
(10) einem spr1418-Antigen, umfassend eine Aminosäuresequenz: (a) mit 80 % oder mehr Identität zu SEQ ID NO: 7; und/oder (b) umfassend ein Fragment von zumindest 10 aufeinanderfolgenden Aminosäuren, umfassend ein Epitop von SEQ ID NO: 8;
(11) einem spr1098-Antigen, umfassend eine Aminosäuresequenz: (a) mit 80 % oder mehr Identität zu SEQ ID NO: 4; und/oder (b) umfassend ein Fragment von zumindest 10 aufeinanderfolgenden Aminosäuren, umfassend ein Epitop von SEQ ID NO: 4; und/oder
(12) einem spr0286-Antigen, umfassend eine Aminosäuresequenz: (a) mit 80 % oder mehr Identität zu SEQ ID NO: 2; und/oder (b) umfassend ein Fragment von zumindest 10 aufeinanderfolgenden Aminosäuren, umfassend ein Epitop von SEQ ID NO: 2;
wobei vorzugsweise das/die eine oder mehreren Antigen(e) aus der Gruppe bestehend aus (1) einem spr1739-Antigen; (2) einem spr0867-Antigen; (3) einem spr1431-Antigen; (4) einem spr1433-Antigen und/oder (5) einem spr1707-Antigen ausgewählt ist/sind.

3. Immunogene Zusammensetzung gemäss Anspruch 1, umfassend ein spr0057-Antigen und/oder ein spr0565-Antigen.

4. Zusammensetzung gemäss Anspruch 3, ferner umfassend eines oder mehrere von:
(a) einem S. pneumoniae-RrgA- und/oder -RrgBpilus-Antigen;
(b) einem S. pneumoniae-Pmp-Antigen; und/oder
(c) einem oder mehreren Konjugat(en) eines Pneumokokken-Kapselsaccharids und eines Trägerproteins, wobei (i) das Kapselsaccharid aus einem oder mehreren der nachstehenden Pneumokokken-Serotypen besteht: 1, 3, 4, 5, 6B, 7F, 9V, 14, 18C, 19F und 23F; und (ii) das Trägerprotein ein bakterielles Toxin oder Toxoid ist oder Protein D von H. influenzae ist,
wobei
das RrgA-Antigen eine Aminosäuresequenz umfasst: (a) mit 80 % oder mehr Identität zu SEQ ID NO: 172 oder 179; und/oder (b) umfassend ein Fragment von zumindest 10 aufeinanderfolgenden Aminosäuren, umfassend ein Epitop von SEQ ID NO: 172 oder 179;
das RrgB-Antigen eine Aminosäuresequenz umfasst: (a) mit 80 % oder mehr Identität zu SEQ ID NO: 173, 174 oder 175; und/oder (b) umfassend ein Fragment von zumindest 10 aufeinanderfolgenden Aminosäuren, umfassend ein Epitop von SEQ ID NO: 173, 174 oder 175; und/oder
das Pmp-Antigen eine Aminosäuresequenz umfasst: (a) mit 80 % oder mehr Identität zu SEQ ID NO: 28; und/oder (b) umfassend ein Fragment von zumindest 10 aufeinanderfolgenden Aminosäuren, umfassend ein Epitop von SEQ ID NO: 28.

5. Immunogene Zusammensetzung gemäss Anspruch 1 oder Anspruch 2, umfassend:
(a) ein oder mehrere Konjugat(e) eines Pneumokokken-Kapselsaccharids und eines Trägerproteins;
(b) ein oder mehrere Antigen(e), ausgewählt aus der Gruppe bestehend aus: Diphtherietoxoid; Tetanustoxoid; einem Hepatitis B-Virus-Oberflächenantigen; einem inaktivierten Poliovirus-Antigen; einem oder mehreren azellulären Pertussis-Antigen(en); einem Konjugat des Kapselsaccharid-Antigens von H. influenzae Typ B; einem Konjugat des Kapselsaccharid-Antigens der Serogruppe C von N. meningitidis; einem Konjugat des Kapselsaccharid-Antigens der Serogruppe Y von N. meningitidis; einem Konjugat des Kapselsaccharid-Antigens der Serogruppe W135 von N. meningitidis und einem Konjugat des Kapselsaccharid-Antigens der Serogruppe A von N. meningitidis; oder
(c) ein Temperaturschutzmittel, welches die thermische Stabilität der Zusammensetzung verbessert.

6. Polypeptid der Formel NH₂-A-{-X-L-}ₙ-B-COOH, worin:
X eine Aminosäuresequenz eines Pneumokokken-Antigens, ausgewählt aus der Gruppe bestehend aus (1) einem spr0057-Antigen; (2) einem spr0096-Antigen; (3) einem spr0565-Antigen; (4) einem spr2021-Antigen; (5) einem RrgA-Antigen und (6) einem RrgB-Antigen, ist;
L eine optionale Linker-Aminosäuresequenz ist;
A eine optionale N-terminale Aminosäuresequenz ist;
B eine optionale C-terminale Aminosäuresequenz ist,
n eine ganze Zahl von 2 oder mehr ist; und
zumindest eine Instanz von X spr0096 ist und zumindest eine Instanz von X spr2021 ist;
wobei:
das spr0057-Antigen eine Aminosäuresequenz umfasst: (a) mit 80 % oder mehr Identität zu SEQ ID NO: 1; und/oder (b) umfassend ein Fragment von zumindest 10 aufeinanderfolgenden Aminosäuren, umfassend ein Epitop von SEQ ID NO: 1;
das spr0096-Antigen eine Aminosäuresequenz umfasst: (a) mit 80 % oder mehr Identität zu SEQ ID NO: 12; und/oder (b) umfassend ein Fragment von zumindest 10 aufeinanderfolgenden Aminosäuren, umfassend ein Epitop von SEQ ID NO: 12;
das spr0565-Antigen eine Aminosäuresequenz umfasst: (a) mit 80 % oder mehr Identität zu SEQ ID NO: 3; und/oder (b) umfassend ein Fragment von zumindest 10 aufeinanderfolgenden Aminosäuren, umfassend ein Epitop von SEQ ID NO: 3;
das spr2021-Antigen eine Aminosäuresequenz umfasst: (a) mit 80 % oder mehr Identität zu SEQ ID NO: 11; und/oder (b) umfassend ein Fragment von zumindest 10 aufeinanderfolgenden Aminosäuren, umfassend ein Epitop von SEQ ID NO: 11;
das RrgA-Antigen eine Aminosäuresequenz umfasst: (a) mit 80 % oder mehr Identität zu SEQ ID NO: 172 oder 179; und/oder (b) umfassend ein Fragment von zumindest 10 aufeinanderfolgenden Aminosäuren, umfassend ein Epitop von SEQ ID NO: 172 oder 179;
das RrgB-Antigen eine Aminosäuresequenz umfasst: (a) mit 80 % oder mehr Identität zu SEQ ID NO: 173, 174 oder 175; und/oder (b) umfassend ein Fragment von zumindest 10 aufeinanderfolgenden Aminosäuren, umfassend ein Epitop von SEQ ID NO: 173, 174 oder 175.

7. Polypeptid gemäss Anspruch 6 mit der Formel NH₂-A-X₁-L₁-X₂-L₂-B-COOH, worin:
(a) X₁ eine Aminosäuresequenz eines spr0096-Pneumokokken-Antigens ist und X₂ eine Aminosäuresequenz eines spr2021-Pneumokokken-Antigens ist; oder
(b) X₁ eine Aminosäuresequenz eines spr2021-Pneumokokken-Antigens ist und X₂ eine Aminosäuresequenz eines spr0096-Pneumokokken-Antigens ist;
L eine optionale Linker-Aminosäuresequenz ist;
A eine optionale N-terminale Aminosäuresequenz ist;
B eine optionale C-terminale Aminosäuresequenz ist;
und wobei:
das spr0096-Antigen eine Aminosäuresequenz umfasst: (a) mit 80 % oder mehr Identität zu SEQ ID NO: 12; und/oder (b) umfassend ein Fragment von zumindest 10 aufeinanderfolgenden Aminosäuren, umfassend ein Epitop von SEQ ID NO: 12; und/oder
das spr2021-Antigen eine Aminosäuresequenz umfasst: (a) mit 80 % oder mehr Identität zu SEQ ID NO: 11; und/oder (b) umfassend ein Fragment von zumindest 10 aufeinanderfolgenden Aminosäuren, umfassend ein Epitop von SEQ ID NO: 11.

8. Polypeptid gemäss Anspruch 6 oder Anspruch 7, wobei zumindest eine L-Sequenz Glyₓ umfasst, worin x = 2, 3, 4, 5, 6, 7, 8, 9 oder 10.

9. Polypeptid gemäss Anspruch 6 oder Anspruch 7, umfassend eine Aminosäuresequenz, ausgewählt aus der Gruppe bestehend aus den SEQ ID NOs: 194 und 205.

10. Polypeptid mit 75 % oder mehr Sequenzidentität zu einer Aminosäuresequenz, ausgewählt aus der Gruppe bestehend aus den SEQ ID NOs: 194 und 205.

11. Immunogene Zusammensetzung, umfassend ein Polypeptid gemäss irgendeinem der Ansprüche 6 bis 10.

12. Nukleinsäure, die das Polypeptid gemäss irgendeinem der Ansprüche 6 bis 10 kodiert.

13. Immunogene Zusammensetzung gemäss irgendeinem der Ansprüche 1 bis 5 oder 11 zur Verwendung in einem Verfahren zur Steigerung der Immunantwort in einem Säuger.

14. Immunogene Zusammensetzung gemäss Anspruch 13 zur Verwendung zum Schutz des Säugers gegen eine Pneumokokken-Infektion.

## Revendications

1. Composition immunogène comprenant une combinaison d'antigènes de *S. pneumoniae,* ladite combinaison comprenant un antigène spr0096 et un antigène spr2021, dans laquelle :
ledit antigène spr0096 est un polypeptide qui comprend une séquence d'acides aminés : (a) ayant une identité de 80 % ou plus avec la SEQ ID N° : 12 ; et/ou (b) comprenant un fragment d'au moins 10 acides aminés consécutifs, comprenant un épitope de la SEQ ID N° : 12 ; et
ledit antigène spr2021 est un polypeptide qui comprend une séquence d'acides aminés : (a) ayant une identité de 80 % ou plus avec la SEQ ID N° : 11 ; et/ou (b) comprenant un fragment d'au moins 10 acides aminés consécutifs, comprenant un épitope de la SEQ ID N° : 11.

2. Composition immunogène selon la revendication 1 comprenant un ou plusieurs antigènes choisis dans le groupe constitué de :
(1) un antigène spr1739 comprenant une séquence d'acides aminés : (a) ayant une identité de 80 % ou plus avec la SEQ ID N° : 10 ; et/ou (b) comprenant un fragment d'au moins 10 acides aminés consécutifs, comprenant un épitope de la SEQ ID N° : 10 ;
(2) un antigène spr0867 comprenant une séquence d'acides aminés : (a) ayant une identité de 80 % ou plus avec la SEQ ID N° : 8 ; et/ou (b) comprenant un fragment d'au moins 10 acides aminés consécutifs, comprenant un épitope de la SEQ ID N° : 8 ;
(3) un antigène spr1431 comprenant une séquence d'acides aminés : (a) ayant une identité de 80 % ou plus avec la SEQ ID N° : 9 ; et/ou (b) comprenant un fragment d'au moins 10 acides aminés consécutifs, comprenant un épitope de la SEQ ID N° : 9 ;
(4) un antigène spr1433 comprenant une séquence d'acides aminés : (a) ayant une identité de 80 % ou plus avec la SEQ ID N° : 13 ; et/ou (b) comprenant un fragment d'au moins 10 acides aminés consécutifs, comprenant un épitope de la SEQ ID N° : 13 ;
(5) un antigène spr1707 comprenant une séquence d'acides aminés : (a) ayant une identité de 80 % ou plus avec la SEQ ID N° : 14 ; et/ou (b) comprenant un fragment d'au moins 10 acides aminés consécutifs, comprenant un épitope de la SEQ ID N° : 14 ;
(6) un antigène spr0057 comprenant une séquence d'acides aminés : (a) ayant une identité de 80 % ou plus avec la SEQ ID N° : 1 ; et/ou (b) comprenant un fragment d'au moins 10 acides aminés consécutifs, comprenant un épitope de la SEQ ID N° : 1 ;
(7) un antigène spr0565 comprenant une séquence d'acides aminés : (a) ayant une identité de 80 % ou plus avec la SEQ ID N° : 3 ; et/ou (b) comprenant un fragment d'au moins 10 acides aminés consécutifs, comprenant un épitope de la SEQ ID N° : 3 ;
(8) un antigène spr1345 comprenant une séquence d'acides aminés : (a) ayant une identité de 80 % ou plus avec la SEQ ID N° : 5 ; et/ou (b) comprenant un fragment d'au moins 10 acides aminés consécutifs, comprenant un épitope de la SEQ ID N° : 5 ;
(9) un antigène spr1416 comprenant une séquence d'acides aminés : (a) ayant une identité de 80 % ou plus avec la SEQ ID N° : 6 ; et/ou (b) comprenant un fragment d'au moins 10 acides aminés consécutifs, comprenant un épitope de la SEQ ID N° : 6 ;
(10) un antigène spr1418 comprenant une séquence d'acides aminés : (a) ayant une identité de 80 % ou plus avec la SEQ ID N° : 7 ; et/ou (b) comprenant un fragment d'au moins 10 acides aminés consécutifs, comprenant un épitope de la SEQ ID N° : 8 ;
(11) un antigène spr1098 comprenant une séquence d'acides aminés : (a) ayant une identité de 80 % ou plus avec la SEQ ID N° : 4 ; et/ou (b) comprenant un fragment d'au moins 10 acides aminés consécutifs, comprenant un épitope de la SEQ ID N° : 4 ; et/ou
(12) un antigène spr0286 comprenant une séquence d'acides aminés : (a) ayant une identité de 80 % ou plus avec la SEQ ID N° : 2 ; et/ou (b) comprenant un fragment d'au moins 10 acides aminés consécutifs, comprenant un épitope de la SEQ ID N° : 2 ;
dans laquelle, de préférence, le ou les antigènes sont choisis dans le groupe constitué due : (1) l'antigène spr1739 ; (2) l'antigène spr0867 ; (3) l'antigène spr1431 ; (4) l'antigène spr1433 ; et/ou (5) l'antigène spr1707.

3. Composition immunogène selon la revendication 1, comprenant un antigène spr0057 et/ou un antigène spr0565.

4. Composition selon la revendication 3, comprenant en outre un ou plusieurs parmi :
(a) un antigène pilus RrgA et/ou RrgB de *S. pneumoniae* ;
(b) un antigène Pmp de *S. pneumoniae* ; et/ou
(c) un ou plusieurs conjugués d'un saccharide capsulaire pneumococcique et une protéine porteuse, dans laquelle (i) le saccharide capsulaire provient d'un ou de plusieurs des sérotypes pneumococciques suivants : 1, 3, 4, 5, 6B, 7F, 9V, 14, 18C, 19F et 23F ; et (ii) la protéine porteuse est une toxine ou un toxoïde bactérien ou est une protéine D provenant de H. *influenzae,*
dans laquelle :
ledit antigène RrgA comprend une séquence d'acides aminés : (a) ayant une identité de 80 % ou plus avec la SEQ ID N° : 172 ou 179 ; et/ou (b) comprend un fragment d'au moins 10 acides aminés consécutifs, comprenant un épitope de la SEQ ID N° : 172 ou 179 ;
ledit antigène RrgB comprend une séquence d'acides aminés : (a) ayant une identité de 80 % ou plus avec la SEQ ID N° : 173, 174 ou 175 ; et/ou (b) comprend un fragment d'au moins 10 acides aminés consécutifs, comprenant un épitope de la SEQ ID N° : 173, 174 ou 175 ; et/ou
ledit antigène Pmp comprend une séquence d'acides aminés : (a) ayant une identité de 80 % ou plus avec la SEQ ID N° : 28 ; et/ou (b) comprend un fragment d'au moins 10 acides aminés consécutifs, comprenant un épitope de la SEQ ID N° : 28.

5. Composition immunogène selon la revendication 1 ou la revendication 2, comprenant :
(a) un ou plusieurs conjugués d'un saccharide capsulaire pneumococcique et une protéine porteuse ;
(b) un ou plusieurs antigènes choisis dans le groupe constitué du : toxoïde de la diphtérie ; toxoïde du tétanos ; l'antigène de surface du virus de l'hépatite B ; l'antigène du virus de la polio inactivé ; un ou plusieurs antigènes de la coqueluche acellulaire ; un conjugué de l'antigène de saccharide capsulaire de *H. influenzae* de type B ; un conjugué de l'antigène de saccharide capsulaire du sérogroupe C de *N. meningitidis* ; un conjugué de l'antigène de saccharide capsulaire du sérogroupe Y de N. *meningitidis* ; un conjugué de l'antigène de saccharide capsulaire du sérogroupe W135 de *N. meningitidis* ; et un conjugué de l'antigène de saccharide capsulaire du sérogroupe A de *N. meningitidis* ; ou
(c) un agent de protection contre la température qui améliore la stabilité thermique de la composition.

6. Polypeptide de formule NH₂-A-{-X-L-}*ₙ*-B-COOH, dans laquelle :
X est une séquence d'acides aminés d'un antigène pneumococcique, choisi dans le groupe constitué de : (1) un antigène spr0057 ; (2) un antigène spr0096 ; (3) un antigène spr0565 ; et (4) un antigène spr2021 ; (5) un antigène RrgA ; et (6) un antigène RrgB ;
L est une séquence d'acides aminés facultative d'un segment de liaison ;
A est une séquence d'acides aminés N-terminale facultative ;
B est une séquence d'acides aminés C-terminale facultative ;
*n* est un nombre entier de 2 ou plus ; et
au moins une apparition de X est spr0096 et au moins une apparition de X est spr2021 ;
dans lequel
ledit antigène spr0057 comprend une séquence d'acides aminés : (a) ayant une identité de 80 % ou plus avec la SEQ ID N° : 1 ; et/ou (b) comprend un fragment d'au moins 10 acides aminés consécutifs, comprenant un épitope de la SEQ ID N° : 1 ;
ledit antigène spr0096 comprend une séquence d'acides aminés : (a) ayant une identité de 80 % ou plus avec la SEQ ID N° : 12 ; et/ou (b) comprend un fragment d'au moins 10 acides aminés consécutifs, comprenant un épitope de la SEQ ID N° : 12 ;
ledit antigène spr0565 comprend une séquence d'acides aminés : (a) ayant une identité de 80 % ou plus avec la SEQ ID N° : 3 ; et/ou (b) comprend un fragment d'au moins 10 acides aminés consécutifs, comprenant un épitope de la SEQ ID N° : 3 ;
ledit antigène spr2021 comprend une séquence d'acides aminés : (a) ayant une identité de 80 % ou plus avec la SEQ ID N° : 11 ; et/ou (b) comprend un fragment d'au moins 10 acides aminés consécutifs, comprenant un épitope de la SEQ ID N° : 11 ;
ledit antigène RrgA comprend une séquence d'acides aminés : (a) ayant une identité de 80 % ou plus avec la SEQ ID N° : 172 ou 179 ; et/ou (b) comprend un fragment d'au moins 10 acides aminés consécutifs, comprenant un épitope de la SEQ ID N° : 172 ou 179 ; et/ou
ledit antigène RrgB comprend une séquence d'acides aminés : (a) ayant une identité de 80 % ou plus avec la SEQ ID N° : 173, 174 ou 175 ; et/ou (b) comprend un fragment d'au moins 10 acides aminés consécutifs, comprenant un épitope de la SEQ ID N° : 173, 174 ou 175.

7. Polypeptide selon la revendication 6, ayant la formule NH₂-A-X₁-L₁-X₂-L₂-B-COOH, dans laquelle :
(a) X₁ est une séquence d'acides aminés d'un antigène pneumococcique de spr0096 et X₂ est une séquence d'acides aminés d'un antigène pneumococcique de spr2021 ; ou
(b) X₁ est une séquence d'acides aminés d'un antigène pneumococcique de spr2021 et X₂ est une séquence d'acides aminés d'un antigène pneumococcique de spr0096 ;
L est une séquence d'acides aminés facultative d'un segment de liaison ;
A est une séquence d'acides aminés N-terminale facultative ;
B est une séquence d'acides aminés C-terminale facultative ;
et dans lequel
ledit antigène spr0096 comprend une séquence d'acides aminés : (a) ayant une identité de 80 % ou plus avec la SEQ ID N° : 12 ; et/ou (b) comprend un fragment d'au moins 10 acides aminés consécutifs, comprenant un épitope de la SEQ ID N° : 12 ; et/ou
ledit antigène spr2021 comprend une séquence d'acides aminés : (a) ayant une identité de 80 % ou plus avec la SEQ ID N° : 11 ; et/ou (b) comprend un fragment d'au moins 10 acides aminés consécutifs, comprenant un épitope de la SEQ ID N° : 11.

8. Polypeptide selon la revendication 6 ou la revendication 7, dans lequel au moins une séquence L comprend Glyₓ, où x = 2, 3, 4, 5, 6, 7, 8, 9 ou 10.

9. Polypeptide selon la revendication 6 ou la revendication 7, comprenant une séquence d'acides aminés choisie dans le groupe constitué des SEQ ID N° : 194 et 205.

10. Polypeptide ayant une identité de séquence de 75 % ou plus avec une séquence d'acides aminés choisie dans le groupe constitué des SEQ ID N° : 194 et 205.

11. Composition immunogène comprenant le polypeptide selon l'une quelconque des revendications 6 à 10.

12. Acide nucléique codant pour le polypeptide selon l'une quelconque des revendications 6 à 10.

13. Composition immunogène selon l'une quelconque des revendications 1 à 5 ou 11 pour une utilisation dans un procédé d'augmentation de la réponse immunitaire chez un mammifère.

14. Composition immunogène selon la revendication 13 pour une utilisation dans la protection dudit mammifère contre une infection pneumococcique.
